(19) Europäisches Patentamt
European Patent Office
Office européen des brevets

(11) **EP 4 455 787 A1**

(12) **EUROPEAN PATENT APPLICATION**
published in accordance with Art. 153(4) EPC

(43) Date of publication:
**30.10.2024 Bulletin 2024/44**

(21) Application number: **22910960.8**

(22) Date of filing: **09.12.2022**

(51) International Patent Classification (IPC):
***G03F 7/004** (2006.01)* ***C07C 25/18** (2006.01)*
***C07C 309/06** (2006.01)* ***C07C 309/12** (2006.01)*
***C07C 309/42** (2006.01)* ***C07C 309/65** (2006.01)*
***C07C 311/53** (2006.01)* ***C07C 381/00** (2006.01)*
***C07C 381/12** (2006.01)* ***C07D 307/00** (2006.01)*
***C07D 317/72** (2006.01)* ***C07D 321/10** (2006.01)*
***C07D 327/06** (2006.01)* ***C07D 333/76** (2006.01)*
***G03F 7/038** (2006.01)* ***G03F 7/039** (2006.01)*
***G03F 7/20** (2006.01)*

(52) Cooperative Patent Classification (CPC):
**C07C 25/18; C07C 309/06; C07C 309/12; C07C 309/42; C07C 309/65; C07C 311/53; C07C 381/00; C07C 381/12; C07D 307/00; C07D 317/72; C07D 321/10; C07D 327/06; C07D 333/76; G03F 7/004; G03F 7/038;** (Cont.)

(86) International application number:
**PCT/JP2022/045518**

(87) International publication number:
**WO 2023/120250 (29.06.2023 Gazette 2023/26)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **23.12.2021 JP 2021210013**

(71) Applicant: **FUJIFILM Corporation**
**Tokyo 106-8620 (JP)**

(72) Inventors:
- **KOJIMA, Masafumi**
  **Haibara-gun, Shizuoka 421-0396 (JP)**
- **GOTO, Akiyoshi**
  **Haibara-gun, Shizuoka 421-0396 (JP)**

(74) Representative: **HGF**
**HGF Limited**
**1 City Walk**
**Leeds LS11 9DX (GB)**

(54) **ACTINIC-RAY-SENSITIVE OR RADIATION-SENSITIVE RESIN COMPOSITION, ACTINIC-RAY-SENSITIVE OR RADIATION-SENSITIVE FILM, PATTERN FORMING METHOD, ELECTRONIC DEVICE MANUFACTURING METHOD, AND COMPOUND**

(57) To provide an actinic ray-sensitive or radiation-sensitive resin composition and the like that provide a very good pattern profile in the formation of an ultrafine pattern (for example, a line-and-space pattern of 25 nm or less, a hole pattern having a hole diameter of 25 nm or less, or the like).

The actinic ray-sensitive or radiation-sensitive resin composition includes a resin, and an ionic compound having, in at least one of a cationic moiety or an anionic moiety, a group represented by $-SF_4R$.

EP 4 455 787 A1

(52) Cooperative Patent Classification (CPC): (Cont.)
**G03F 7/039; G03F 7/20**

## Description

## BACKGROUND OF THE INVENTION

1. Field of the Invention

The present invention relates to an actinic ray-sensitive or radiation-sensitive resin composition, an actinic ray-sensitive or radiation-sensitive film, a pattern forming method, a method for producing an electronic device, and compounds.

2. Description of the Related Art

In the manufacturing process of semiconductor devices such as IC (Integrated Circuit, integrated circuits) and LSI (LargeScale Integrated circuit, large-scale integrated circuits), microfabrication by lithography using photosensitive compositions has been performed.
Examples of the lithography method include a method of using a photosensitive composition to form a resist film, subsequently exposing the obtained film, and subsequently developing the film. In particular, in recent years, the use of, in addition to the ArF excimer laser, EB (Electron Beam) or EUV (Extreme ultraviolet) light during exposure has been studied, and an actinic ray-sensitive or radiation-sensitive resin composition suitable for EUV exposure has been developed.
In the formation of a resist pattern using EUV (having a wavelength of 13.5 nm) or an electron beam for the purpose of forming a fine pattern, requirements for various performances are stricter than in the related-art cases of using ArF (having a wavelength of 193 nm) light or the like.
For example, JP2020-180121A discloses a resist composition containing an acid generator containing a salt represented by a specified formula and a resin having a resin having an acid-labile group.

## SUMMARY OF THE INVENTION

In recent years, the size of patterns formed using EUV light or an electron beam has been reduced, and further improvements in the pattern profiles and the like have been in demand.
Thus, an object of the present invention is to provide an actinic ray-sensitive or radiation-sensitive resin composition that provides a very good pattern profile in the formation of an ultrafine pattern (for example, a line-and-space pattern of 25 nm or less, a hole pattern having a hole diameter of 25 nm or less, or the like).
Another object of the present invention is to provide an actinic ray-sensitive or radiation-sensitive film, a pattern forming method, and a method for producing an electronic device that use the above-described actinic ray-sensitive or radiation-sensitive resin composition, and compounds.
The inventors of the present invention have found that the following features can address the above-described objects.
[1] An actinic ray-sensitive or radiation-sensitive resin composition including:

a resin; and
an ionic compound having, in at least one of a cationic moiety or an anionic moiety, a group represented by $-SF_4R$, wherein R represents a hydrogen atom, a halogen atom, or a monovalent substituent.

[2] The actinic ray-sensitive or radiation-sensitive resin composition according to [1], wherein the ionic compound is an ionic compound having, in the cationic moiety, the group represented by $-SF_4R$.
[3] The actinic ray-sensitive or radiation-sensitive resin composition according to [1] or [2], wherein the cationic moiety of the ionic compound is a sulfonium cation or an iodonium cation.
[4] The actinic ray-sensitive or radiation-sensitive resin composition according to any one of [1] to [3], wherein the ionic compound is an ionic compound having, in the anionic moiety, the group represented by $-SF_4R$.
[5] The actinic ray-sensitive or radiation-sensitive resin composition according to any one of [1] to [4], wherein the group represented by $-SF_4R$ is $-SF_5$.
[6] The actinic ray-sensitive or radiation-sensitive resin composition according to any one of [1] to [5], wherein the resin has a repeating unit having a phenolic hydroxy group.
[7] The actinic ray-sensitive or radiation-sensitive resin composition according to any one of [1] to [6], wherein the resin has a repeating unit having at least one selected from the group consisting of a lactone group, a sultone group,

and a carbonate group.
[8] An actinic ray-sensitive or radiation-sensitive film formed from the actinic ray-sensitive or radiation-sensitive resin composition according to any one of [1] to [7].
[9] A pattern forming method including:

a step of using the actinic ray-sensitive or radiation-sensitive resin composition according to any one of [1] to [7] to form an actinic ray-sensitive or radiation-sensitive film on a substrate;
a step of exposing the actinic ray-sensitive or radiation-sensitive film; and
a step of using a developer to develop the exposed actinic ray-sensitive or radiation-sensitive film to form a pattern.

[10] A method for producing an electronic device, the method including the pattern forming method according to [9].
[11] A sulfonium salt compound including, in at least one of a cationic moiety or an anionic moiety, a group represented by $-SF_4R$,
wherein R represents a hydrogen atom, a halogen atom, or a monovalent substituent.
[12] The compound according to [11], wherein the sulfonium salt compound is a sulfonium salt compound having, in the cationic moiety, the group represented by $-SF_4R$.
[13] The compound according to [11] or [12], wherein the sulfonium salt compound is a sulfonium salt compound having, in the anionic moiety, the group represented by $-SF_4R$.
[14] An iodonium salt compound including, in at least one of a cationic moiety or an anionic moiety, a group represented by $-SF_4R$, wherein R represents a hydrogen atom, a halogen atom, or a monovalent substituent and the anionic moiety is represented by any one of formulas (N1) to (N3) below:

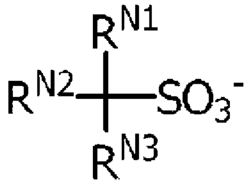
(N1)
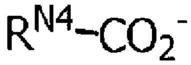
(N2)
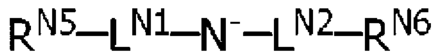
(N3)

in the formula (N1),

$R^{N1}$ represents a hydrogen atom, a chlorine atom, a bromine atom, an iodine atom, a hydroxy group, a group represented by $-SF_4R$, or an organic group, wherein R represents a hydrogen atom, a halogen atom, or a monovalent substituent,
$R^{N2}$ to $R^{N3}$ each independently represent a hydrogen atom, a fluorine atom, a chlorine atom, a bromine atom, an iodine atom, a hydroxy group, a group represented by $-SF_4R$, or an organic group, wherein R represents a hydrogen atom, a halogen atom, or a monovalent substituent,
$R^{N1}$ to $R^{N3}$ may be bonded together to form a ring,
in the formula (N2),
$R^{N4}$ represents an organic group,
in the formula (N3),
$L^{N1}$ and $L^{N2}$ each independently represent $-SO_2-$, -CO-, or an alkylene group,
$R^{N5}$ and $R^{N6}$ each independently represent an organic group, and $R^{N5}$ and $R^{N6}$ may be bonded together to form a ring.

[15] The compound according to [14], wherein the iodonium salt compound is an iodonium salt compound having, in the cationic moiety, the group represented by $-SF_4R$.
[16] The compound according to [14] or [15], wherein the iodonium salt compound is an iodonium salt compound having, in the anionic moiety, the group represented by $-SF_4R$.
[17] The compound according to any one of [11] to [16], wherein the group represented by $-SF_4R$ is $-SF_5$.

The present invention can provide an actinic ray-sensitive or radiation-sensitive resin composition that provides a very good pattern profile in the formation of an ultrafine pattern (for example, a line-and-space pattern of 25 nm or less, a hole pattern having a hole diameter of 25 nm or less, or the like).
The present invention can also provide an actinic ray-sensitive or radiation-sensitive film, a pattern forming method, and a method for producing an electronic device that use the above-described actinic ray-sensitive or radiation-sensitive resin composition, and compounds.

## DESCRIPTION OF THE PREFERRED EMBODIMENTS

Hereinafter, the present invention will be described in detail.

Features may be described below with reference to representative embodiments according to the present invention; however, the present invention is not limited to the embodiments.

In this Specification, for written forms of groups (atomic groups), written forms without referring to substituted or unsubstituted encompass, in addition to groups not having a substituent, groups including a substituent without departing from the spirit and scope of the present invention. For example, "alkyl group" encompasses not only alkyl groups not having a substituent (unsubstituted alkyl groups), but also alkyl groups having a substituent (substituted alkyl groups). In this Specification, "organic group" refers to a group including at least one carbon atom.

The substituent is preferably a monovalent substituent unless otherwise specified.

In this Specification, in the case of using a phrase "may have a substituent", the type of the substituent, the position of the substituent, and the number of such substituents are not particularly limited. The number of the substituents may be, for example, one, two, three, or more. Examples of the substituents include monovalent non-metallic atomic groups except for the hydrogen atom and, for example, can be selected from the group consisting of the following Substituents T.

Substituents T

Substituents T include halogen atoms such as a fluorine atom, a chlorine atom, a bromine atom, and an iodine atom; alkoxy groups such as a methoxy group, an ethoxy group, and a tert-butoxy group; aryloxy groups such as a phenoxy group and a p-tolyloxy group; alkoxycarbonyl groups such as a methoxycarbonyl group, a butoxycarbonyl group, and a phenoxycarbonyl group; acyloxy groups such as an acetoxy group, a propionyloxy group, and a benzoyloxy group; acyl groups such as an acetyl group, a benzoyl group, an isobutyryl group, an acryloyl group, a methacryloyl group, and a methoxalyl group; alkylsulfanyl groups such as a methylsulfanyl group and a tert-butylsulfanyl group; arylsulfanyl groups such as a phenylsulfanyl group and a p-tolylsulfanyl group; groups represented by $-SF_4R$ (where R represents a hydrogen atom, a halogen atom, or a monovalent substituent) such as tetrafluorosulfanyl groups; alkyl groups; cycloalkyl groups; aryl groups; heteroaryl groups; a hydroxy group; a carboxy group; a formyl group; a sulfo group; a cyano group; alkylaminocarbonyl groups; arylaminocarbonyl groups; a sulfonamide group; a silyl group; an amino group; monoalkylamino groups; dialkylamino groups; arylamino groups; a nitro group; and combinations of the foregoing.

In this Specification, "actinic ray" or "radiation" means, for example, the emission line spectrum of a mercury lamp, far-ultraviolet rays represented by excimer lasers, extreme ultraviolet rays (EUV light: Extreme Ultraviolet), X-rays, or an electron beam (EB: Electron Beam).

In this Specification, "light" means an actinic ray or a radiation.

In this Specification, "exposure" includes, unless otherwise specified, not only exposure using, for example, the emission line spectrum of a mercury lamp, far-ultraviolet rays represented by excimer lasers, extreme ultraviolet rays (EUV: Extreme ultraviolet), or X-rays, but also patterning using a corpuscular beam such as an electron beam or an ion beam.

In this Specification, "a value 'to' another value" is used to mean that it includes the value and the other value as the lower limit value and the upper limit value.

In this Specification, the described bonding directions of divalent linking groups are not limited unless otherwise specified. For example, in a compound represented by a formula "X-Y-Z" where Y is -COO-, Y may be - CO-O- or -O-CO-. The compound may be "X-CO-O-Z" or may be "X-O-CO-Z".

In this Specification, (meth)acrylate represents acrylate and methacrylate, and (meth)acrylic represents acrylic and methacrylic.

In this Specification, the weight-average molecular weight (Mw), the number-average molecular weight (Mn), and the dispersity (hereafter, also referred to as "molecular weight distribution") (Mw/Mn) are defined as polystyrene-equivalent values measured using a GPC (Gel Permeation Chromatography) apparatus (HLC-8120GPC manufactured by Tosoh Corporation) by GPC measurement (solvent: tetrahydrofuran, flow rate (sample injection amount): 10 μL, column: TSK gel Multipore HXL-M manufactured by Tosoh Corporation, column temperature: 40°C, flow rate: 1.0 mL/min, detector: differential refractive index detector (Refractive Index Detector)).

In this Specification, the acid dissociation constant (pKa) represents pKa in an aqueous solution, specifically, a value determined using the following Software package 1, on the basis of the Hammett's substituent constant and the database of values in publicly known documents, by calculation.

Software package 1: Advanced Chemistry Development (ACD/Labs) Software V8.14 for Solaris (1994-2007 ACD/Labs)

Alternatively, pKa can be determined by the molecular orbital calculation method. Specifically, this method may be a method of, on the basis of a thermodynamic cycle, calculating $H^+$ dissociation free energy in an aqueous solution to achieve the determination. As the calculation method for $H^+$ dissociation free energy, for example, DFT (density function theory) can be performed for calculation; however, other various methods have been reported in documents and the like and the calculation method is not limited to DFT. Note that there are a plurality of pieces of software for performing DFT, such as Gaussian 16.

In this Specification, as described above, pKa refers to a value determined using Software package 1, on the basis of the Hammett's substituent constant and the database of values in publicly known documents, by calculation; however, when use of this method cannot determine pKa, a value determined on the basis of DFT (density function theory) using Gaussian 16 is employed.

In this Specification, as described above, pKa refers to "pKa in an aqueous solution"; however, when pKa in an aqueous solution cannot be determined, "pKa in a dimethyl sulfoxide (DMSO) solution" is employed.

"Solid content" means components forming the actinic ray-sensitive film and does not include solvents. As long as a component forms the actinic ray-sensitive film, even when the component has the form of liquid, it is regarded as the solid content.

## Actinic ray-sensitive or radiation-sensitive resin composition

An actinic ray-sensitive or radiation-sensitive resin composition of the present invention includes:

a resin; and
an ionic compound having, in at least one of a cationic moiety or an anionic moiety, a group represented by - $SF_4R$ (where R represents a hydrogen atom, a halogen atom, or a monovalent substituent).

The mechanism by which such features address objects of the present invention is not necessarily clear, but is inferred as follows: the group represented by $-SF_4R$ included in the ionic compound has a structure in which fluorine atoms are present at high density, and such a structure provides, compared with just halogen atoms or the like, a stronger interaction with the acid generated in the exposed region (for example, the acid generated from a photoacid generator). As a result, diffusion of the acid to the unexposed region can be suppressed at a very high level, to thereby inferentially provide a very good pattern profile in the formation of an ultrafine pattern (for example, a line-and-space pattern of 25 nm or less, a hole pattern having a hole diameter of 25 nm or less, or the like).

## Components of actinic ray-sensitive or radiation-sensitive resin composition

Hereinafter, components that the actinic ray-sensitive or radiation-sensitive resin composition can include will be described in detail.

The actinic ray-sensitive or radiation-sensitive resin composition of the present invention (hereafter, also referred to as "composition of the present invention") is typically a resist composition, and may be a positive resist composition or may be a negative resist composition. The resist composition may be a resist composition for alkali development or may be a resist composition for organic solvent development. The composition of the present invention is typically a chemical amplification resist composition.

## Resin

The composition of the present invention includes a resin (hereafter, also referred to as "resin (A)").

The resin (A) ordinarily includes a group that is decomposed by the action of an acid to provide increased polarity (hereafter, also referred to as "acid-decomposable group") and preferably includes a repeating unit having the acid-decomposable group.

When the resin (A) has an acid-decomposable group, in a pattern forming method in this Specification, typically, in the case of employing a developer that is an alkali developer, a positive-type pattern is suitably formed or, in the case of employing a developer that is an organic-based developer, a negative-type pattern is suitably formed.

Preferred examples of the repeating unit having an acid-decomposable group include, in addition to a repeating unit having an acid-decomposable group described later, a repeating unit having an acid-decomposable group including an unsaturated bond.

Repeating unit having acid-decomposable group

When the resin (A) includes a repeating unit having an acid-decomposable group, the resin (A) is an acid-decomposable resin.

The acid-decomposable group is preferably a group that is decomposed by the action of an acid to generate a polar group.

The acid-decomposable group preferably has a structure in which the polar group is protected with a group (leaving group) that leaves by the action of an acid. Thus, the resin (A) has a repeating unit having a group that is decomposed by the action of an acid to generate a polar group. The resin having the repeating unit is subjected to the action of an acid to have increased polarity to have an increased degree of solubility in the alkali developer, but have a decreased degree of solubility in organic solvents.

The polar group is preferably an alkali soluble group; examples include acidic groups such as a carboxyl group, a phenolic hydroxy group, fluorinated alcohol groups, a sulfonic group, a phosphate group, a sulfonamide group, a sulfonylimide group, (alkylsulfonyl)(alkylcarbonyl)methylene groups, (alkylsulfonyl)(alkylcarbonyl)imide groups, bis(alkylcarbonyl)methylene groups, bis(alkylcarbonyl)imide groups, bis(alkylsulfonyl)methylene groups, bis(alkylsulfonyl)imide groups, tris(alkylcarbonyl)methylene groups, and tris(alkylsulfonyl)methylene groups, and an alcoholic hydroxy group.

In particular, the polar group is preferably a carboxyl group, a phenolic hydroxy group, a fluorinated alcohol group (preferably a hexafluoroisopropanol group), or a sulfonic group.

Examples of the group that leaves by the action of an acid include groups represented by formulas (Y1) to (Y4).

formula (Y1): $-C(Rx_1)(Rx_2)(Rx_3)$

formula (Y2): $-C(=O)OC(Rx_1)(Rx_2)(Rx_3)$

formula (Y3): $-C(R_{36})(R_{37})(OR_{38})$

formula (Y4): $-C(Rn)(H)(Ar)$

In the formula (Y1) and the formula (Y2), $Rx_1$ to $Rx_3$ each independently represent a hydrocarbon group and preferably represents an alkyl group (linear or branched), a cycloalkyl group (monocyclic or polycyclic), an alkenyl group (linear or branched), or an aryl group (monocyclic or polycyclic). Note that, when $Rx_1$ to $Rx_3$ are all alkyl groups (linear or branched), at least two among $Rx_1$ to $Rx_3$ are preferably methyl groups.

In particular, $Rx_1$ to $Rx_3$ preferably each independently represent a linear or branched alkyl group, and $Rx_1$ to $Rx_3$ more preferably each independently represent a linear alkyl group.

Two among $Rx_1$ to $Rx_3$ may be bonded together to form a ring (that may be a monocycle or a polycycle).

For $Rx_1$ to $Rx_3$, the alkyl group is preferably an alkyl group having 1 to 5 carbon atoms such as a methyl group, an ethyl group, an n-propyl group, an isopropyl group, an n-butyl group, an isobutyl group, or a t-butyl group.

For $Rx_1$ to $Rx_3$, the cycloalkyl group is preferably a monocyclic cycloalkyl group such as a cyclopentyl group or a cyclohexyl group, or a polycyclic cycloalkyl group such as a norbornyl group, a tetracyclodecanyl group, a tetracyclododecanyl group, or an adamantyl group. In the cycloalkyl group, for example, one of the methylene groups constituting the ring may be replaced by a heteroatom such as an oxygen atom or a sulfur atom, a group including a heteroatom such as a carbonyl group, or a vinylidene group. In the cycloalkyl group, one or more of the ethylene groups constituting the cycloalkane ring may be replaced by vinylene groups.

For $Rx_1$ to $Rx_3$, the aryl group is preferably an aryl group having 6 to 10 carbon atoms, and may be, for example, a phenyl group, a naphthyl group, or an anthryl group.

For $Rx_1$ to $Rx_3$, the alkenyl group is preferably a vinyl group.

The ring formed by bonding together two among $Rx_1$ to $Rx_3$ is preferably a cycloalkyl group. The cycloalkyl group formed by bonding together two among $Rx_1$ to $Rx_3$ is preferably a monocyclic cycloalkyl group such as a cyclopentyl group or a cyclohexyl group, or a polycyclic cycloalkyl group such as a norbornyl group, a tetracyclodecanyl group, a tetracyclododecanyl group, or an adamantyl group, and more preferably a monocyclic cycloalkyl group having 5 to 6 carbon atoms.

In the cycloalkyl group formed by bonding together two among $Rx_1$ to $Rx_3$, one of methylene groups constituting the ring may be replaced by a heteroatom such as an oxygen atom, a group including a heteroatom such as a carbonyl group, or a vinylidene group. In the cycloalkyl group, one or more ethylene groups constituting the cycloalkane ring may be replaced by vinylene groups.

The group represented by the formula (Y1) or the formula (Y2) preferably has a form in which, for example, $Rx_1$ is a methyl group or an ethyl group, and $Rx_2$ and $Rx_3$ are bonded together to form the above-described cycloalkyl

group.

When the composition of the present invention is, for example, an EUV-exposure resist composition, the alkyl groups, the cycloalkyl groups, the alkenyl groups, and the aryl groups represented by $Rx_1$ to $Rx_3$ and the ring formed by bonding together two among $Rx_1$ to $Rx_3$ also preferably further have, as a substituent, a fluorine atom or an iodine atom.

In the formula (Y3), $R_{36}$ to $R_{38}$ each independently represent a hydrogen atom or a monovalent organic group. $R_{37}$ and $R_{38}$ may be bonded together to form a ring. The monovalent organic group may be an alkyl group, a cycloalkyl group, an aryl group, an aralkyl group, or an alkenyl group. $R_{36}$ is also preferably a hydrogen atom.

Note that the alkyl group, the cycloalkyl group, the aryl group, and the aralkyl group may include a heteroatom such as an oxygen atom and/or a group including a heteroatom such as a carbonyl group. For example, in the alkyl group, the cycloalkyl group, the aryl group, and the aralkyl group, one or more methylene groups may be replaced by a heteroatom such as an oxygen atom and/or a group including a heteroatom such as a carbonyl group.

$R_{38}$ and another substituent of the main chain of the repeating unit may be bonded together to form a ring. The group formed by bonding together $R_{38}$ and another substituent of the main chain of the repeating unit is preferably an alkylene group such as a methylene group.

When the composition of the present invention is, for example, an EUV-exposure resist composition, the monovalent organic groups represented by $R_{36}$ to $R_{38}$ and the ring formed by bonding together $R_{37}$ and $R_{38}$ also preferably further have, as a substituent, a fluorine atom or an iodine atom.

The formula (Y3) is preferably a group represented by the following formula (Y3-1).

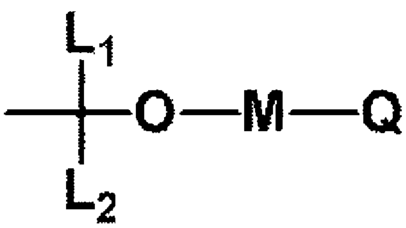

(Y3-1)

$L_1$ and $L_2$ each independently represent a hydrogen atom, an alkyl group, a cycloalkyl group, an aryl group, or a group that is a combination of the foregoing (for example, a group that is a combination of an alkyl group and an aryl group).

M represents a single bond or a divalent linking group.

Q represents an alkyl group that may include a heteroatom, a cycloalkyl group that may include a heteroatom, an aryl group that may include a heteroatom, an amino group, an ammonium group, a mercapto group, a cyano group, an aldehyde group, or a group that is a combination of the foregoing (for example, a group that is a combination of an alkyl group and a cycloalkyl group).

In the alkyl group and the cycloalkyl group, for example, one of methylene groups may be replaced by a heteroatom such as an oxygen atom or a group including a heteroatom such as a carbonyl group.

Note that one of $L_1$ and $L_2$ is preferably a hydrogen atom and the other is preferably an alkyl group, a cycloalkyl group, an aryl group, or a group that is a combination of an alkylene group and an aryl group.

At least two among Q, M, and $L_1$ may be bonded together to form a ring (preferably a 5-membered or 6-membered ring).

From the viewpoint of forming finer patterns, $L_2$ is preferably a secondary or tertiary alkyl group, and more preferably a tertiary alkyl group. Examples of the secondary alkyl group include an isopropyl group, a cyclohexyl group, and a norbornyl group; examples of the tertiary alkyl group include a tert-butyl group and an adamantane group. In such examples, Tg (glass transition temperature) and activation energy are increased, so that film hardness is ensured and fogging can be suppressed.

When the composition of the present invention is, for example, an EUV-exposure resist composition, the alkyl groups, the cycloalkyl groups, the aryl groups, and the groups that are combinations of the foregoing represented by $L_1$ and $L_2$ also preferably further have, as a substituent, a fluorine atom or an iodine atom. The alkyl groups, the cycloalkyl groups, the aryl groups, and the aralkyl groups also preferably include, in addition to a fluorine atom and an iodine atom, a heteroatom such as an oxygen atom. Specifically, in the alkyl groups, the cycloalkyl groups, the aryl groups, and the aralkyl groups, for example, one of methylene groups may be replaced by a heteroatom such as an oxygen atom or a group including a heteroatom such as a carbonyl group.

When the composition of the present invention is, for example, an EUV-exposure resist composition, in the alkyl group that may include a heteroatom, the cycloalkyl group that may include a heteroatom, the aryl group that may include a heteroatom, the amino group, the ammonium group, the mercapto group, the cyano group, the aldehyde group, and the group that is a combination of the foregoing represented by Q, such a heteroatom is also preferably a heteroatom

selected from the group consisting of a fluorine atom, an iodine atom, and an oxygen atom.

In the formula (Y4), Ar represents an aromatic ring group. Rn represents an alkyl group, a cycloalkyl group, or an aryl group. Rn and Ar may be bonded together to form a non-aromatic ring. Ar is preferably an aryl group.

When the composition of the present invention is, for example, an EUV-exposure resist composition, the aromatic ring group represented by Ar and the alkyl group, the cycloalkyl group, and the aryl group represented by Rn also preferably have, as a substituent, a fluorine atom or an iodine atom.

From the viewpoint of providing a repeating unit having high acid-decomposability, in the leaving group protecting the polar group, when a non-aromatic ring is directly bonded to the polar group (or its residue), in the non-aromatic ring, a ring-member atom adjacent to a ring-member atom directly bonded to the polar group (or its residue) also preferably does not have, as a substituent, a halogen atom such as a fluorine atom.

Alternatively, the group that leaves by the action of an acid may be a 2-cyclopentenyl group having a substituent (such as an alkyl group) such as 3-methyl-2-cyclopentenyl group, or a cyclohexyl group having a substituent (such as an alkyl group) such as a 1,1,4,4-tetramethylcyclohexyl group.

The repeating unit having an acid-decomposable group is also preferably a repeating unit represented by a formula (A).

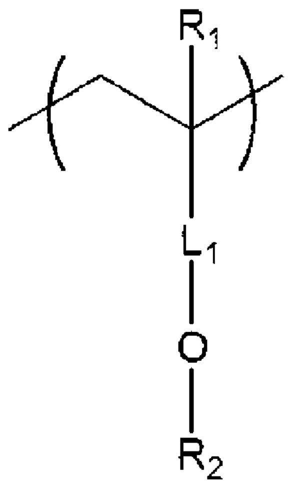

(A)

$L_1$ represents a divalent linking group that may have a fluorine atom or an iodine atom; $R_1$ represents a hydrogen atom, a fluorine atom, an iodine atom, or an alkyl group that may have a fluorine atom or an iodine atom, or an aryl group that may have a fluorine atom or an iodine atom; $R_2$ represents a leaving group that leaves by the action of an acid and that may have a fluorine atom or an iodine atom. Note that at least one of $L_1$, $R_1$, or $R_2$ has a fluorine atom or an iodine atom.

Examples of the divalent linking group that is represented by $L_1$ and may have a fluorine atom or an iodine atom include -CO-, -O-, -S-, -SO-, $-SO_2-$, hydrocarbon groups that may have a fluorine atom or an iodine atom (for example, alkylene groups, cycloalkylene groups, alkenylene groups, and arylene groups), and linking groups provided by linking together a plurality of the foregoing. In particular, $L_1$ is preferably -CO-, an arylene group, or an -arylene group-alkylene group having a fluorine atom or an iodine atom-, and more preferably -CO- or an -arylene group-alkylene group having a fluorine atom or an iodine atom-.

The arylene group is preferably a phenylene group.

The alkylene group may be linear or may be branched. The number of carbon atoms of the alkylene group is not particularly limited, but is preferably 1 to 10, and more preferably 1 to 3.

In the alkylene group having a fluorine atom or an iodine atom, the total number of fluorine atoms and iodine atoms is not particularly limited, but is preferably 2 or more, more preferably 2 to 10, and still more preferably 3 to 6.

The alkyl group represented by $R_1$ may be linear or may be branched. The number of carbon atoms of the alkyl group is not particularly limited, but is preferably 1 to 10, and more preferably 1 to 3.

In the alkyl group represented by $R_1$ and having a fluorine atom or an iodine atom, the total number of fluorine atoms and iodine atoms is not particularly limited, but is preferably 1 or more, more preferably 1 to 5, and still more preferably 1 to 3.

The alkyl group represented by $R_1$ may include a heteroatom other than halogen atoms, such as an oxygen atom.

Examples of the leaving group that is represented by $R_2$ and may have a fluorine atom or an iodine atom include leaving groups that are represented by the above-described formulas (Y1) to (Y4) and that have a fluorine atom or an iodine atom.

The repeating unit having an acid-decomposable group is also preferably a repeating unit represented by a formula (AI).

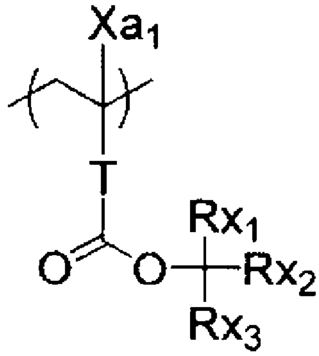

(A I)

In the formula (AI),

$Xa_1$ represents a hydrogen atom or an organic group.
T represents a single bond or a divalent linking group.
$Rx_1$ to $Rx_3$ each independently represent a hydrocarbon group.

Two among $Rx_1$ to $Rx_3$ may be bonded together to form a ring.

The organic group represented by $Xa_1$ is preferably an alkyl group. The alkyl group may be linear or may be branched. The alkyl group may have a substituent. Examples of the alkyl group include a methyl group and a group represented by $-CH_2-R_{11}$. $R_{11}$ represents a halogen atom (such as a fluorine atom), a hydroxy group, or a monovalent organic group. The monovalent organic group represented by $R_{11}$ is, for example, an alkyl group that has 5 or less carbon atoms and that may be substituted with a halogen atom, an acyl group that has 5 or less carbon atoms and that may be substituted with a halogen atom, or an alkoxy group that has 5 or less carbon atoms and that may be substituted with a halogen atom, and is preferably an alkyl group having 3 or less carbon atoms, and more preferably a methyl group. $Xa_1$ is preferably a hydrogen atom, a methyl group, a trifluoromethyl group, or a hydroxymethyl group.

For T, the divalent linking group may be an alkylene group, an aromatic ring group, a -COO-Rt- group, or an -O-Rt- group. In the formulas, Rt represent an alkylene group or a cycloalkylene group.

T is preferably a single bond or a -COO-Rt-group, and more preferably a single bond. When T represents a -COO-Rt- group, Rt is preferably an alkylene group having 1 to 5 carbon atoms, and more preferably a $-CH_2-$ group, a $-(CH_2)_2-$ group, or a $-(CH_2)_3-$ group.

For $Rx_1$ to $Rx_3$, the hydrocarbon group preferably has 1 to 10 carbon atoms. The hydrocarbon group may have a substituent.

For $Rx_1$ to $Rx_3$, the hydrocarbon group is preferably an alkyl group, a cycloalkyl group, an alkenyl group, an aryl group, or an aromatic heterocyclic group.

For $Rx_1$ to $Rx_3$, the alkyl group may be linear or may be branched. The alkyl group may have a substituent. For $Rx_1$ to $Rx_3$, the alkyl group is preferably an alkyl group having 1 to 4 carbon atoms such as a methyl group, an ethyl group, an n-propyl group, an isopropyl group, an n-butyl group, an isobutyl group, or a t-butyl group.

For $Rx_1$ to $Rx_3$, the cycloalkyl group may be a monocyclic cycloalkyl group, or may be a polycyclic cycloalkyl group. The cycloalkyl group may have a substituent. For $Rx_1$ to $Rx_3$, the cycloalkyl group is preferably a monocyclic cycloalkyl group such as a cyclopentyl group or a cyclohexyl group, or a polycyclic cycloalkyl group such as a norbornyl group, a tetracyclodecanyl group, a tetracyclododecanyl group, or an adamantyl group. In the cycloalkyl group, for example, one of the methylene groups constituting the ring may be replaced by a heteroatom such as an oxygen atom or a sulfur atom, a group including a heteroatom such as a carbonyl group, or a vinylidene group. In the cycloalkyl group, one or more of the ethylene groups constituting the cycloalkane ring may be replaced by vinylene groups

For $Rx_1$ to $Rx_3$, the aryl group may be a monocyclic aryl group or may be a polycyclic aryl group. The aryl group may have a substituent. For $Rx_1$ to $Rx_3$, the aryl group is preferably an aryl group having 6 to 10 carbon atoms, and may be, for example, a phenyl group, a naphthyl group, or an anthryl group.

For $Rx_1$ to $Rx_3$, the alkenyl group may be linear or may be branched. The alkenyl group may have a substituent. For $Rx_1$ to $Rx_3$, the alkenyl group is preferably a vinyl group.

For $Rx_1$ to $Rx_3$, the aromatic heterocyclic group may be a monocyclic aromatic heterocyclic group, or may be a polycyclic aromatic heterocyclic group. The aromatic heterocycle constituting the aromatic heterocyclic group is preferably an aromatic heterocycle having 3 to 10 carbon atoms and may be, for example, thiophene.

When two among $Rx_1$ to $Rx_3$ are bonded together to form a ring, the formed ring may be monocyclic or polycyclic. The formed ring is preferably a cycloalkyl group.

The cycloalkyl group is preferably a monocyclic cycloalkyl group such as a cyclopentyl group or a cyclohexyl group. Also preferred are polycyclic cycloalkyl groups such as a norbornyl group, a tetracyclodecanyl group, a tetracyclododecanyl group, and an adamantyl group.

In particular, preferred is a monocyclic cycloalkyl group having 5 to 6 carbon atoms.

In the cycloalkyl group formed by bonding together two among $Rx_1$ to $Rx_3$, for example, one of methylene groups constituting the ring may be replaced by a heteroatom such as an oxygen atom, a group including a heteroatom such as a carbonyl group, or a vinylidene group. In the cycloalkyl group, one or more of the ethylene groups constituting the cycloalkane ring may be replaced by vinylene groups.

The repeating unit represented by the formula (AI) preferably has a form in which, for example, $Rx_1$ is a methyl group or an ethyl group, and $Rx_2$ and $Rx_3$ are bonded together to form the above-described cycloalkyl group.

When the above-described groups each have a substituent, examples of the substituent include alkyl groups (having 1 to 4 carbon atoms), halogen atoms, a hydroxy group, alkoxy groups (having 1 to 4 carbon atoms), a carboxyl group, and alkoxycarbonyl groups (having 2 to 6 carbon atoms). The substituent preferably has 8 or less carbon atoms.

The repeating unit represented by the formula (AI) is preferably an acid-decomposable (meth)acrylic acid tertiary alkyl ester-based repeating unit (the repeating unit where $Xa_1$ represents a hydrogen atom or a methyl group and T represents a single bond).

The following are non-limiting specific examples of the repeating unit having an acid-decomposable group. Note that, in the formulas, $Xa_1$ represent H, $CH_3$, $CF_3$, or $CH_2OH$, and Rxa and Rxb each independently represent a linear or branched alkyl group having 1 to 5 carbon atoms.

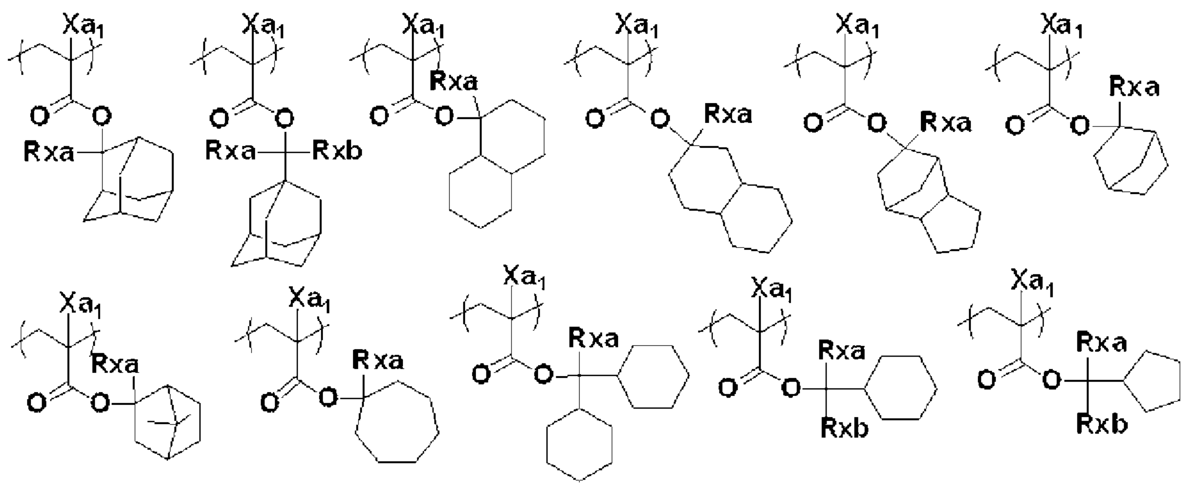

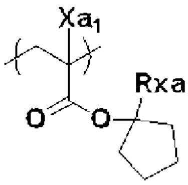
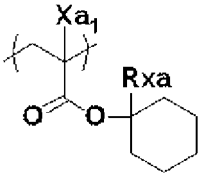
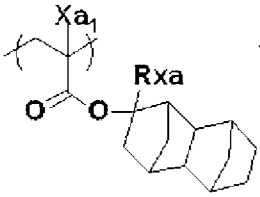
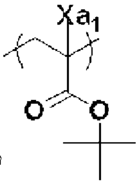
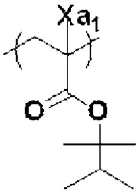
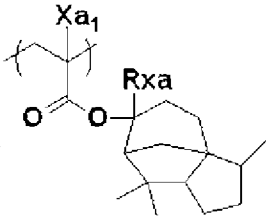

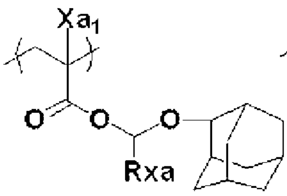
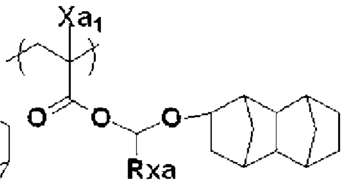
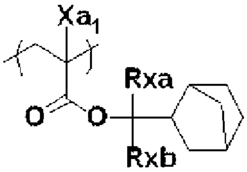
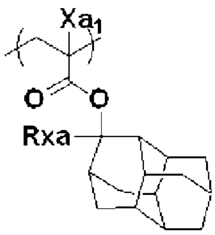

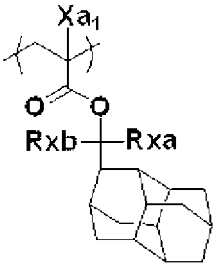
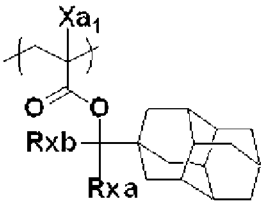
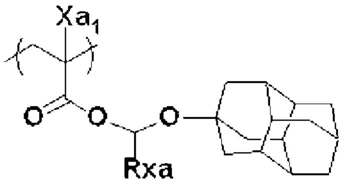
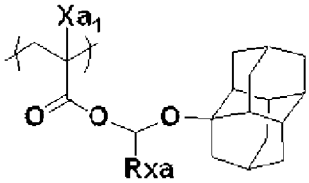

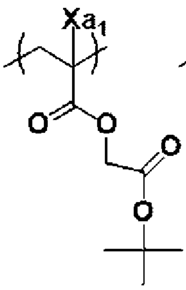
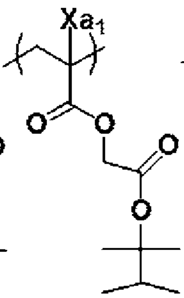
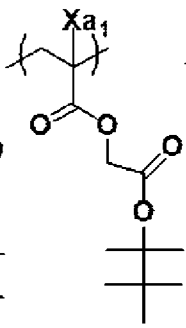
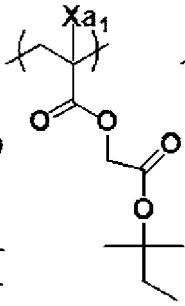
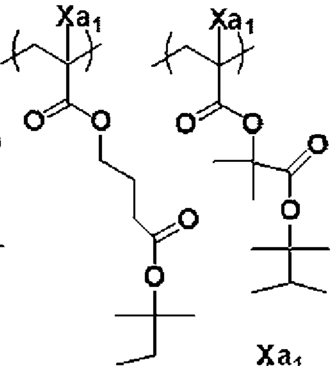

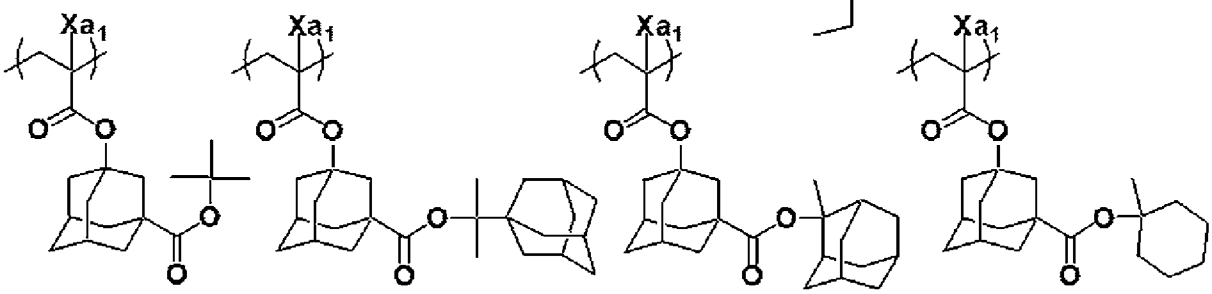

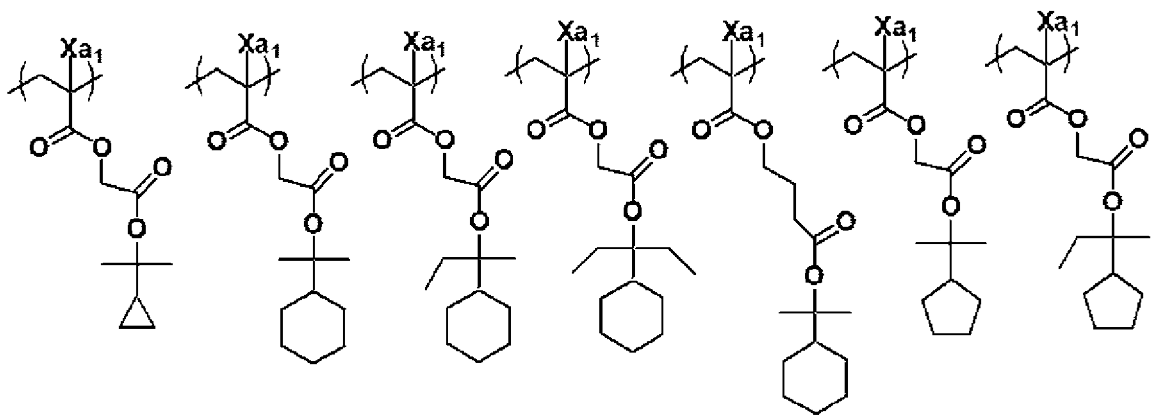

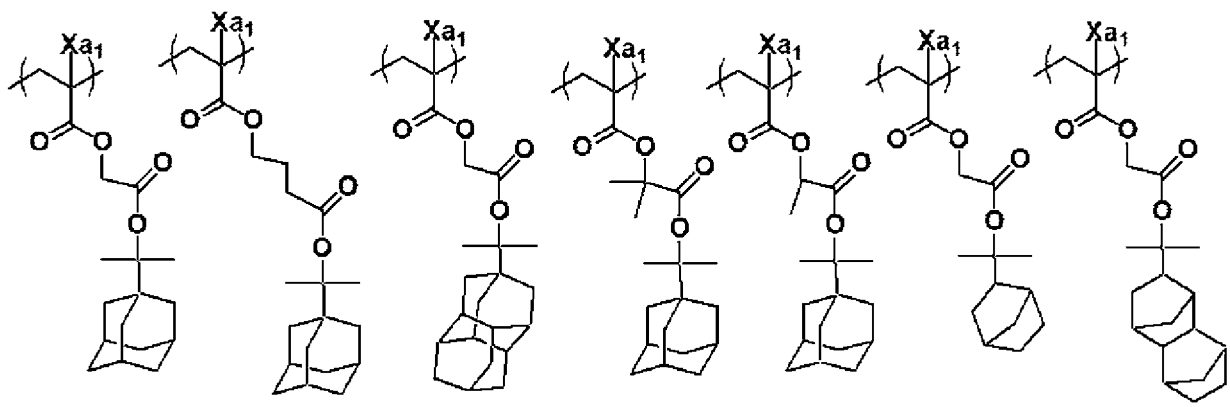

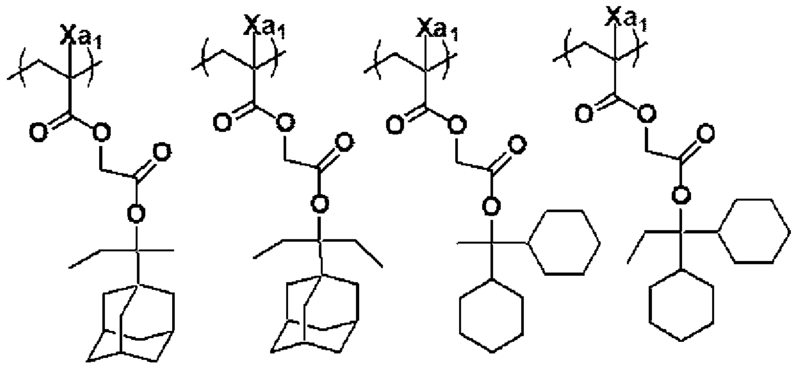

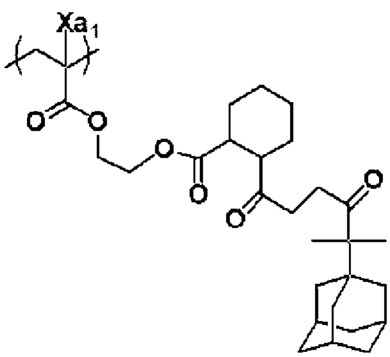

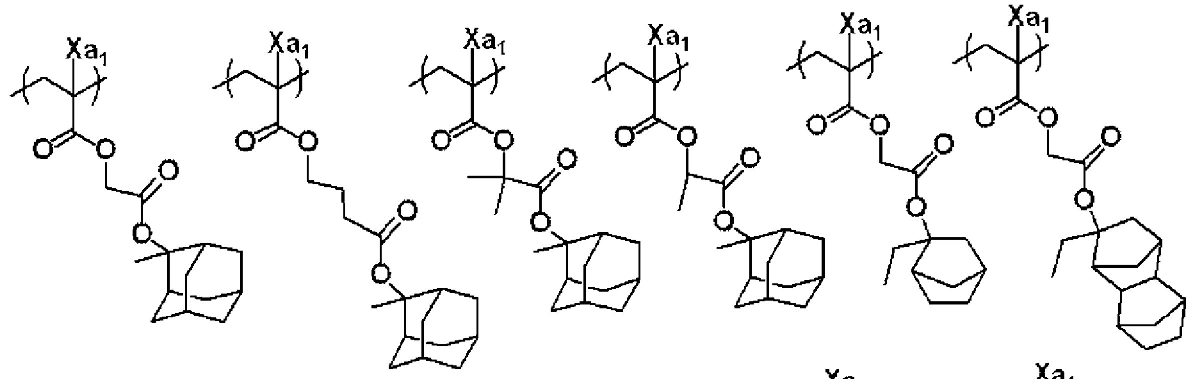

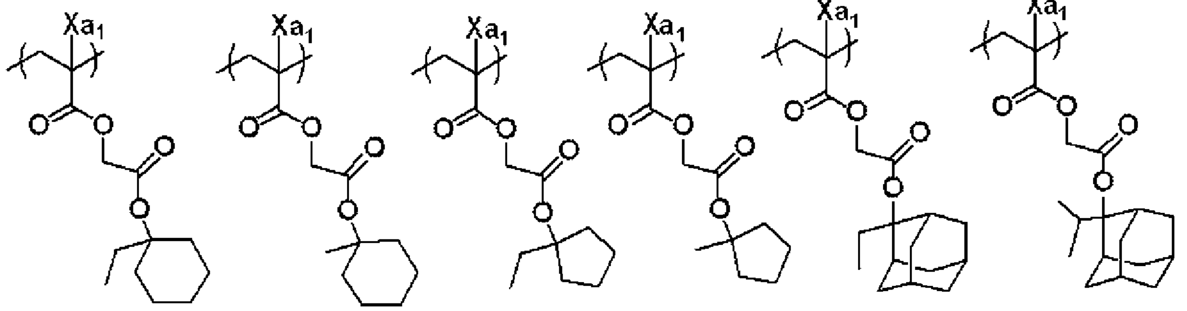

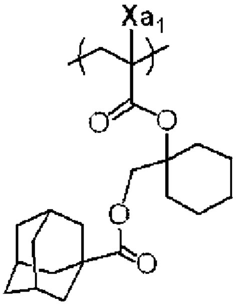

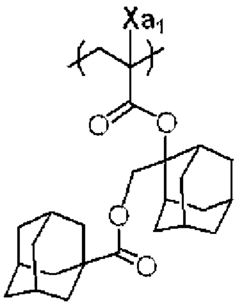

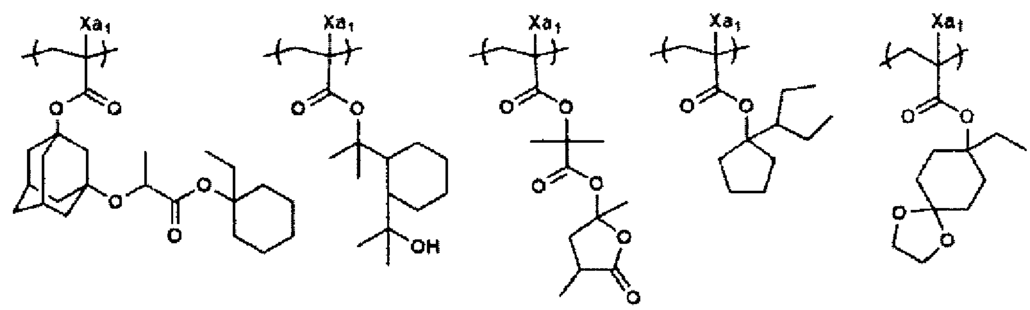

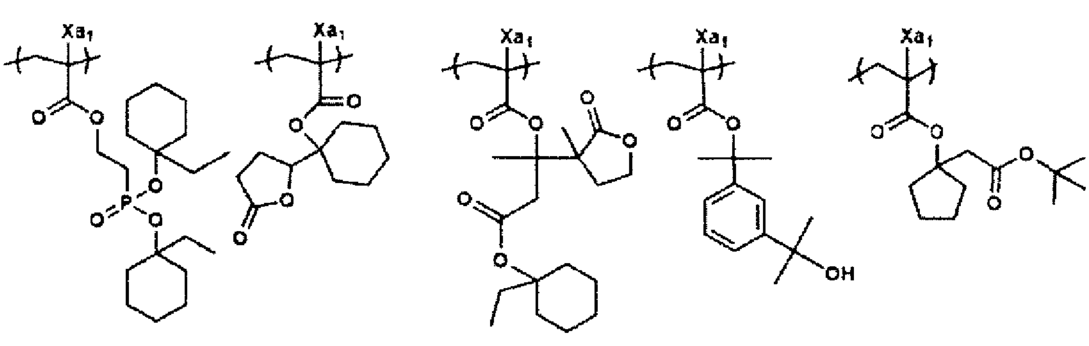

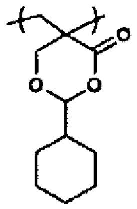
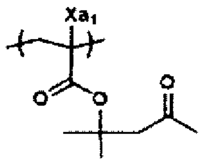
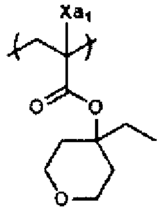
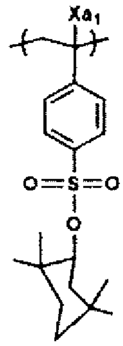
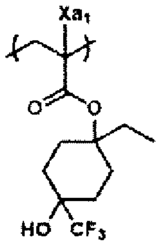
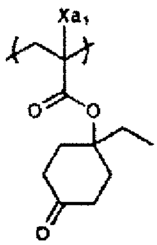
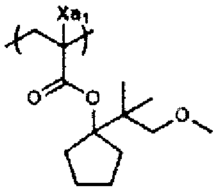

The resin (A) may have, as a repeating unit having an acid-decomposable group, a repeating unit having an acid-decomposable group including an unsaturated bond.

The repeating unit having an acid-decomposable group including an unsaturated bond is preferably a repeating unit represented by a formula (B).

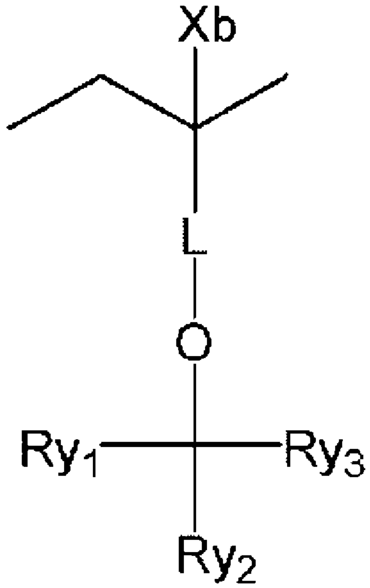

(B)

In the formula (B), Xb represents a hydrogen atom, a halogen atom, or an alkyl group that may have a substituent. L represents a single bond or a divalent linking group that may have a substituent. $Ry_1$ to $Ry_3$ each independently represent a linear or branched alkyl group, a monocyclic or polycyclic cycloalkyl group, an alkenyl group, an alkynyl group, or a monocyclic or polycyclic aryl group. Note that at least one of $Ry_1$ to $Ry_3$ represents an alkenyl group, an alkynyl group, a monocyclic or polycyclic cycloalkenyl group, or a monocyclic or polycyclic aryl group.

Two among $Ry_1$ to $Ry_3$ may be bonded together to form a monocycle or polycycle (such as a monocyclic or polycyclic cycloalkyl group or cycloalkenyl group).

For Xb, the alkyl group that may have a substituent may be, for example, a methyl group or a group represented by $-CH_2-R_{11}$. $R_{11}$ represents a halogen atom (such as a fluorine atom), a hydroxy group, or a monovalent organic group such as an alkyl group that has 5 or less carbon atoms and that may be substituted with a halogen atom, an acyl group that has 5 or less carbon atoms and that may be substituted with a halogen atom, or an alkoxy group that has 5 or less carbon atoms and that may be substituted with a halogen atom, is preferably an alkyl group having 3 or less carbon atoms, and more preferably a methyl group. Xb is preferably a hydrogen atom, a fluorine atom, a methyl group, a trifluoromethyl group, or a hydroxymethyl group.

For L, the divalent linking group may be an -Rt- group, a -CO- group, a -COO-Rt- group, a -COO-Rt-CO-group, an -Rt-CO- group, or an -O-Rt- group. In the formulas, Rt represent an alkylene group, a cycloalkylene group, or an aromatic ring group, and is preferably an aromatic ring group.

L is preferably an -Rt- group, a -CO- group, a -COO-Rt-CO- group, or an -Rt-CO- group. Rt may have a substituent such as a halogen atom, a hydroxy group, or an alkoxy group.

For $Ry_1$ to $Ry_3$, the alkyl group is preferably an alkyl group having 1 to 4 carbon atoms such as a methyl group, an ethyl group, an n-propyl group, an isopropyl group, an n-butyl group, an isobutyl group, or a t-butyl group.

For $Ry_1$ to $Ry_3$, the cycloalkyl group is preferably a monocyclic cycloalkyl group such as a cyclopentyl group or a cyclohexyl group, or a polycyclic cycloalkyl group such as a norbornyl group, a tetracyclodecanyl group, a tetracyclododecanyl group, or an adamantyl group.

For $Ry_1$ to $Ry_3$, the aryl group is preferably an aryl group having 6 to 10 carbon atoms, and may be, for example, a phenyl group, a naphthyl group, or an anthryl group.

For $Ry_1$ to $Ry_3$, the alkenyl group is preferably a vinyl group.

For $Ry_1$ to $Ry_3$, the alkynyl group is preferably an ethynyl group.

For $Ry_1$ to $Ry_3$, the cycloalkenyl group is preferably a structure in which a monocyclic cycloalkyl group such as a cyclopentyl group or a cyclohexyl group includes partially a double bond.

The cycloalkyl group formed by bonding together two among $Ry_1$ to $Ry_3$ is preferably a monocyclic cycloalkyl group such as a cyclopentyl group or a cyclohexyl group, or a polycyclic cycloalkyl group such as a norbornyl group, a tetracyclodecanyl group, a tetracyclododecanyl group, or an adamantyl group. In particular, more preferred is a monocyclic cycloalkyl group having 5 to 6 carbon atoms.

In the cycloalkyl group or the cycloalkenyl group formed by bonding together two among $Ry_1$ to $Ry_3$, for example, one of methylene groups constituting the ring may be replaced by a heteroatom such as an oxygen atom, a group including a heteroatom such as a carbonyl group, a $-SO_2-$ group, or a $-SO_3-$ group, a vinylidene group, or a combination of the foregoing. In the cycloalkyl group or the cycloalkenyl group, one or more ethylene groups constituting the cycloalkane ring or the cycloalkene ring may be replaced by vinylene groups.

The repeating unit represented by the formula (B) preferably has a form in which, for example, $Ry_1$ is a methyl group, an ethyl group, a vinyl group, an allyl group, or an aryl group, and $Ry_2$ and $Ry_3$ are bonded together to form the above-described cycloalkyl group or cycloalkenyl group.

When the above-described groups each have a substituent, examples of the substituent include alkyl groups (having 1 to 4 carbon atoms), halogen atoms, a hydroxy group, alkoxy groups (having 1 to 4 carbon atoms), a carboxyl group, and alkoxycarbonyl groups (having 2 to 6 carbon atoms). The substituent preferably has 8 or less carbon atoms.

The repeating unit represented by the formula (B) is preferably an acid-decomposable (meth)acrylic acid tertiary ester-based repeating unit (the repeating unit where Xb represents a hydrogen atom or a methyl group, and L represents a -CO- group), an acid-decomposable hydroxystyrene tertiary alkyl ether-based repeating unit (the repeating unit where Xb represents a hydrogen atom or a methyl group, and L represents a phenyl group), or an acid-decomposable styrenecarboxylic acid tertiary ester-based repeating unit (the repeating unit where Xb represents a hydrogen atom or a methyl group, and L represents an -Rt-CO- group (where Rt is an aromatic group)).

The content of the repeating unit having an acid-decomposable group including an unsaturated bond relative to all the repeating units in the resin (A) is preferably 15 mol% or more, more preferably 20 mol% or more, and still more preferably 30 mol% or more. The upper limit value relative to all the repeating units in the resin (A) is preferably 80 mol% or less, more preferably 70 mol% or less, and still more preferably 60 mol% or less.

The following are non-limiting specific examples of the repeating unit having an acid-decomposable group including an unsaturated bond. Note that, in the formulas, Xb and L 1 represent any one of the above-described substituents and linking groups; Ar represent an aromatic group; R represent a substituent such as a hydrogen atom, an alkyl group, a cycloalkyl group, an aryl group, an aralkyl group, an alkenyl group, a hydroxy group, an alkoxy group, an acyloxy group, a cyano group, a nitro group, an amino group, a halogen atom, an ester group (-OCOR'" or -COOR'" where R'" is an alkyl group or a fluorinated alkyl group having 1 to 20 carbon atoms), or a carboxyl group; R' represent a linear or branched alkyl group, a monocyclic or polycyclic cycloalkyl group, an alkenyl group, an alkynyl group, or a monocyclic or polycyclic aryl group; Q represent a heteroatom such as an oxygen atom, a group including a heteroatom such as a carbonyl group, a $-SO_2-$ group, or a $-SO_3-$ group, a vinylidene group, or a combination of the foregoing; n, m, and 1 represent an integer of 0 or more.

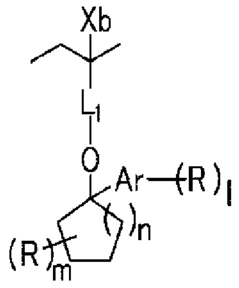
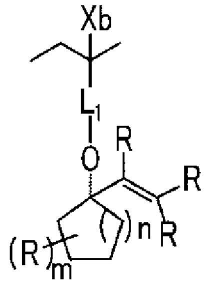
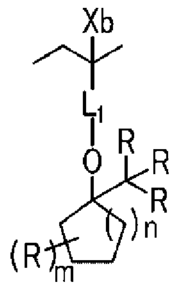

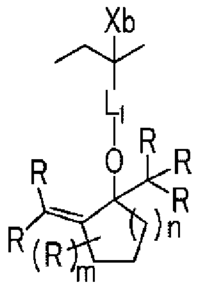
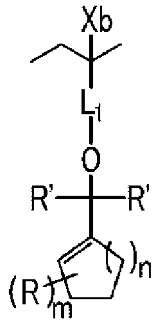
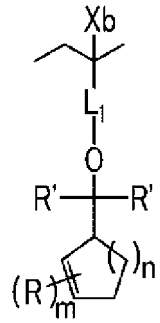

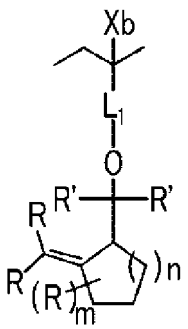
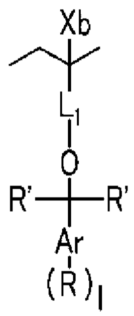
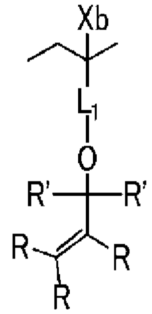

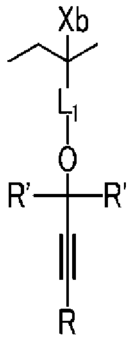
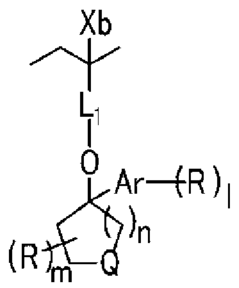
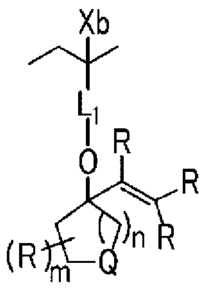

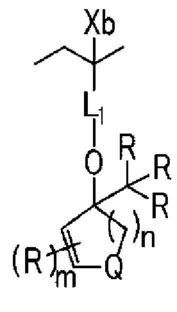
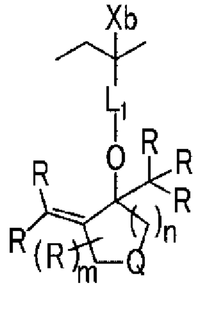

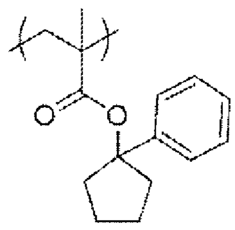
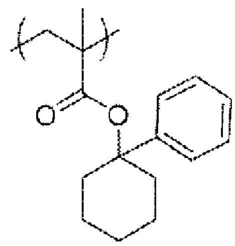
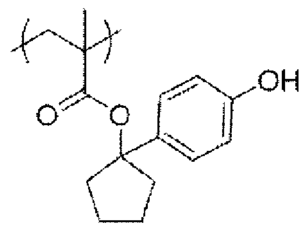
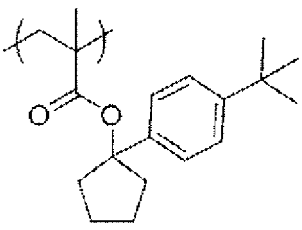
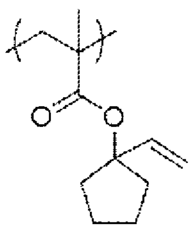
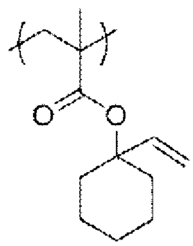
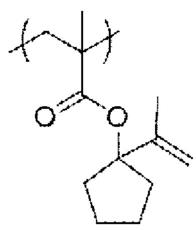
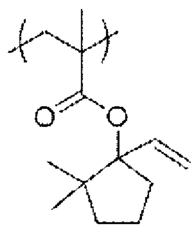
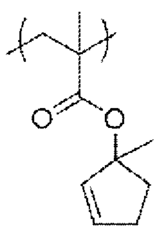
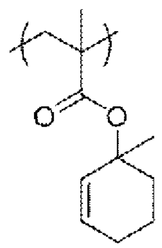
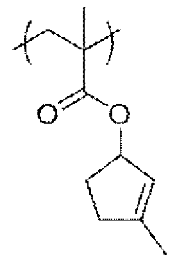
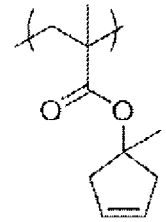
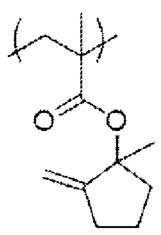
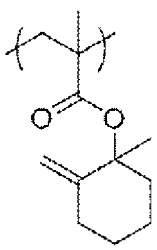
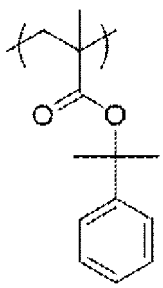
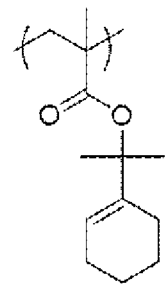
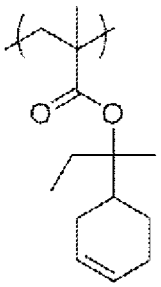
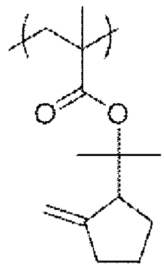
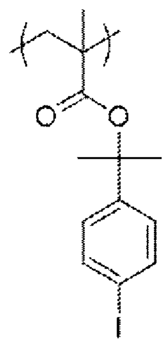
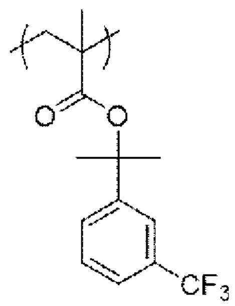

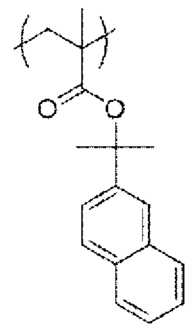
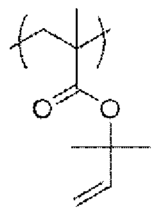
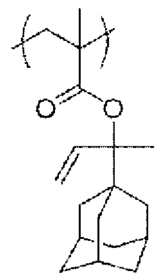
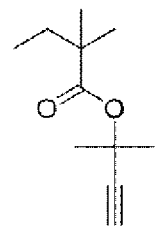

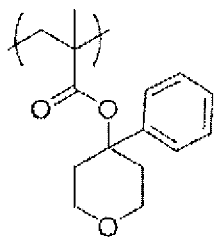
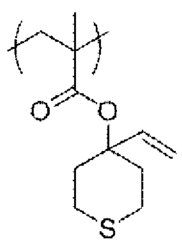
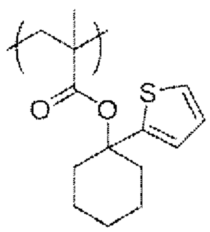
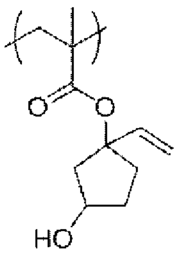

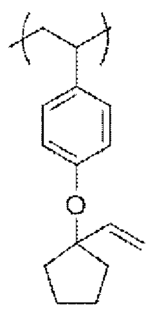
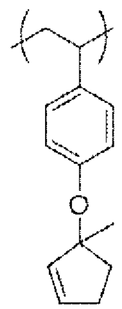
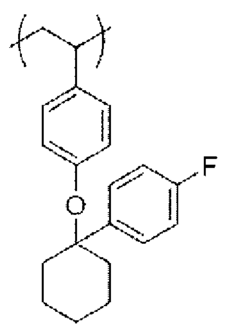
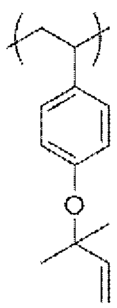

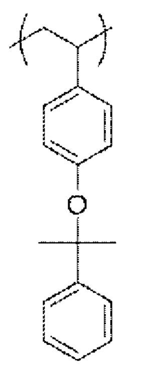
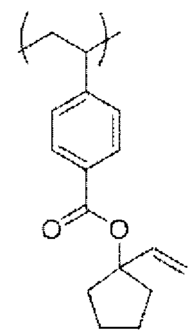
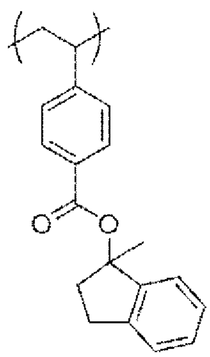
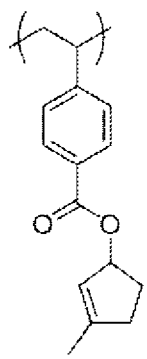

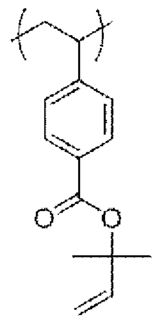
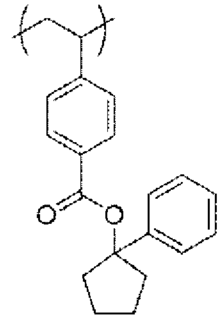
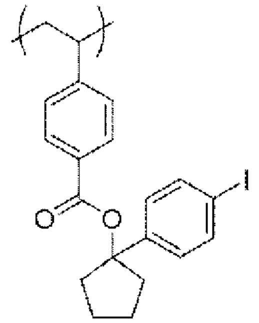
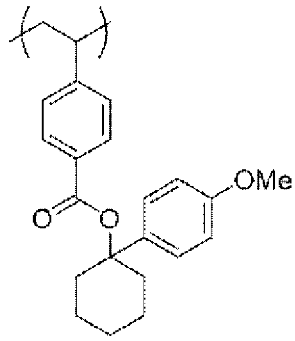

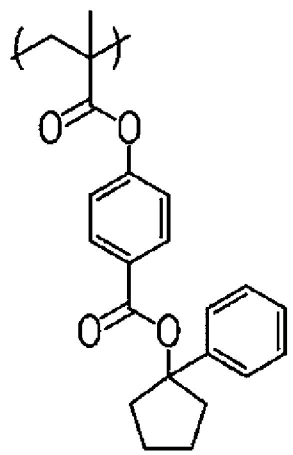
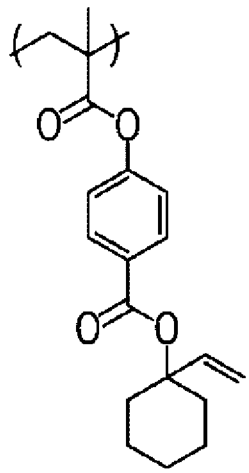
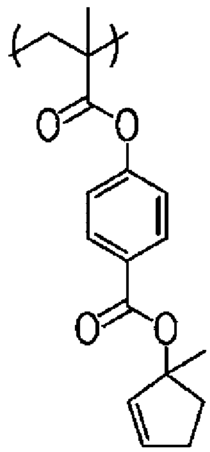

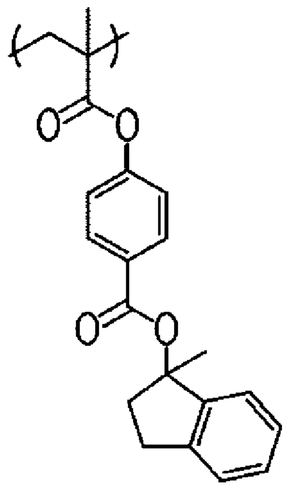
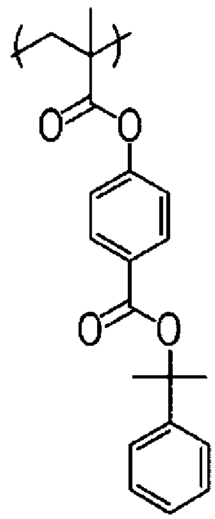
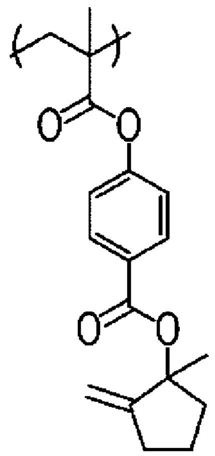

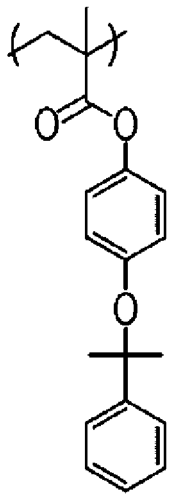 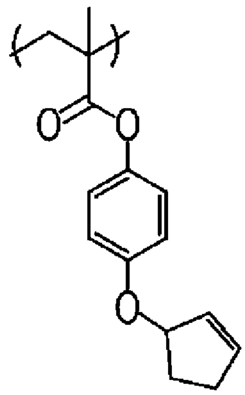

When the resin (A) has a repeating unit having an acid-decomposable group, the content of the repeating unit having an acid-decomposable group relative to all the repeating units in the resin (A) is preferably 15 mol% or more, more preferably 20 mol% or more, and still more preferably 30 mol% or more. The upper limit value relative to all the repeating units in the resin (A) is preferably 90 mol% or less, more preferably 80 mol% or less, still more preferably 70 mol% or less, and particularly preferably 60 mol% or less.
The resin (A) may have only a single species of the repeating unit having an acid-decomposable group, or may have two or more species of the repeating unit having an acid-decomposable group.

Repeating unit having polar group

The resin (A) may have a repeating unit having a polar group.
Examples of the polar group include a hydroxy group, a cyano group, and a carboxy group.
The repeating unit having a polar group is preferably a repeating unit having an alicyclic hydrocarbon structure substituted with a polar group. The repeating unit having a polar group preferably does not have an acid-decomposable group. In the alicyclic hydrocarbon structure substituted with a polar group, the alicyclic hydrocarbon structure is preferably an adamantyl group or a norbornyl group.
Specific examples of the monomer corresponding to the repeating unit having a polar group will be described below, but the present invention is not limited to these specific examples. The following specific examples are described as methacrylic acid ester compounds, but may alternatively be acrylic acid ester compounds.

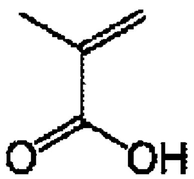 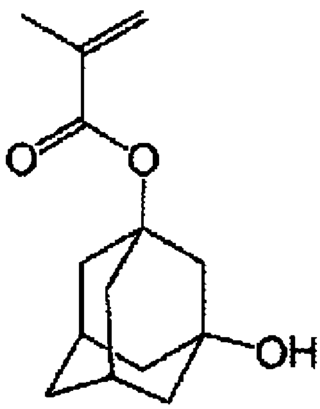 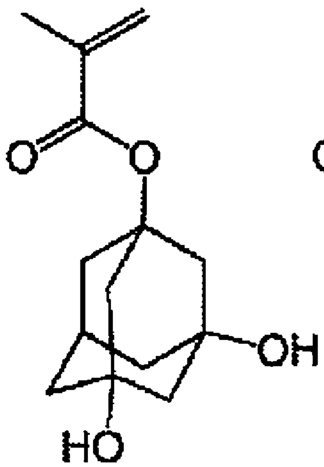 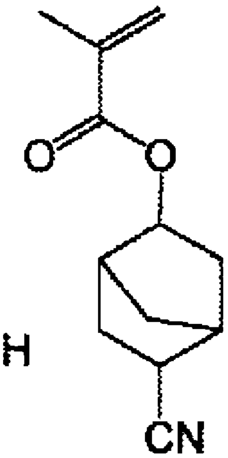

Other specific examples of the repeating unit having a polar group include the constitutional units disclosed in Paragraphs 0415 to 0433 of US2016/0070167A.
The resin (A) may have only a single species of the repeating unit having a polar group, or may have two or more species of the repeating unit having a polar group.
When the resin (A) includes a repeating unit having a polar group, the content thereof relative to all the repeating

units in the resin (A) is preferably 0.1 mol% to 40 mol%, and more preferably 1 to 30 mol%.

The resin (A) may include at least one repeating unit species selected from the group consisting of the following Group A and/or at least one repeating unit species selected from the group consisting of the following Group B.

Group A: a group consisting of the following repeating units (20) to (25)

(20) a repeating unit (described later) having an acid group
(21) a repeating unit (described later) not having an acid-decomposable group or an acid group, but having a fluorine atom, a bromine atom, or an iodine atom
(22) a repeating unit (described later) having a lactone group, a sultone group, or a carbonate group
(23) a repeating unit (described later) having a photoacid generation group
(24) a repeating unit (described later) represented by a formula (V-1) or a formula (V-2) below
(25) a repeating unit for lowering the mobility of the main chain

Note that repeating units represented by a formula (A) to a formula (E) described later correspond to the repeating unit (25) for lowering the mobility of the main chain.

Group B: a group consisting of the following repeating units (30) to (32)

(30) a repeating unit (described later) having at least one group species selected from the group consisting of a lactone group, a sultone group, a carbonate group, a hydroxy group, a cyano group, and an alkali-soluble group
(31) a repeating unit (described later) having an alicyclic hydrocarbon structure and not exhibiting acid-decomposability
(32) a repeating unit (described later) not having a hydroxy group or a cyano group and represented by a formula (III)

The resin (A) preferably has an acid group and preferably includes a repeating unit having an acid group as described later. Note that the definition of the acid group will be described in a later part together with preferred examples of the repeating unit having an acid group. When the resin (A) has an acid group, a better interaction between the resin (A) and the acid generated from the photoacid generator is provided. This results in further suppression of diffusion of the acid to form a pattern having a more square profile.

The resin (A) may have at least one repeating unit species selected from the group consisting of Group A above. When the composition of the present invention is used as an EUV-exposure actinic ray-sensitive or radiation-sensitive resin composition, the resin (A) preferably has at least one repeating unit species selected from the group consisting of Group A above.

The resin (A) may include at least one of a fluorine atom or an iodine atom. When the composition of the present invention is used as an EUV-exposure actinic ray-sensitive or radiation-sensitive resin composition, the resin (A) preferably includes at least one of a fluorine atom or an iodine atom. When the resin (A) includes both of a fluorine atom and an iodine atom, the resin (A) may have a repeating unit including both of a fluorine atom and an iodine atom, or the resin (A) may include two species that are a repeating unit having a fluorine atom and a repeating unit including an iodine atom.

The resin (A) may have a repeating unit having an aromatic group. When the composition of the present invention is used as an EUV-exposure actinic ray-sensitive or radiation-sensitive resin composition, the resin (A) also preferably has a repeating unit having an aromatic group.

The resin (A) may also have at least one repeating unit species selected from the group consisting of Group B above. When the composition of the present invention is used as an ArF actinic ray-sensitive or radiation-sensitive resin composition, the resin (A) preferably has at least one repeating unit species selected from the group consisting of Group B above.

Note that, when the composition of the present invention is used as an ArF actinic ray-sensitive or radiation-sensitive resin composition, the resin (A) preferably does not include a fluorine atom or a silicon atom.

When the composition of the present invention is used as an ArF actinic ray-sensitive or radiation-sensitive resin composition, the resin (A) preferably does not have an aromatic group.

Repeating unit having acid group

The resin (A) may have a repeating unit having an acid group.

The acid group is preferably an acid group having a pKa of 13 or less. The acid group preferably has an acid dissociation constant of 13 or less, more preferably 3 to 13, and still more preferably 5 to 10.

When the resin (A) has an acid group having a pKa of 13 or less, the content of the acid group in the resin (A) is not particularly limited, but is often 0.2 to 6.0 mmol/g. In particular, preferred is 0.8 to 6.0 mmol/g, more preferred is 1.2 to 5.0 mmol/g, and still more preferred is 1.6 to 4.0 mmol/g. When the content of the acid group is within such a range, development suitably proceeds to form a pattern having a good profile at high resolution.

The acid group is preferably, for example, a carboxyl group, a phenolic hydroxy group, a fluoroalcohol group (preferably a hexafluoroisopropanol group), a sulfonic group, a sulfonamide group, or an isopropanol group.

In the hexafluoroisopropanol group, one or more (preferably one to two) of the fluorine atoms may be substituted with groups other than fluorine atoms (such as alkoxycarbonyl groups).

The acid group is also preferably $-C(CF_3)(OH)-CF_2-$ formed in this manner. Alternatively, one or more of the fluorine atoms may be substituted with groups other than fluorine atoms, to form a ring including $-C(CF_3)(OH)-CF_2-$.

The repeating unit having an acid group is preferably a repeating unit different from the above-described repeating unit having a structure in which a polar group is protected with a group that leaves by the action of an acid and repeating units described later and having a lactone group, a sultone group, or a carbonate group.

The repeating unit having an acid group may have a fluorine atom or an iodine atom.

Examples of the repeating unit having an acid group include the following repeating units.

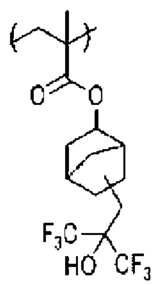
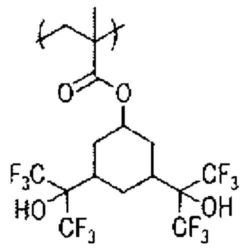
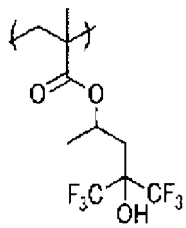

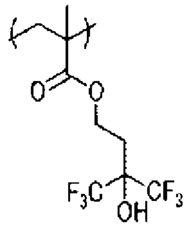
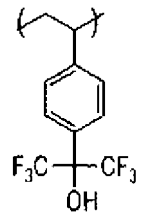
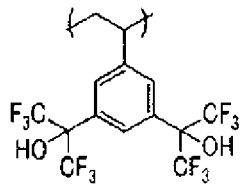

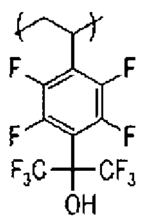
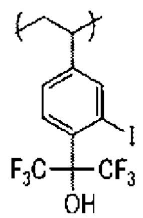
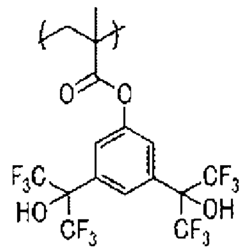
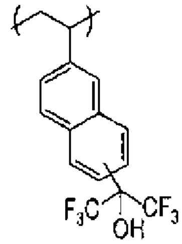

The resin (A) preferably has a repeating unit having a phenolic hydroxy group.

The repeating unit having a phenolic hydroxy group is preferably a repeating unit represented by a formula (Y) below.

The repeating unit having an acid group is preferably a repeating unit represented by the formula (Y) below.

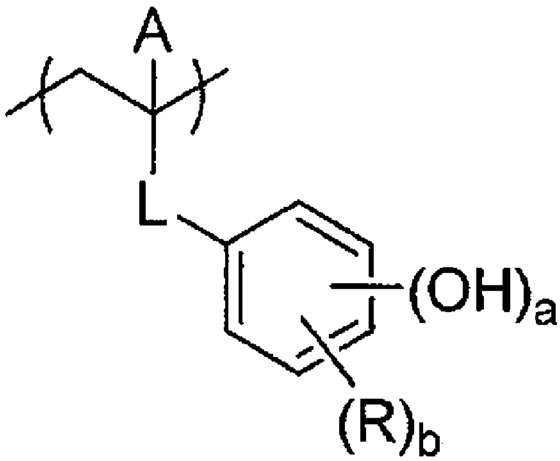

(Y)

In the formula (Y),

A represents a hydrogen atom, an alkyl group, a cycloalkyl group, a halogen atom, or a cyano group.
L represents a single bond or a divalent linking group having an oxygen atom.
R represents a halogen atom, an alkyl group, a cycloalkyl group, an aryl group, an alkenyl group, an aralkyl group, an alkoxy group, an alkylcarbonyloxy group, an alkylsulfonyloxy group, an alkyloxycarbonyl group, or an aryloxycarbonyl group; when there are a plurality of R's, they may be the same or different. When there are a plurality of R's, they may together form a ring. R is preferably a hydrogen atom.
a represents an integer of 1 to 3.
b represents an integer of 0 to (5 - a).

In the formula (Y), R is preferably a hydrogen atom. L is preferably a single bond.
The following are examples of the repeating unit having an acid group. In the formulas, a represent an integer of 1 to 3.
R represent a hydrogen atom or a methyl group.

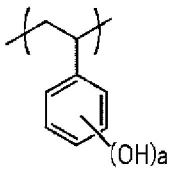

(B-1)

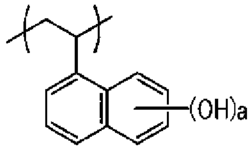

(B-2)

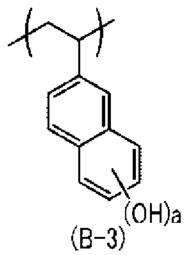

(B-3)

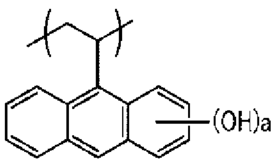

(B-4)

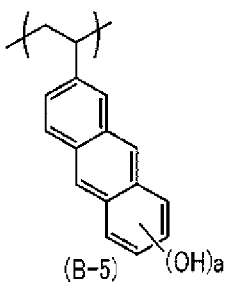

(B-5)

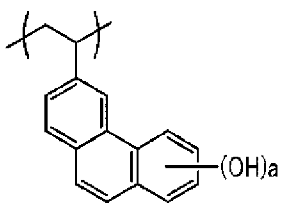

(B-6)

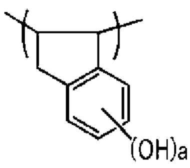
(B-7)

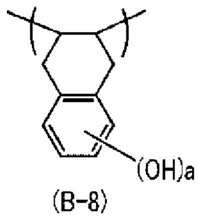
(B-8)

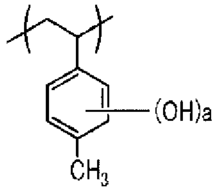
(B-9)

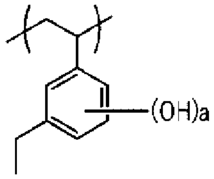
(B-10)

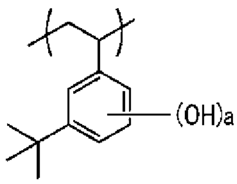
(B-11)

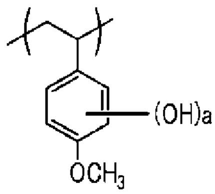
(B-12)

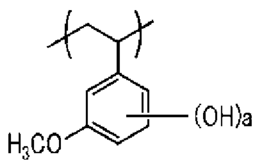
(B-13)

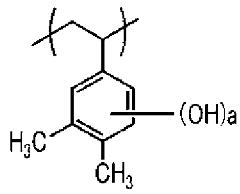
(B-14)

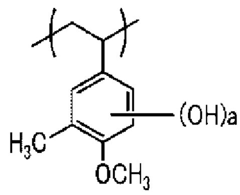
(B-15)

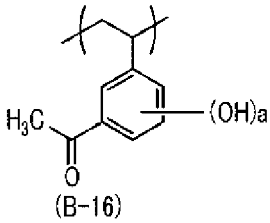
(B-16)

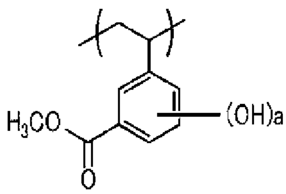
(B-17)

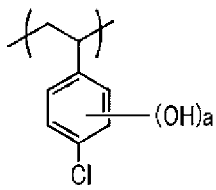
(B-18)

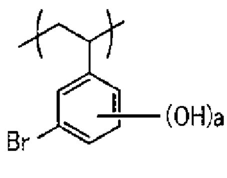
(B-19)

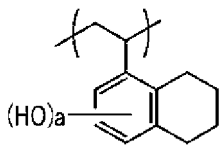
(B-20)

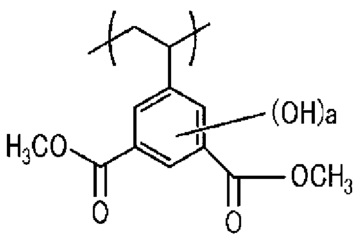
(B-21)

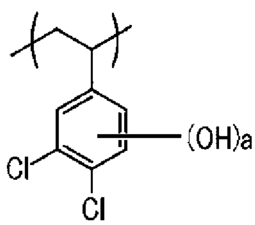
(B-22)

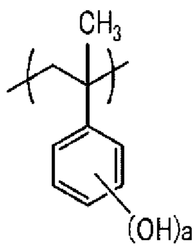
(B-23)

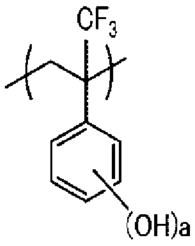

(B-24)

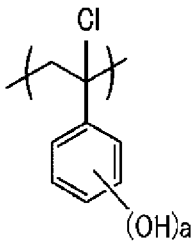

(B-25)

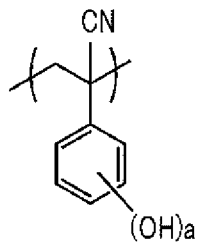

(B-26)

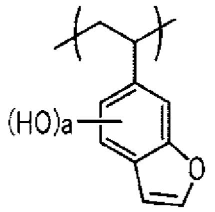

(B-27)

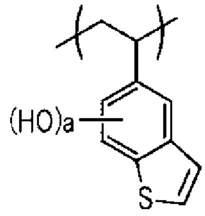

(B-28)

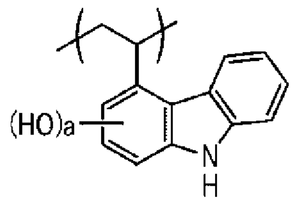

(B-29)

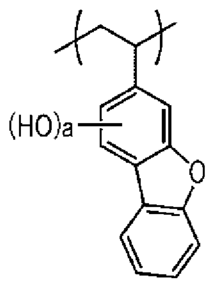

(B-30)

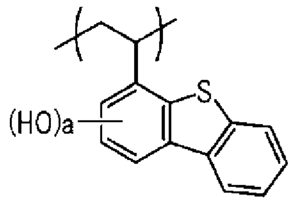

(B-31)

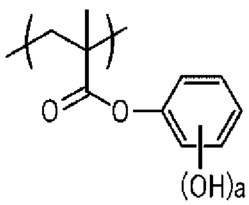

(B-32)

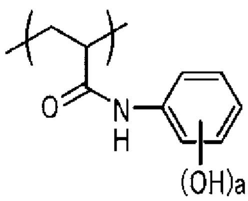

(B-33)

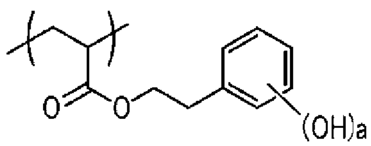

(B-34)

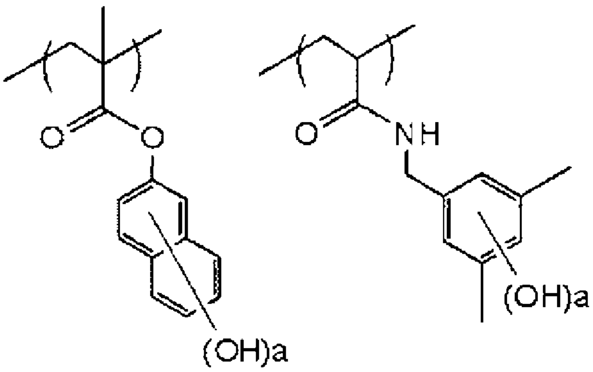

(B-35) (B-36)

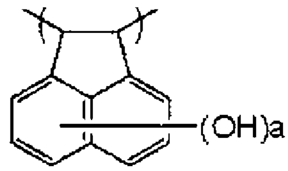

(B-37)

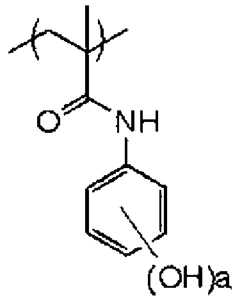

(B-38)

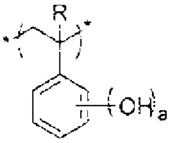

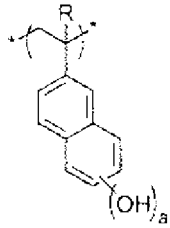

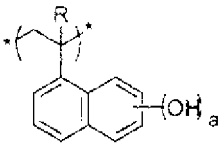

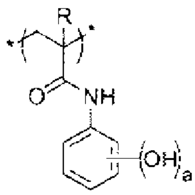

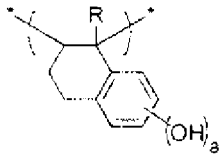

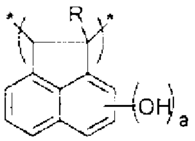

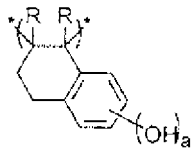

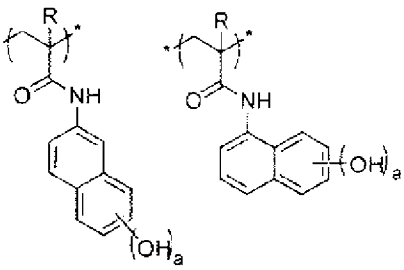

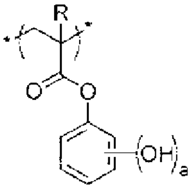

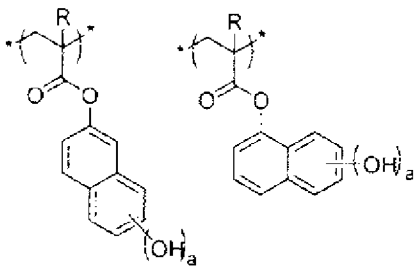

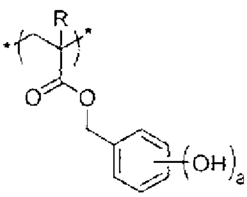

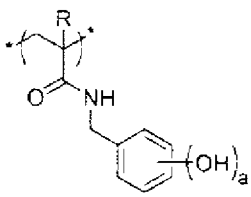

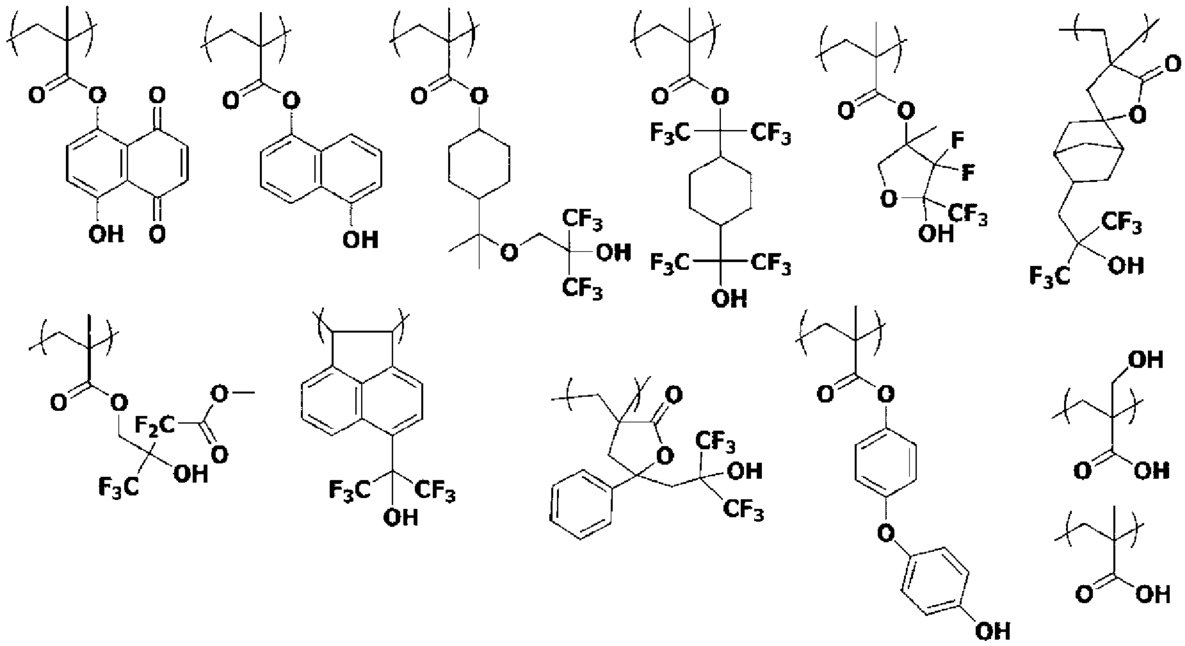

The content of the repeating unit having an acid group relative to all the repeating units in the resin (A) is preferably 10 mol% or more, and more preferably 15 mol% or more. The upper limit value relative to all the repeating units in the resin (A) is preferably 70 mol% or less, more preferably 65 mol% or less, and still more preferably 60 mol% or less.

The resin (A) may have only a single species of the repeating unit having an acid group, or may have two or more species of the repeating unit having an acid group.

Repeating unit not having acid-decomposable group or acid group, but having fluorine atom, bromine atom, or iodine atom

The resin (A) may have, in addition to the above-described <repeating unit having an acid-decomposable group> and <repeating unit having an acid group>, a repeating unit not having an acid-decomposable group or an acid group, but having a fluorine atom, a bromine atom, or an iodine atom (hereafter, also referred to as unit X). This <repeating unit not having an acid-decomposable group or an acid group, but having a fluorine atom, a bromine atom, or an iodine atom> is preferably different from other repeating unit species belonging to Group A such as the <repeating unit having a lactone group, a sultone group, or a carbonate group> and the <repeating unit having a photoacid generation group> described later.

The unit X is preferably a repeating unit represented by a formula (C).

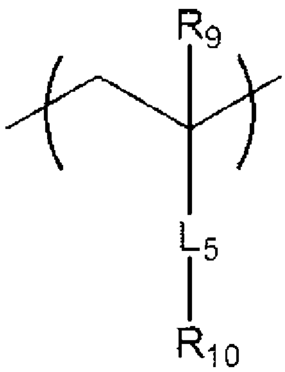

(C)

$L_5$ represents a single bond or an ester group. $R_9$ represents a hydrogen atom or an alkyl group that may have a fluorine atom or an iodine atom. $R_{10}$ represents a hydrogen atom, an alkyl group that may have a fluorine atom or an iodine atom, a cycloalkyl group that may have a fluorine atom or an iodine atom, an aryl group that may have a fluorine atom or an iodine atom, or a group that is a combination of the foregoing.

The following are examples of the repeating unit having a fluorine atom or an iodine atom.

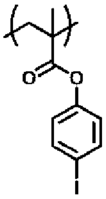
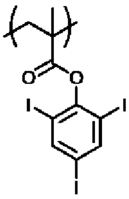
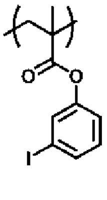
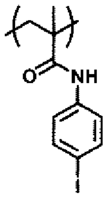
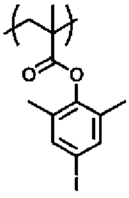
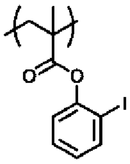
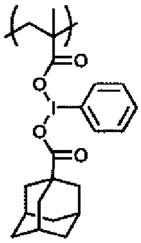
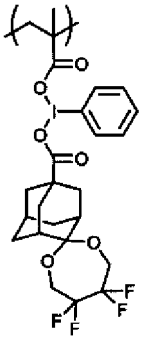
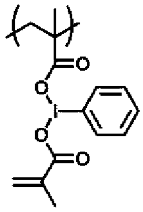

The unit X content relative to all the repeating units in the resin (A) is preferably 0 mol% or more, more preferably 5 mol% or more, and still more preferably 10 mol% or more. The upper limit value relative to all the repeating units in the resin (A) is preferably 50 mol% or less, more preferably 45 mol% or less, and still more preferably 40 mol% or less.
The resin (A) may have only a single species of the repeating unit having a fluorine atom, a bromine atom, or an iodine atom, or two or more species of the repeating unit having a fluorine atom, a bromine atom, or an iodine atom.
Of the repeating units of the resin (A), the total content of the repeating unit including at least one of a fluorine atom, a bromine atom, or an iodine atom relative to all the repeating units of the resin (A) is preferably 10 mol% or more, more preferably 20 mol% or more, still more preferably 30 mol% or more, and particularly preferably 40 mol% or more. The upper limit value is not particularly limited, but is, for example, relative to all the repeating units of the resin (A), 100 mol% or less.
Note that examples of the repeating unit including at least one of a fluorine atom, a bromine atom, or an iodine atom include a repeating unit having a fluorine atom, a bromine atom, or an iodine atom and having an acid-decomposable group, a repeating unit having a fluorine atom, a bromine atom, or an iodine atom and having an acid group, and a repeating unit having a fluorine atom, a bromine atom, or an iodine atom.

Repeating unit having at least one group species selected from the group consisting of lactone group, sultone group, carbonate group, hydroxy group, cyano group, and alkali-soluble group

The resin (A) may have a repeating unit having at least one group species selected from the group consisting of a lactone group, a sultone group, a carbonate group, a hydroxy group, a cyano group, and an alkali-soluble group.
The resin (A) preferably has a repeating unit having at least one species selected from the group consisting of a lactone group, a sultone group, and a carbonate group.
First, the repeating unit having at least one species selected from the group consisting of a lactone group, a sultone group, and a carbonate group (hereafter, also collectively referred to as "repeating unit having a lactone group, a sultone group, or a carbonate group") will be described.
The repeating unit having a lactone group, a sultone group, or a carbonate group also preferably does not have acid groups such as a hydroxy group and a hexafluoropropanol group.
The lactone group or the sultone group has a lactone structure or a sultone structure. The lactone structure or the sultone structure is preferably a 5- to 7-membered lactone structure or a 5- to 7-membered sultone structure. In particular, more preferred is a 5- to 7-membered lactone structure to which another ring structure is fused so as to form a bicyclo structure or a spiro structure, or a 5- to 7-membered sultone structure to which another ring structure is fused so as to form a bicyclo structure or a spiro structure.
The resin (A) preferably has a repeating unit having a lactone group or a sultone group provided by withdrawing, from ring-member atoms of the lactone structure represented by any one of formulas (LC1-1) to (LC1-21) below or the sultone structure represented by any one of formulas (SL1-1) to (SL1-3) below, one or more hydrogen atoms.
The lactone group or the sultone group may be directly bonded to the main chain. For example, ring-member atoms of a lactone group or a sultone group may constitute the main chain of the resin (A).

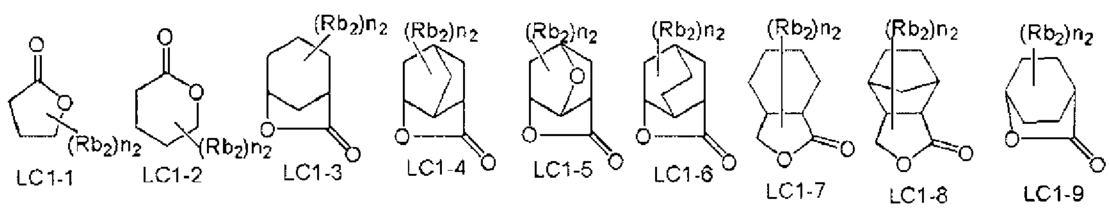

LC1-1 LC1-2 LC1-3 LC1-4 LC1-5 LC1-6 LC1-7 LC1-8 LC1-9

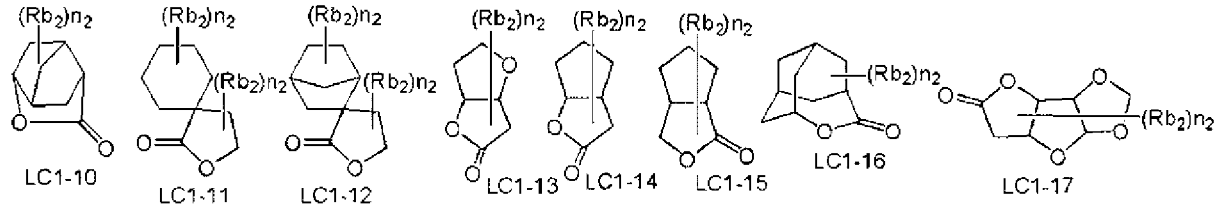

LC1-10 LC1-11 LC1-12 LC1-13 LC1-14 LC1-15 LC1-16 LC1-17

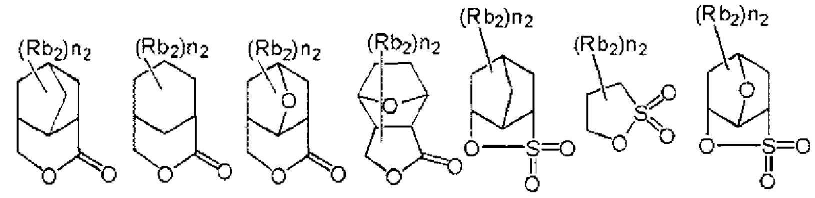

LC1-18 LC1-19 LC1-20 LC1-21 SL1-1 SL1-2 SL1-3

The lactone structure or the sultone structure may have a substituent ($Rb_2$). Preferred examples of the substituent ($Rb_2$) include an alkyl group having 1 to 8 carbon atoms, a cycloalkyl group having 4 to 7 carbon atoms, an alkoxy group having 1 to 8 carbon atoms, an alkoxycarbonyl group having 1 to 8 carbon atoms, a carboxyl group, a halogen atom, a cyano group, and an acid-decomposable group. n2 represent an integer of 0 to 4. When n2 is 2 or more, the plurality of $Rb_2$'s present may be different, and the plurality of $Rb_2$'s present may be bonded together to form a ring.

The repeating unit having a group including the lactone structure represented by any one of the formulas (LC1-1) to (LC1-21) or the sultone structure represented by any one of the formulas (SL1-1) to (SL1-3) may be, for example, a repeating unit represented by the following formula (AI-2).

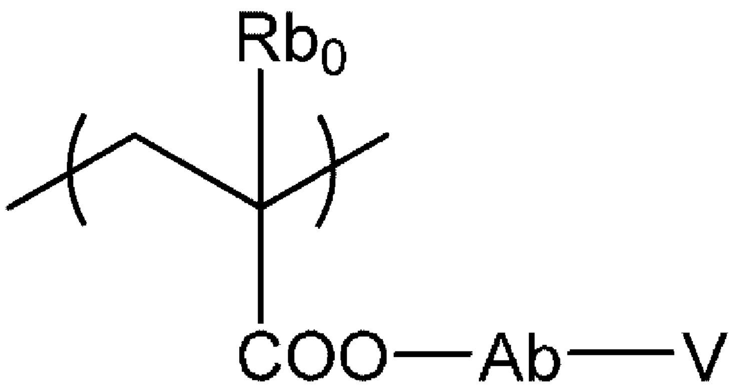

**(AI-2)**

In the formula (AI-2), $Rb_0$ represents a hydrogen atom, a halogen atom, or an alkyl group having 1 to 4 carbon atoms. Preferred examples of the substituent that the alkyl group of $Rb_0$ may have include a hydroxy group and a halogen atom.

Examples of the halogen atom of $Rb_0$ include a fluorine atom, a chlorine atom, a bromine atom, and an iodine atom. $Rb_0$ is preferably a hydrogen atom or a methyl group.

Ab represents a single bond, an alkylene group, a divalent linking group having a monocyclic or polycyclic alicyclic hydrocarbon structure, an ether group, an ester group, a carbonyl group, a carboxyl group, or a divalent linking group that is a combination of the foregoing. In particular, Ab is preferably a single bond or a linking group represented by $-Ab_1-CO_2-$. $Ab_1$ is a linear or branched alkylene group or a monocyclic or polycyclic cycloalkylene group, and preferably a methylene group, an ethylene group, a cyclohexylene group, an adamantylene group, or a norbomylene group.

V represents a group formed by withdrawing, from a ring-member atom of the lactone structure represented by any one of the formulas (LC1-1) to (LC1-21), a single hydrogen atom, or a group formed by withdrawing, from a ring-member atom of the sultone structure represented by any one of the formulas (SL1-1) to (SL1-3), a single hydrogen atom.

When the repeating unit having a lactone group or a sultone group has an optical isomer, any optical isomer may be used. A single optical isomer may be used alone, or a plurality of optical isomers may be used in combination. In the case of mainly using one of the optical isomers, its optical purity (ee) is preferably 90 or more, and more preferably 95 or more.

The carbonate group is preferably a cyclic carbonate group (cyclic carbonic acid ester group).

The repeating unit having a cyclic carbonic acid ester group is preferably a repeating unit represented by the following formula (A-1).

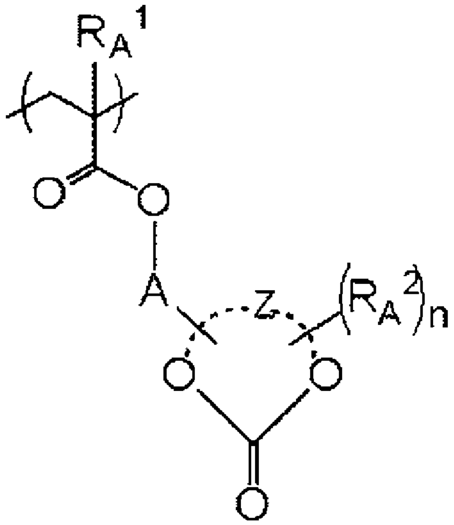

(A-1)

In the formula (A-1), $R_A{}^1$ represents a hydrogen atom, a halogen atom, or a monovalent organic group (preferably a methyl group).

n represents an integer of 0 or more.

$R_A{}^2$ represents a substituent. When n is 2 or more, the plurality of $R_A{}^2$'s present may be the same or different.

A represents a single bond or a divalent linking group. The divalent linking group is preferably an alkylene group, a divalent linking group having a monocyclic or polycyclic alicyclic hydrocarbon structure, an ether group, an ester group, a carbonyl group, a carboxyl group, or a divalent linking group that is a combination of the foregoing.

Z represents an atomic group that forms, together with the group represented by -O-CO-O- in the formula, a monocycle or a polycycle.

The following are examples of the repeating unit having a lactone group, a sultone group, or a carbonate group. In the formulas, Rx represent a hydrogen atom, $-CH_3$, $-CH_2OH$, or $-CF_3$.

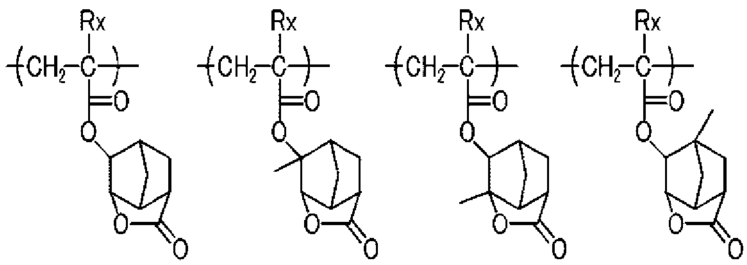

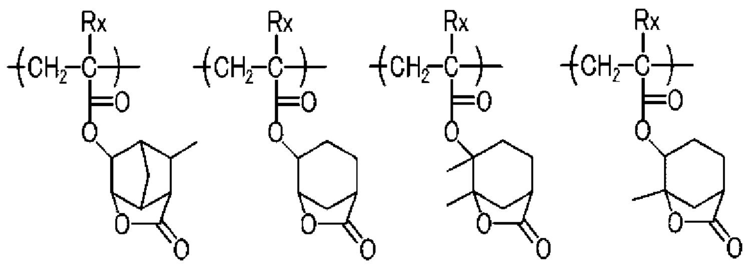

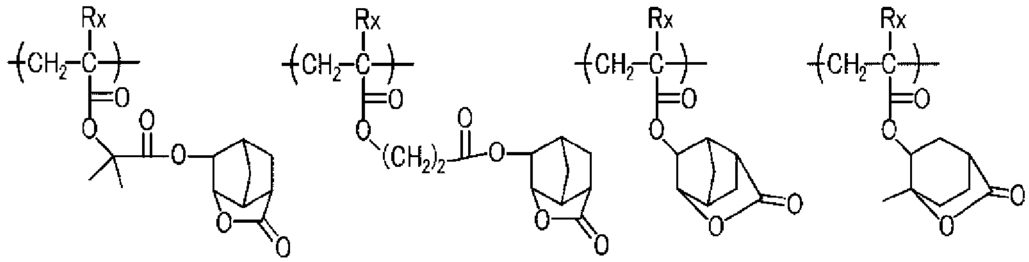

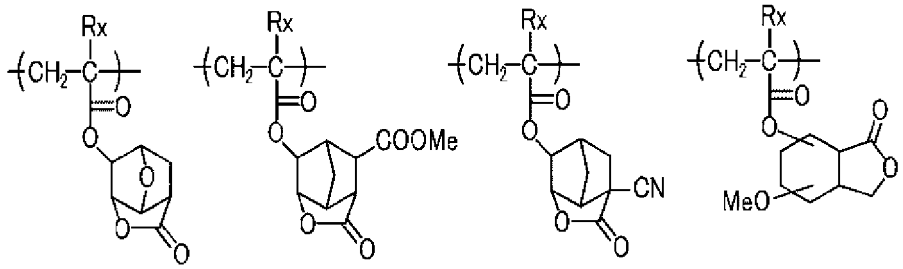

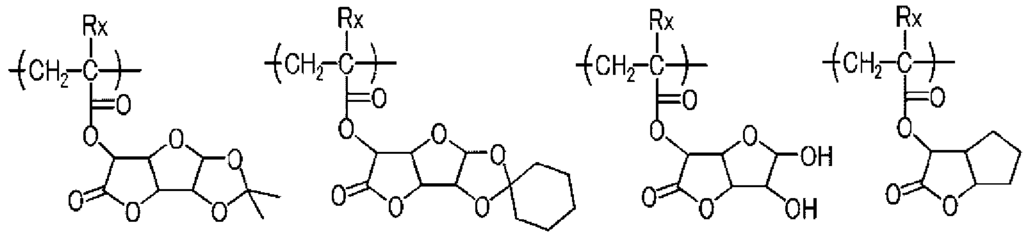

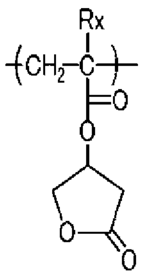

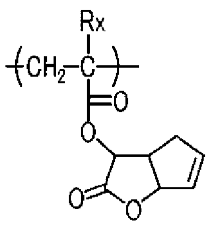

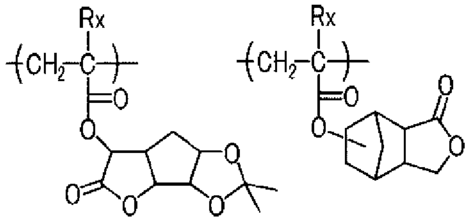

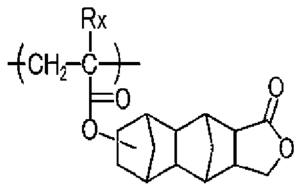

(In the formulas, Rx are H, $CH_3$, $CH_2OH$, or $CF_3$.)

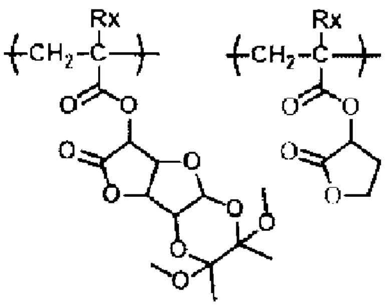

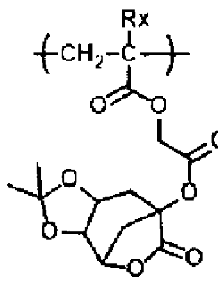

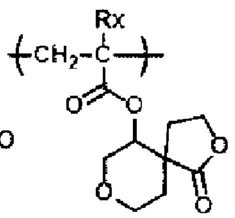

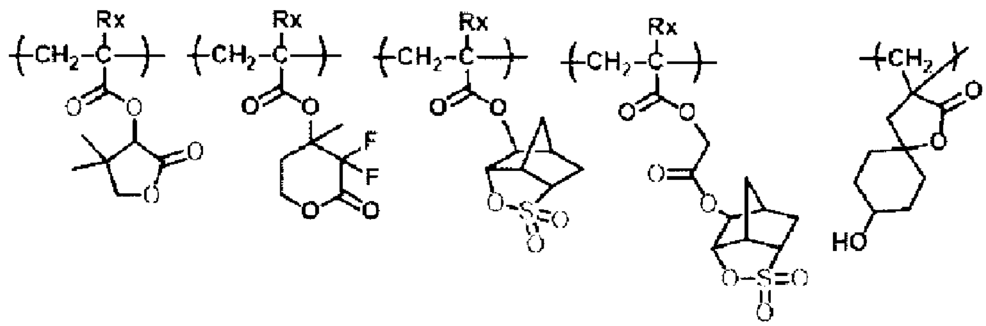

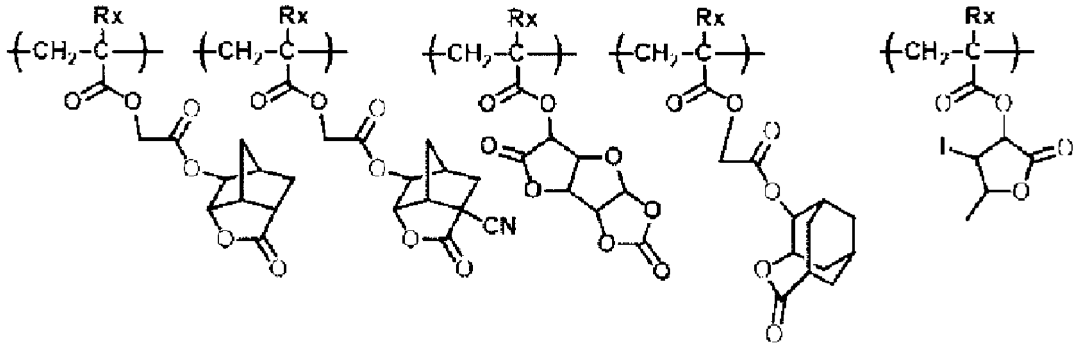

(In the formulas, Rx represent H, $CH_3$, $CH_2OH$, or $CF_3$.)

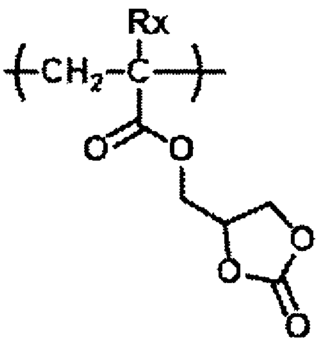

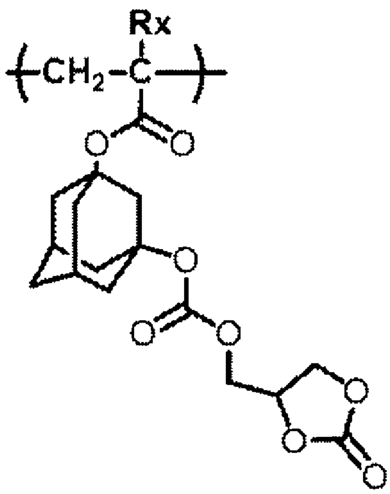

Hereinafter, the repeating unit having a hydroxy group or a cyano group will be described.

The resin (A) may have a repeating unit having a hydroxy group or a cyano group. This results in improvement in adhesiveness to the substrate and affinity for the developer.

The repeating unit having a hydroxy group or a cyano group is preferably a repeating unit having an alicyclic hydrocarbon structure substituted with a hydroxy group or a cyano group.

The repeating unit having a hydroxy group or a cyano group preferably does not have an acid-decomposable group. Examples of the repeating unit having a hydroxy group or a cyano group include those described in Paragraphs [0081] to [0084] of JP2014-098921A.

Hereinafter, the repeating unit having an alkali-soluble group will be described.

The resin (A) may have a repeating unit having an alkali-soluble group.

The alkali-soluble group may be a carboxyl group, a sulfonamide group, a sulfonylimide group, a bissulfonylimide group, or an aliphatic alcohol group substituted, at the $\alpha$ position, with an electron-withdrawing group (for example, a hexafluoroisopropanol group), and is preferably a carboxyl group. When the resin (A) includes the repeating unit having an alkali-soluble group, increased resolution is provided in the contact hole application. Examples of the repeating unit

having an alkali-soluble group include those described in Paragraphs [0085] and [0086] of JP2014-098921A.

The content of the repeating unit having at least one group species selected from the group consisting of a lactone group, a sultone group, a carbonate group, a hydroxy group, a cyano group, and an alkali-soluble group relative to all the repeating units in the resin (A) is preferably 1 mol% or more, and more preferably 10 mol% or more. The upper limit value relative to all the repeating units in the resin (A) is preferably 85 mol% or less, more preferably 80 mol% or less, still more preferably 70 mol% or less, and particularly preferably 60 mol% or less.

The resin (A) may have only one species of the repeating unit having at least one group species selected from the group consisting of a lactone group, a sultone group, a carbonate group, a hydroxy group, a cyano group, and an alkali-soluble group, or two or more species of the repeating unit having at least one group species selected from the group consisting of a lactone group, a sultone group, a carbonate group, a hydroxy group, a cyano group, and an alkali-soluble group.

Repeating unit having photoacid generation group

The resin (A) may have, as a repeating unit other than the above-described repeating units, a repeating unit having a group that generates an acid upon irradiation with an actinic ray or a radiation (hereafter, also referred to as "photoacid generation group").

The repeating unit having a photoacid generation group may be a repeating unit represented by a formula (4).

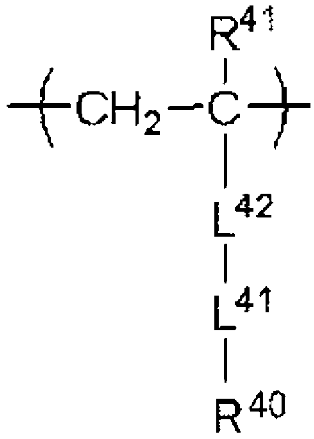

(4)

$R^{41}$ represents a hydrogen atom or a methyl group. $L^{41}$ represents a single bond or a divalent linking group. $L^{42}$ represents a divalent linking group. $R^{40}$ represents a structural moiety that is decomposed upon irradiation with an actinic ray or a radiation to generate an acid in the side chain.

The following are examples of the repeating unit having a photoacid generation group.

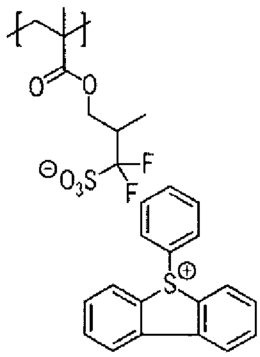

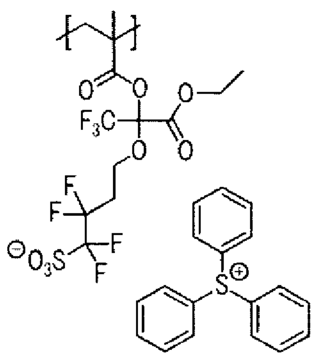

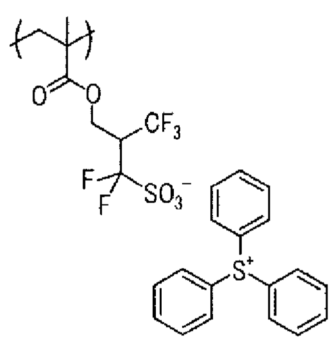

Other examples of the repeating unit represented by the formula (4) include the repeating units described in Paragraphs [0094] to [0105] of JP2014-041327A and the repeating units described in Paragraph [0094] of WO2018/193954A.

The content of the repeating unit having a photoacid generation group relative to all the repeating units in the resin (A) is preferably 1 mol% or more, and more preferably 5 mol% or more. The upper limit value relative to all the repeating units in the resin (A) is preferably 40 mol% or less, more preferably 35 mol% or less, and still more preferably

30 mol% or less.

Repeating unit represented by formula (V-1) or formula (V-2) below

The resin (A) may have a repeating unit represented by a formula (V-1) below or a formula (V-2) below.
The repeating unit represented by the formula (V-1) below or the formula (V-2) below is preferably a repeating unit different from the above-described repeating units.

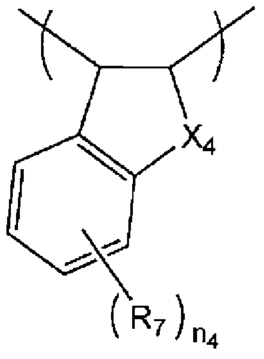

(V-1)

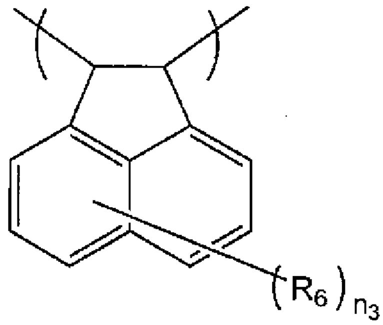

(V-2)

In the formulas,

$R_6$ and $R_7$ each independently represent a hydrogen atom, a hydroxy group, an alkyl group, an alkoxy group, an acyloxy group, a cyano group, a nitro group, an amino group, a halogen atom, an ester group (-OCOR or -COOR: R is an alkyl group or a fluorinated alkyl group having 1 to 6 carbon atoms), or a carboxyl group. The alkyl group is preferably a linear, branched, or cyclic alkyl group having 1 to 10 carbon atoms.
$n_3$ represents an integer of 0 to 6.
$n_4$ represents an integer of 0 to 4.
$X_4$ is a methylene group, an oxygen atom, or a sulfur atom.

The following are examples of the repeating unit represented by the formula (V-1) or (V-2).
Examples of the repeating unit represented by the formula (V-1) or (V-2) include the repeating units described in Paragraph [0100] of WO2018/193954A.

Repeating unit for lowering mobility of main chain

Examples of the repeating unit for lowering the mobility of the main chain include those described in Paragraphs [0120] to [0151] of WO2020/004306A.

Repeating unit having alicyclic hydrocarbon structure and not exhibiting acid-decomposability

The resin (A) may have a repeating unit having an alicyclic hydrocarbon structure and not exhibiting acid-decomposability. This results in, during liquid immersion exposure, a reduction in leaching of, from the resist film to the immersion liquid, low-molecular-weight components. Examples of the repeating unit having an alicyclic hydrocarbon structure and not exhibiting acid-decomposability include a repeating unit derived from 1-adamantyl (meth)acrylate, diadamantyl (meth)acrylate, tricyclodecanyl (meth)acrylate, or cyclohexyl (meth)acrylate.

Repeating unit not having hydroxy group or cyano group and represented by formula (III)

The resin (A) may have a repeating unit not having a hydroxy group or a cyano group and represented by a formula (III).

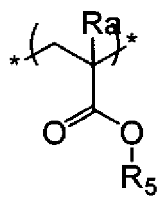
(III)

In the formula (III), $R_5$ represents a hydrocarbon group having at least one ring structure and not having a hydroxy group or a cyano group.

Ra represents a hydrogen atom, an alkyl group, or a $-CH_2-O-Ra_2$ group. In the formula, $Ra_2$ represents a hydrogen atom, an alkyl group, or an acyl group.

Examples of the repeating unit not having a hydroxy group or a cyano group and represented by the formula (III) include those described in Paragraphs [0087] to [0094] of JP2014-098921A.

Other repeating unit

Furthermore, the resin (A) may have another repeating unit other than the above-described repeating units.

For example, the resin (A) may have a repeating unit selected from the group consisting of a repeating unit having an oxathiane ring group, a repeating unit having an oxazolone ring group, a repeating unit having a dioxane ring group, and a repeating unit having a hydantoin ring group.

The following are specific examples of the other repeating unit other than the above-described repeating units.

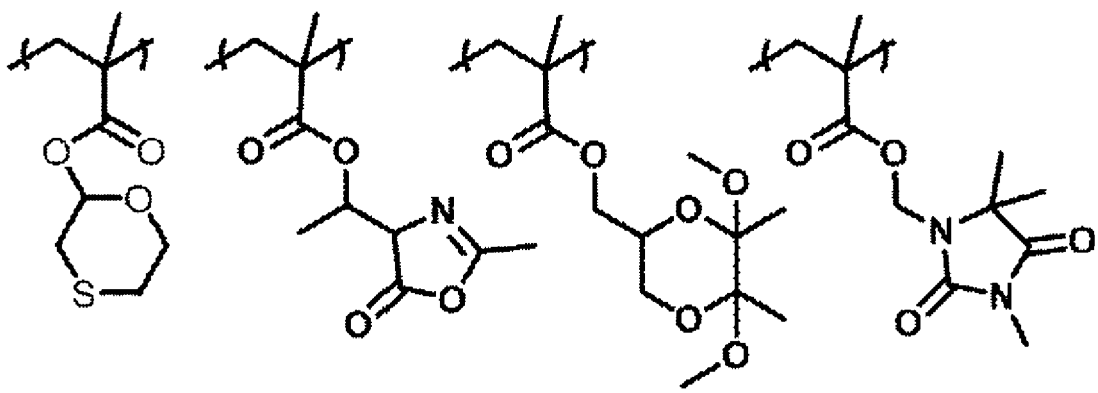

The resin (A) may have, in addition to the above-described repeating structural units, various repeating structural units for the purpose of adjusting, for example, dry etching resistance, standard developer droplet property, substrate adhesiveness, resist profile, resolution, heat resistance, and sensitivity.

Note that the resin (A) may have a group represented by $-SF_4R$, or may not have a group represented by $-SF_4R$. Here, R represents a hydrogen atom, a halogen atom, or a monovalent substituent. For R, specific examples and the like of the halogen atom and the monovalent substituent are the same as those in the ionic compound described later in detail.

When the resin (A) has a group represented by $-SF_4R$, the resin (A) is preferably a resin having a repeating unit having $-SF_4R$, and such repeating units having $-SF_4R$ may correspond to the above-described various repeating units that the resin (A) may have. Specific examples of such repeating units include, in the above-described various repeating units that the resin (A) may have, repeating units in which a group represented by $-SF_4R$ is included.

The resin (A) may have only one species of the repeating unit having a group represented by $-SF_4R$, or two or more species of the repeating unit having a group represented by $-SF_4R$.

For the resin (A), particularly when the composition is used as an ArF actinic ray-sensitive or radiation-sensitive resin composition, all the repeating units are preferably constituted by a repeating unit derived from a compound having an ethylenically unsaturated bond. In particular, all the repeating units are also preferably constituted by a (meth)acrylate-based repeating unit. In the case in which all the repeating units are constituted by a (meth)acrylate-based repeating unit, all the repeating units may be a methacrylate-based repeating unit, all the repeating units may be an acrylate-based repeating unit, or all the repeating units may be a methacrylate-based repeating unit and an acrylate-based repeating unit; the acrylate-based repeating unit content relative to all the repeating units is preferably 50 mol% or less.

The resin (A) can be synthesized by standard procedures (for example, radical polymerization).

The resin (A) has a weight-average molecular weight of, as a polystyrene-equivalent value determined by GPC method, preferably 30,000 or less, more preferably 1,000 to 30,000, still more preferably 3,000 to 30,000, and particularly preferably 5,000 to 15,000.

The resin (A) has a dispersity (molecular weight distribution) of preferably 1 to 5, more preferably 1 to 3, still more preferably 1.2 to 3.0, and particularly preferably 1.2 to 2.0. As the dispersity lowers, the resolution becomes higher, the resist profile becomes better, the sidewalls of the resist pattern become smoother, and the roughness performance becomes higher.

In the composition of the present invention, the content of the resin (A) relative to the total solid content of the composition is preferably 30.0 to 99.9 mass%, more preferably 40.0 to 90.0 mass%, and still more preferably 50.0 to 90.0 mass%.

Such resins (A) may be used alone or in combination of two or more thereof.

Ionic compound (Y) having, in at least one of cationic moiety or anionic moiety, group represented by $-SF_4R$

The composition of the present invention includes an ionic compound (Y) having, in at least one of a cationic moiety or an anionic moiety, a group represented by $-SF_4R$ (hereinafter, also referred to as "ionic compound (Y)" or "compound (Y)"). Here, R represents a hydrogen atom, a halogen atom, or an organic group, and the same applies hereinafter.

The ionic compound (Y) is a compound having an anionic moiety and a cationic moiety, and is a compound having, in at least one of the cationic moiety or the anionic moiety, a group represented by $-SF_4R$.

The ionic compound (Y) may be an ionic compound having, in the cationic moiety, a group represented by $-SF_4R$, or may be an ionic compound having, in the anionic moiety, a group represented by $-SF_4R$.

Alternatively, the ionic compound (Y) may be an ionic compound having, in the cationic moiety and the anionic moiety, a group represented by $-SF_4R$.

When the ionic compound (Y) has a plurality of groups represented by $-SF_4R$, the plurality of groups represented by $-SF_4R$ may be the same or different.

In the group represented by $-SF_4R$, R represents a hydrogen atom, a halogen atom, or a monovalent substituent.

Examples of the halogen atom include a fluorine atom, a chlorine atom, a bromine atom, and an iodine atom, and preferred is a fluorine atom.

The monovalent substituent is not particularly limited, but examples thereof include organic groups. The organic groups are not particularly limited, but examples thereof include organic groups having 1 to 30 carbon atoms.

The group represented by $-SF_4R$ is preferably $-SF_5$.

In a preferred embodiment, the ionic compound (Y) is preferably an ionic compound having, in the cationic moiety, a group represented by $-SF_4R$.

The cationic moiety of the ionic compound is preferably a sulfonium cation or an iodonium cation.

In a preferred embodiment, the ionic compound (Y) is preferably an ionic compound having, in the anionic moiety, a group represented by $-SF_4R$.

The cationic moiety in the ionic compound is preferably an organic anion. The organic anion is not particularly limited, but examples thereof include $M^+$ described later.

The ionic compound (Y) is typically an onium salt, and examples thereof include a sulfonium salt and an iodonium salt.

From the viewpoint of improving LWR (Line Width Roughness), in a preferred embodiment, the ionic compound (Y) does not have a perfluoroalkyl group.

From the viewpoint of improving LWR, in another preferred embodiment, the ionic compound (Y) is also an ionic compound (Y') having an acid-decomposable group in the cationic moiety.

The acid-decomposable group is a group that is decomposed by the action of an acid to generate a polar group. The acid-decomposable group is the same as the acid-decomposable group having been described for the resin (A).

In a preferred embodiment, the ionic compound (Y) is a compound having a group represented by $-SF_4R$ in a photoacid generator (B) described later.

In the photoacid generator (B) described later, the cationic moiety may have a group represented by $-SF_4R$, or the anionic moiety may have a group represented by $-SF_4R$. Alternatively, the cationic moiety and the anionic moiety may have a group represented by $-SF_4R$.

In a preferred embodiment, the ionic compound (Y) is a compound having a group represented by $-SF_4R$ in an acid diffusion control agent described later.

In the acid diffusion control agent described later, the cationic moiety may have a group represented by $-SF_4R$, or the anionic moiety may have a group represented by $-SF_4R$. Alternatively, the cationic moiety and the anionic moiety may have a group represented by $-SF_4R$.

The content of the ionic compound (Y) relative to the total solid content of the composition is 5.0 mass% or

more, but is preferably 10.0 mass% or more, and more preferably 15.0 mass% or more.

The upper limit value of the content of the ionic compound (Y) is not particularly limited, but is, relative to the total solid content of the composition, ordinarily 80.0 mass% or less, preferably 70.0 mass% or less, and more preferably 60.0 mass% or less.

Such ionic compounds (Y) may be used alone, or may be used in combination of two or more thereof. Compound (B) that generates acid upon irradiation with actinic ray or radiation

The composition of the present invention preferably includes a compound that generates an acid upon irradiation with an actinic ray or a radiation (also referred to as a photoacid generator or a photoacid generator (B)). The photoacid generator is a compound that generates an acid upon exposure (preferably exposure using EUV light, KrF, ArF, or EB, and more preferably exposure using EUV light).

The photoacid generator (B) may have the form of a low-molecular-weight compound, or the form of being incorporated into a portion of a polymer (for example, the resin (A) described later). Alternatively, the form of a low-molecular-weight compound and the form of being incorporated into a portion of a polymer (for example, the resin (A) described above) may be used in combination.

When the photoacid generator (B) has the form of a low-molecular-weight compound, the photoacid generator preferably has a molecular weight of 3000 or less, more preferably 2000 or less, and still more preferably 1000 or less. The lower limit is not particularly limited, but is preferably 100 or more.

When the photoacid generator (B) has the form of being incorporated into a portion of a polymer, it may be incorporated into a portion of the resin (A) or may be incorporated into a resin different from the resin (A).

In this Specification, the photoacid generator (B) preferably has the form of a low-molecular-weight compound.

The photoacid generator is not particularly limited, but may be a photoacid generator having a non-anionic structure not having an anionic moiety or a cationic moiety, or may be a photoacid generator having an anionic moiety and a cationic moiety, namely, an onium salt.

In a preferred embodiment, the photoacid generator is preferably an onium salt.

The photoacid generator may have a group represented by $-SF_4R$, or may not have a group represented by $-SF_4R$. Here, R represents a hydrogen atom, a halogen atom, or a monovalent substituent. For R, specific examples and the like of the halogen atom and the monovalent substituent are the same as those having been described for the ionic compound.

In the photoacid generator (B), the cationic moiety may have a group represented by $-SF_4R$, or may not have a group represented by $-SF_4R$. The anionic moiety may have a group represented by $-SF_4R$, or may not have a group represented by $-SF_4R$. Alternatively, the cationic moiety and the anionic moiety may have a group represented by $-SF_4R$.

In the photoacid generator (B), a compound having, in at least one of the cationic moiety or the anionic moiety, a group represented by $-SF_4R$ is included in the above-described ionic compound (Y).

The photoacid generator (B) may be, for example, a compound represented by "$M^+ X^-$" (onium salt), and is preferably a compound that generates an organic acid by exposure.

Examples of the organic acid include sulfonic acids (such as aliphatic sulfonic acids, aromatic sulfonic acids, and camphorsulfonic acid), carboxylic acids (such as aliphatic carboxylic acids, aromatic carboxylic acids, and aralkyl carboxylic acids), carbonylsulfonylimidic acid, bis(alkylsulfonyl)imidic acids, and tris(alkylsulfonyl)methide acids.

In the compound represented by "$M^+ X^-$", $M^+$ represents an organic cation.

The organic cation is not particularly limited. For the valence, the organic cation may be mono-, di-, or higher valent.

The organic cation may have a group represented by $-SF_4R$, or may not have a group represented by $-SF_4R$.

The organic cation is preferably a sulfonium cation or an iodonium cation.

In particular, the organic cation is preferably a cation represented by a formula (ZaI) (hereafter, also referred to as "cation (ZaI)") or a cation represented by a formula (ZaII) (hereafter, also referred to as "cation (ZaII)").

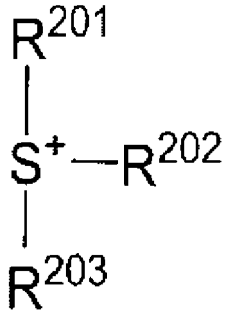

(ZaI)

$$R^{204}—I^{+}—R^{205} \quad \text{(ZaII)}$$

In the above-described formula (ZaI), $R^{201}$, $R^{202}$, and $R^{203}$ each independently represent an organic group.

For $R^{201}$, $R^{202}$, and $R^{203}$, such an organic group preferably has 1 to 30, and more preferably 1 to 20 carbon atoms.

Among $R^{201}$ to $R^{203}$, two may be bonded together to form a ring structure and the ring may include an oxygen atom, a sulfur atom, an ester group, an amide group, or a carbonyl group. Examples of the group formed by bonding together two among $R^{201}$ to $R^{201}$ include alkylene groups (such as a butylene group and a pentylene group), and $-CH_2-CH_2-O-CH_2-CH_2-$.

The organic group may have a group represented by $-SF_4R$, or may not have a group represented by $-SF_4R$.

The formula (ZaI) may be bonded to another formula (ZaI). This bonding may be formed via a linking group or may be formed not via a linking group.

Preferred examples of the organic cation in the formula (ZaI) include a cation (ZaI-1), a cation (ZaI-2), a cation (ZaI-3b), and a cation (ZaI-4b) described later.

First, the cation (ZaI-1) will be described.

The cation (ZaI-1) is an aryl sulfonium cation represented by the above-described formula (ZaI) where at least one of $R^{201}$ to $R^{201}$ is an aryl group.

In the aryl sulfonium cation, all of $R^{201}$ to $R^{201}$ may be aryl groups, or a part of $R^{201}$ to $R^{203}$ may be an aryl group and the other may be an alkyl group, a cycloalkyl group, or an alkenyl group. Two among $R^{201}$ to $R^{203}$ may be bonded together to form a ring structure.

Alternatively, one among $R^{201}$ to $R^{201}$ may be an aryl group and the other two among $R^{201}$ to $R^{201}$ may be bonded together to form a ring structure in which the ring may include an oxygen atom, a sulfur atom, an ester group, an amide group, or a carbonyl group.

Alternatively, the aryl group and another group may be bonded together to form a ring structure. As described above, two among $R^{201}$ to $R^{203}$ may be bonded together to form a ring structure.

Examples of the group formed by bonding together two among $R^{201}$ to $R^{201}$ include alkylene groups in which one or more methylene groups may be substituted with an oxygen atom, a sulfur atom, an ester group, an amide group, and/or a carbonyl group (such as a butylene group, a pentylene group, and $-CH_2-CH_2-O-CH_2-CH_2-$).

Examples of the aryl sulfonium cation include triaryl sulfonium cations, diaryl alkyl sulfonium cations, aryl dialkyl sulfonium cations, diaryl cycloalkyl sulfonium cations, and aryl dicycloalkyl sulfonium cations.

The aryl group included in the aryl sulfonium cation is not particularly limited, but is preferably a phenyl group or a naphthyl group, and more preferably a phenyl group. The aryl group may be an aryl group having a heterocyclic structure having an oxygen atom, a nitrogen atom, or a sulfur atom, for example. Examples of the heterocyclic structure include a pyrrole residue, a furan residue, a thiophene residue, an indole residue, a benzofuran residue, and a benzothiophene residue. When the aryl sulfonium cation has two or more aryl groups, the two or more aryl groups may be the same or different.

The alkyl group or cycloalkyl group that the aryl sulfonium cation optionally has is preferably a linear alkyl group having 1 to 15 carbon atoms, a branched alkyl group having 3 to 15 carbon atoms, or a cycloalkyl group having 3 to 15 carbon atoms, and more preferably a methyl group, an ethyl group, a propyl group, an n-butyl group, a sec-butyl group, a t-butyl group, a cyclopropyl group, a cyclobutyl group, or a cyclohexyl group.

The alkenyl group that the arylsulfonium cation optionally has is preferably a linear alkenyl group having 2 to 15 carbon atoms or a branched alkenyl group having 3 to 15 carbon atoms.

For $R^{201}$ to $R^{203}$, the aryl group, the alkyl group, the cycloalkyl group, and the alkenyl group may have a substituent.

For $R^{201}$ to $R^{203}$, the substituent that the aryl group, the alkyl group, the cycloalkyl group, and the alkenyl group

may have is not particularly limited, but is preferably an alkyl group (for example, having 1 to 15 carbon atoms), a cycloalkyl group (for example, having 3 to 15 carbon atoms), an aryl group (for example, having 6 to 14 carbon atoms), an alkoxy group (for example, having 1 to 15 carbon atoms), a cycloalkylalkoxy group (for example, having 1 to 15 carbon atoms), an alkoxycarbonyl group (for example, having 1 to 15 carbon atoms), a halogen atom (for example, fluorine and iodine), a hydroxyl group, a carboxyl group, an ester group, a sulfinyl group, a sulfonyl group, a group represented by $-SF_4R$ (where R represents a hydrogen atom, a halogen atom, or a monovalent substituent), an alkylthio group, or a phenylthio group.

When possible, the substituent may further have a substituent. In a preferred embodiment, the alkyl group also preferably has, as a substituent, a halogen atom to serve as an alkyl halide group such as a trifluoromethyl group.

Such substituents are also preferably combined appropriately to form an acid-decomposable group.

Note that the acid-decomposable group means a group that is decomposed by the action of an acid to generate a polar group, and preferably has a structure in which a group that leaves by the action of an acid protects the polar group. The polar group and the leaving group are as described above.

For $R^{201}$ to $R^{203}$, each group may have a group represented by $-SF_4R$, or may not have a group represented by $-SF_4R$.

Hereinafter, the cation (ZaI-2) will be described.

The cation (ZaI-2) is a cation represented by the formula (ZaI) where $R^{201}$ to $R^{203}$ each independently represent an organic group not having an aromatic ring. The aromatic ring also encompasses aromatic rings including a heteroatom.

For $R^{201}$ to $R^{203}$, the organic group not having an aromatic ring preferably has 1 to 30 carbon atoms and more preferably 1 to 20 carbon atoms.

The organic group not having an aromatic ring may have a group represented by $-SF_4R$, or may not have a group represented by $-SF_4R$.

$R^{201}$ to $R^{203}$ are each independently preferably an alkyl group, a cycloalkyl group, an allyl group, or a vinyl group, more preferably a linear or branched 2-oxoalkyl group, a 2-oxocycloalkyl group, or an alkoxycarbonylmethyl group, and still more preferably a linear or branched 2-oxoalkyl group.

For $R^{201}$ to $R^{203}$, the alkyl group and the cycloalkyl group may be, for example, a linear alkyl group having 1 to 10 carbon atoms or a branched alkyl group having 3 to 10 carbon atoms (for example, a methyl group, an ethyl group, a propyl group, a butyl group, or a pentyl group), or a cycloalkyl group having 3 to 10 carbon atoms (for example, a cyclopentyl group, a cyclohexyl group, or a norbornyl group).

$R^{201}$ to $R^{201}$ may be further substituted with, for example, a halogen atom, an alkoxy group (for example, having 1 to 5 carbon atoms), a hydroxy group, a cyano group, a group represented by $-SF_4R$ (where R represents a hydrogen atom, a halogen atom, or a monovalent substituent), or a nitro group.

For $R^{201}$ to $R^{203}$, substituents are also preferably provided independently as appropriate combinations of substituents to form acid-decomposable groups.

Hereinafter, the cation (ZaI-3b) will be described.

The cation (ZaI-3b) is a cation represented by the following formula (ZaI-3b).

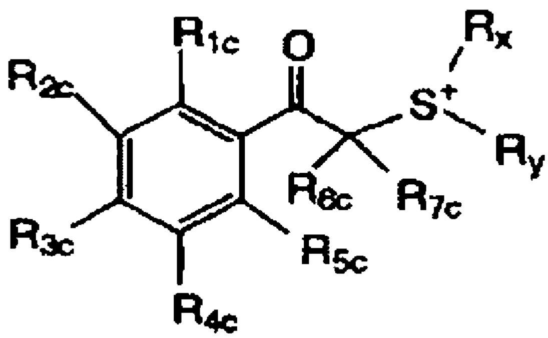

(ZaI-3b)

In the formula (ZaI-3b), $R_{1c}$ to $R_{5c}$ each independently represent a hydrogen atom, an alkyl group, a cycloalkyl group, an aryl group, an alkoxy group, an aryloxy group, an alkoxycarbonyl group, an alkylcarbonyloxy group, a cycloalkylcarbonyloxy group, a halogen atom, a hydroxy group, a nitro group, a group represented by $-SF_4R$ (where R represents a hydrogen atom, a halogen atom, or a monovalent substituent), an alkylthio group, or an arylthio group.

For $R_{1c}$ to $R_{5c}$, the alkyl group, the cycloalkyl group, the aryl group, the alkoxy group, the aryloxy group, the alkoxycarbonyl group, the alkylcarbonyloxy group, the cycloalkylcarbonyloxy group, the group represented by $-SF_4R$, the alkylthio group, or the arylthio group may have a substituent.

$R_{6c}$ and $R_{7}$. each independently represent a hydrogen atom, an alkyl group (for example, a t-butyl group), a cycloalkyl group, a halogen atom, a cyano group, a group represented by $-SF_4R$ (where R represents a hydrogen atom,

a halogen atom, or a monovalent substituent), and an aryl group.

For $R_{6c}$ and $R_{7c}$, the alkyl group, the cycloalkyl group, the group represented by $-SF_4R$, and the aryl group may have a substituent.

$R_x$ and $R_y$ each independently represent an alkyl group, a cycloalkyl group, a 2-oxoalkyl group, a 2-oxocycloalkyl group, an alkoxycarbonylalkyl group, an allyl group, or a vinyl group.

For $R_x$ and $R_y$, the alkyl group, the cycloalkyl group, the 2-oxoalkyl group, the 2-oxocycloalkyl group, the alkoxycarbonylalkyl group, the allyl group, and the vinyl group may have a substituent.

For $R_{1c}$ to $R_{7c}$ and $R_x$ and $R_y$, such substituents are also preferably provided independently as appropriate combinations of substituents to form acid-decomposable groups.

Any two or more among $R_{1c}$ to $R_{5c}$, $R_{5c}$ and $R_{6c}$, $R_{6c}$ and $R_{7c}$, $R_{5c}$ and $R_x$, and $R_x$ and $R_y$ may be individually bonded together to form rings; these rings may each independently include an oxygen atom, a sulfur atom, a ketone group, an ester bond, or an amide bond.

Such a ring may be an aromatic or non-aromatic hydrocarbon ring, an aromatic or non-aromatic heterocycle, or a polycyclic fused ring formed as a combination of two or more of these rings. The ring may be a 3 - to 10-membered ring, and is preferably a 4- to 8-membered ring, and more preferably a 5- or 6-membered ring.

Examples of the groups formed by bonding together any two or more among $R_{1c}$ to $R_{5c}$, $R_{6c}$ and $R_{7c}$, and $R_x$ and $R_y$ include alkylene groups such as a butylene group and a pentylene group. In such an alkylene group, a methylene group may be substituted with a heteroatom such as an oxygen atom.

The groups formed by bonding together $R_{5c}$ and $R_{6c}$, and $R_{5c}$ and $R_x$ are preferably single bonds or alkylene groups. Examples of the alkylene groups include a methylene group and an ethylene group.

$R_{1c}$ to $R_{5c}$, $R_{6c}$, $R_{7c}$, $R_x$, $R_y$, and the rings formed by individually bonding together any two or more among $R_{1c}$ to $R_{5c}$, $R_{5c}$ and $R_{6c}$, $R_{6c}$ and $R_{7c}$, $R_{5c}$ and $R_x$, and $R_x$ and $R_y$ may have a substituent.

Hereinafter, the cation (ZaI-4b) will be described.

The cation (ZaI-4b) is a cation represented by the following formula (ZaI-4b).

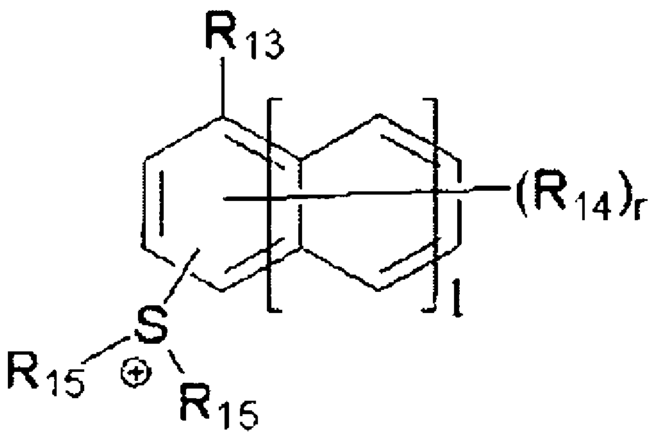

(ZaI-4b)

In the formula (ZaI-4b), 1 represents an integer of 0 to 2, and r represents an integer of 0 to 8.

$R_{13}$ represents a hydrogen atom, a halogen atom (for example, a fluorine atom or an iodine atom), a hydroxy group, an alkyl group, an alkyl halide group, an alkoxy group, a carboxyl group, an alkoxycarbonyl group, a group including a cycloalkyl group (may be the cycloalkyl group itself or may be a group including, as a part thereof, the cycloalkyl group), or a group represented by $-SF_4R$ (where R represents a hydrogen atom, a halogen atom, or a monovalent substituent). These groups may have a substituent.

$R_{14}$ represents a hydroxy group, a halogen atom (for example, a fluorine atom or an iodine atom), an alkyl group, an alkyl halide group, an alkoxy group, an alkoxycarbonyl group, an alkylcarbonyl group, an alkylsulfonyl group, a cycloalkylsulfonyl group, a group including a cycloalkyl group (may be the cycloalkyl group itself or may be a group including, as a part thereof, the cycloalkyl group), or a group represented by $-SF_4R$ (where R represents a hydrogen atom, a halogen atom, or a monovalent substituent). These groups may have a substituent. When there are a plurality of $R_{14}$'s, $R_{14}$'s each independently represent such a group, for example, a hydroxy group.

$R_{15}$'s each independently represent an alkyl group, a cycloalkyl group, or a naphthyl group. Two $R_{15}$'s may be bonded together to form a ring. When two $R_{15}$'s are bonded together to form a ring, the ring skeleton may include a heteroatom such as an oxygen atom or a nitrogen atom.

In an example, two $R_{15}$'s are preferably alkylene groups and bonded together to form a ring structure. Note that the alkyl group, the cycloalkyl group, the naphthyl group, and the ring formed by bonding together two $R_{15}$'s may have a substituent.

In the formula (ZaI-4b), for $R_{13}$, $R_{14}$, and $R_{15}$, the alkyl groups may be linear or branched. Such an alkyl group preferably has 1 to 10 carbon atoms. Preferred examples of the alkyl group include a methyl group, an ethyl group, an n-butyl group, and a t-butyl group.

For $R_{13}$ to $R_{15}$, and $R_x$ and $R_y$, such substituents are also preferably provided independently as appropriate combinations of substituents to form acid-decomposable groups.

Hereinafter, the formula (ZaII) will be described.

In the formula (ZaII), $R^{204}$ and $R^{205}$ each independently represent an aryl group, an alkyl group, or a cycloalkyl group.

For $R^{204}$ and $R^{205}$, the aryl group is preferably a phenyl group or a naphthyl group, and more preferably a phenyl group. For $R^{204}$ and $R^{205}$, the aryl group may be an aryl group having a heterocycle having an oxygen atom, a nitrogen atom, or a sulfur atom, for example. Examples of the skeleton of the aryl group having a heterocycle include pyrrole, furan, thiophene, indole, benzofuran, and benzothiophene.

For $R^{204}$ and $R^{205}$, the alkyl group and the cycloalkyl group are preferably a linear alkyl group having 1 to 10 carbon atoms, a branched alkyl group having 3 to 10 carbon atoms (for example, a methyl group, an ethyl group, a propyl group, a butyl group, or a pentyl group), or a cycloalkyl group having 3 to 10 carbon atoms (for example, a cyclopentyl group, a cyclohexyl group, or a norbornyl group).

For $R^{204}$ and $R^{205}$, the aryl group, the alkyl group, and the cycloalkyl group may each independently have a substituent. For $R^{204}$ and $R^{205}$, examples of the substituent that the aryl group, the alkyl group, and the cycloalkyl group may have include alkyl groups (having, for example, 1 to 15 carbon atoms), cycloalkyl groups (having, for example, 3 to 15 carbon atoms), aryl groups (having, for example, 6 to 15 carbon atoms), alkoxy groups (having, for example, 1 to 15 carbon atoms), halogen atoms, a hydroxy group, a group represented by $-SF_4R$ (where R represents a hydrogen atom, a halogen atom, or a monovalent substituent), and a phenylthio group.

For $R^{204}$ and $R^{205}$, substituents are also preferably provided independently as appropriate combinations of substituents to form acid-decomposable groups.

The following are specific examples of the organic cation; however, the present invention is not limited to these.

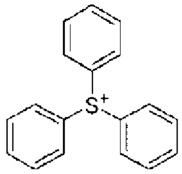
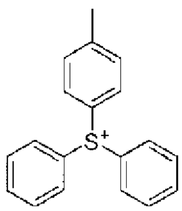
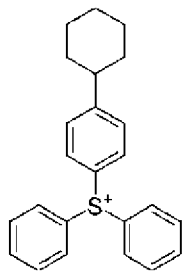
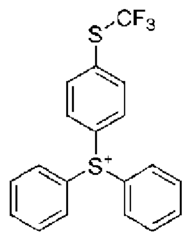
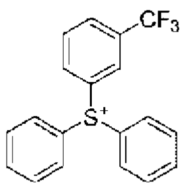
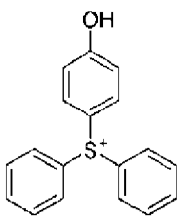
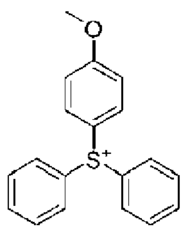
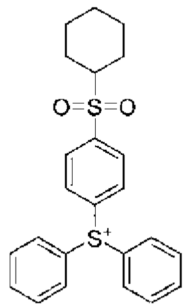
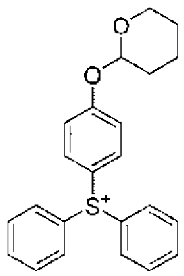
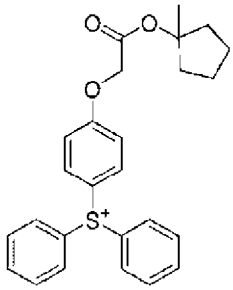
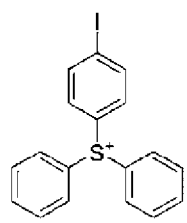
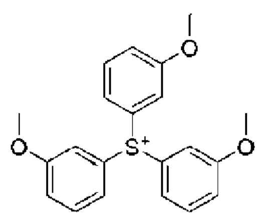

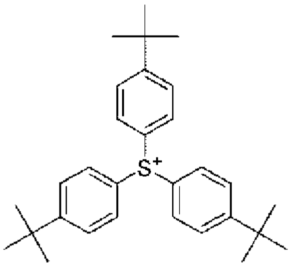
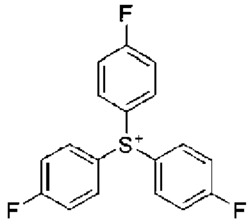
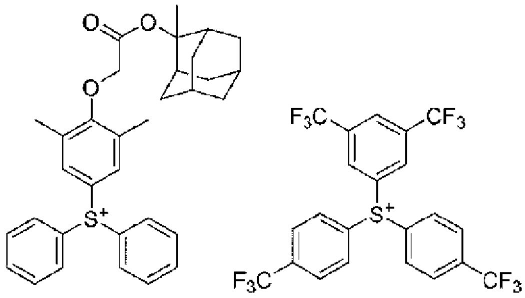

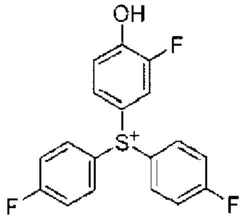
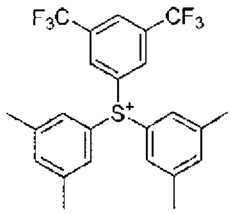
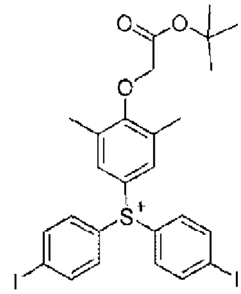
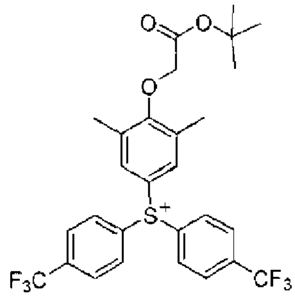

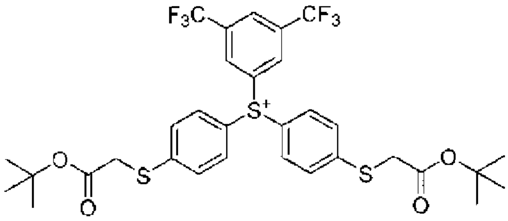
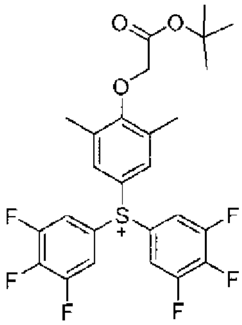
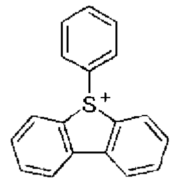

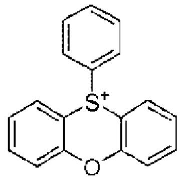
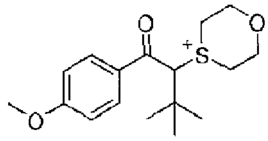
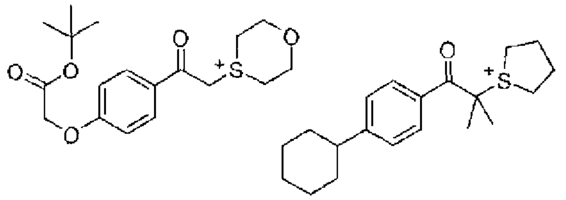

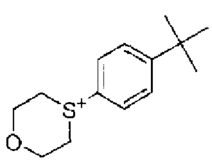
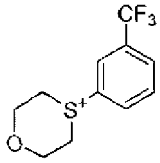
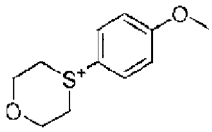
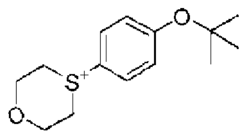

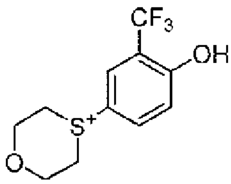
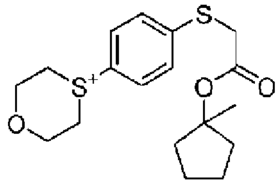
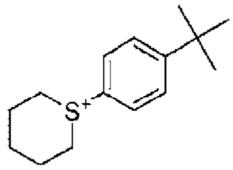
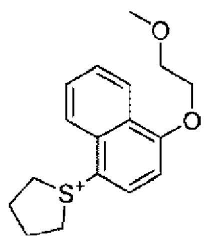

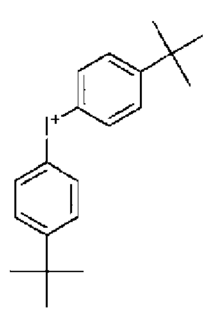
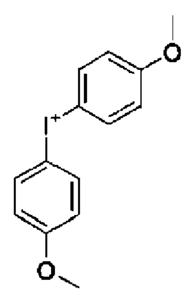
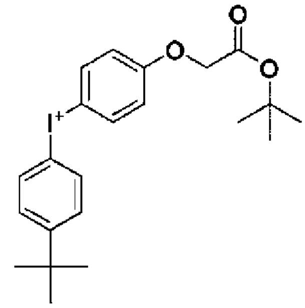

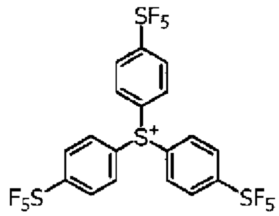
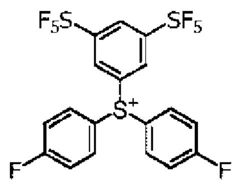
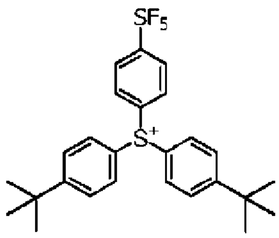
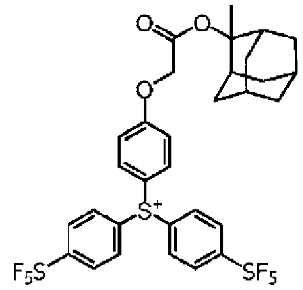

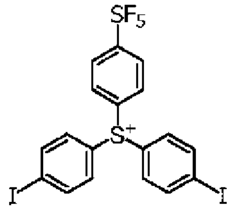
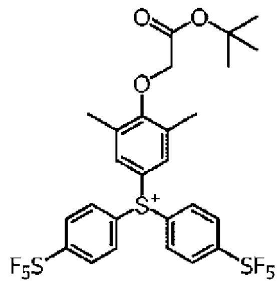
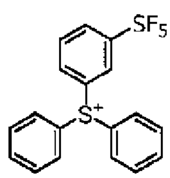
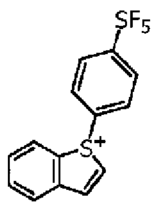

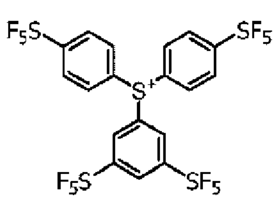
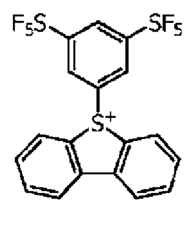
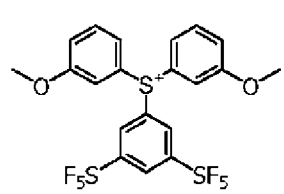
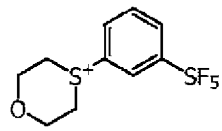

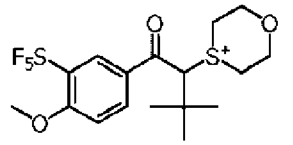
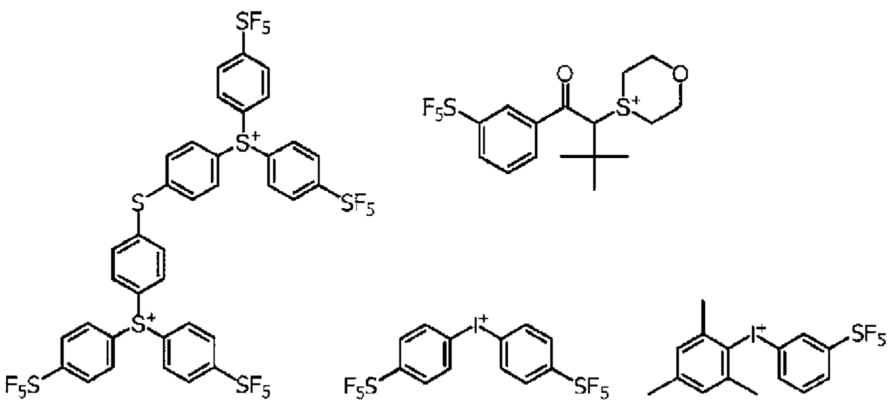
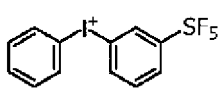

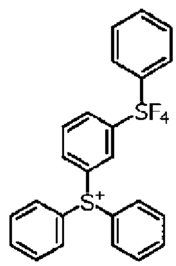

In the compound represented by "$M^+ X^-$", $X^-$ represents an organic anion.

The organic anion is not particularly limited, but may be a mono-, di-, or higher valent organic anion.

The organic anion is preferably an anion that has a very low capability of causing a nucleophilic reaction, and more preferably a non-nucleophilic anion.

The organic anion may have a group represented by $-SF_4R$, or may not have a group represented by $-SF_4R$.

Examples of the non-nucleophilic anion include sulfonate anions (such as aliphatic sulfonate anions, aromatic sulfonate anions, and a camphorsulfonate anion), carboxylate anions (such as aliphatic carboxylate anions, aromatic carboxylate anions, and aralkyl carboxylate anions), a sulfonylimide anion, bis(alkylsulfonyl)imide anions, and tris(alkylsulfonyl)methide anions.

In such an aliphatic sulfonate anion or aliphatic carboxylate anion, the aliphatic moiety may be a linear or branched alkyl group or may be a cycloalkyl group, and is preferably a linear or branched alkyl group having 1 to 30 carbon atoms, or a cycloalkyl group having 3 to 30 carbon atoms.

The alkyl group may be, for example, a fluoroalkyl group (that may have a substituent other than a fluorine atom, or may be a perfluoroalkyl group).

In such an aromatic sulfonate anion or aromatic carboxylate anion, the aryl group is preferably an aryl group having 6 to 14 carbon atoms, and may be, for example, a phenyl group, a tolyl group, or a naphthyl group.

The above-described alkyl group, cycloalkyl group, and aryl group may have a substituent. The substituent is not particularly limited; examples include a nitro group, halogen atoms such as a fluorine atom and a chlorine atom, a carboxyl group, a hydroxy group, an amino group, a cyano group, alkoxy groups (preferably having 1 to 15 carbon atoms), alkyl groups (preferably having 1 to 10 carbon atoms), cycloalkyl groups (preferably having 3 to 15 carbon atoms), aryl groups (preferably having 6 to 14 carbon atoms), alkoxycarbonyl groups (preferably having 2 to 7 carbon atoms), acyl groups (preferably having 2 to 12 carbon atoms), alkoxycarbonyloxy groups (preferably having 2 to 7 carbon atoms), alkylthio groups (preferably having 1 to 15 carbon atoms), alkylsulfonyl groups (preferably having 1 to 15 carbon atoms), alkyliminosulfonyl groups (preferably having 1 to 15 carbon atoms), and aryloxysulfonyl groups (preferably having 6 to 20 carbon atoms).

In such an aralkyl carboxylate anion, the aralkyl group is preferably an aralkyl group having 7 to 14 carbon atoms.

Examples of the aralkyl group having 7 to 14 carbon atoms include a benzyl group, a phenethyl group, a naphthylmethyl group, a naphthylethyl group, and a naphthylbutyl group.

The sulfonylimide anion may be, for example, a saccharin anion.

In such a bis(alkylsulfonyl)imide anion or a tris(alkylsulfonyl)methide anion, the alkyl groups are preferably an alkyl group having 1 to 5 carbon atoms. In the alkyl group, a substituent may be a halogen atom, an alkyl group substituted with a halogen atom, an alkoxy group, an alkylthio group, an alkyloxysulfonyl group, an aryloxysulfonyl group, or a cycloalkylaryloxysulfonyl group, and is preferably a fluorine atom or an alkyl group substituted with a fluorine atom.

In the bis(alkylsulfonyl)imide anion, the alkyl groups may be bonded together to form a ring structure. This results in an increase in the acid strength.

Other examples of the non-nucleophilic anion include phosphorus fluoride (for example, $PF_6^-$), boron fluoride (for example, $BF_4^-$), and antimony fluoride (for example, $SbF_6^-$).

The non-nucleophilic anion is preferably an aliphatic sulfonate anion in which at least the $\alpha$ position of sulfonic acid is substituted with a fluorine atom, an aromatic sulfonate anion substituted with a fluorine atom or a group having a fluorine atom, a bis(alkylsulfonyl)imide anion in which the alkyl groups are substituted with fluorine atoms, or a tris(alkylsulfonyl)methide anion in which the alkyl groups are substituted with fluorine atoms. In particular, the anion is more preferably a perfluoroaliphatic sulfonate anion (preferably having 4 to 8 carbon atoms) or a benzenesulfonate anion having a fluorine atom, and still more preferably a nonafluorobutanesulfonate anion, a perfluorooctanesulfonate anion, a pentafluorobenzenesulfonate anion, or a 3,5-bis(trifluoromethyl)benzenesulfonate anion.

The non-nucleophilic anion is also preferably an anion represented by the following formula (AN1).

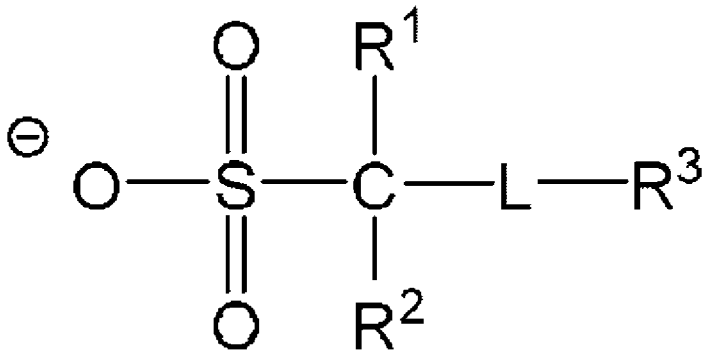

(AN1)

In the formula (AN1), $R^1$ and $R^2$ each independently represent a hydrogen atom or a substituent.

The substituent is not particularly limited, but is preferably a group that is not electron-withdrawing groups. Examples of the group that is not electron-withdrawing groups include hydrocarbon groups, a hydroxy group, oxyhydrocarbon groups, oxycarbonylhydrocarbon groups, an amino group, hydrocarbon-substituted amino groups, and hydrocarbon-substituted amide groups.

Such groups that are not electron-withdrawing groups are each independently preferably -R', -OH, -OR', -OCOR', $-NH_2$, $-NR'_2$, -NHR', or -NHCOR'. R' are monovalent hydrocarbon groups.

Examples of the monovalent hydrocarbon groups represented by R' above include monovalent linear or branched hydrocarbon groups such as alkyl groups such as a methyl group, an ethyl group, a propyl group, and a butyl group; alkenyl groups such as an ethenyl group, a propenyl group, and a butenyl group; and alkynyl groups such as an ethynyl group, a propynyl group, and a butynyl group; monovalent alicyclic hydrocarbon groups such as cycloalkyl groups such as a cyclopropyl group, a cyclobutyl group, a cyclopentyl group, a cyclohexyl group, a norbornyl group, and an adamantyl group; and cycloalkenyl groups such as a cyclopropenyl group, a cyclobutenyl group, a cyclopentenyl group, and a norbornenyl group; and monovalent aromatic hydrocarbon groups such as aryl groups such as a phenyl group, a tolyl group, a xylyl group, a mesityl group, a naphthyl group, a methylnaphthyl group, an anthryl group, and methylanthryl group; and aralkyl groups such as a benzyl group, a phenethyl group, a phenylpropyl group, a naphthylmethyl group, and an anthrylmethyl group.

In particular, $R^1$ and $R^2$ are each independently preferably a hydrocarbon group (preferably a cycloalkyl group) or a hydrogen atom.

L represents a divalent linking group.

When a plurality of L's are present, L's may be the same or different.

The divalent linking group may be, for example, -O-CO-O-, -COO-, -CONH-, -CO-, -O-, -S-, -SO-, $-SO_2-$, an alkylene group (preferably having 1 to 6 carbon atoms), or a cycloalkylene group (that may be monocyclic or may be polycyclic and preferably has 3 to 15 carbon atoms), an alkenylene group (preferably having 2 to 6 carbon atoms), a lactone ring group, a sultone ring group, or a divalent linking group that is a combination of a plurality of the foregoing. In particular, the divalent linking group is preferably -O-CO-O-, -COO-, -CONH-, -CO-, -O-, $-SO_2-$, -O-CO-O-alkylene group-, -COO-alkylene group-, or -CONH-alkylene group-, and more preferably -O-CO-O-, -O-CO-O-alkylene group-, -COO-, -CONH-, $-SO_2-$, or -COO-alkylene group-.

The lactone ring group and the sultone ring group are, for example, preferably a group formed by removing two hydrogen atoms from the ring-member atoms constituting the lactone structure or the sultone structure in any one of the above-described structures represented by the formulas (LC1 -1) to (LC1-21) and the above-described structures represented by the formulas (SL 1 -1) to (SL 1-3).

The alkylene group, the cycloalkylene group, and the alkenylene group may have a substituent.

L is preferably, for example, a group represented by the following formula (AN1-1).

$$*^{a}-(CR^{2a}{}_2)_X-Q-(CR^{2b}{}_2)_Y-*^{b} \qquad \text{(AN1-1)}$$

In the formula (AN1-1), $*^a$ represents the bonding site to $R^3$ in the formula (AN1).

$*^b$ represents the bonding site to $-C(R^1)(R^2)-$ in the formula (AN1).
X and Y each independently represent an integer of 0 to 10, and is preferably an integer of 0 to 3.
$R^{2a}$ and $R^{2b}$ each independently represent a hydrogen atom or a substituent.

When a plurality of $R^{2a}$'s and a plurality of $R^{2b}$'s are present, the plurality of $R^{2a}$'s and the plurality of $R^{2b}$'s present may be individually the same or different.

Note that, when Y is 1 or more, in the formula (AN1), in $CR^{2b}_2$ directly bonded to -C($R^1$)($R^2$)-, $R^{2b}$'s are not fluorine atoms.

Q represents $*^A$-O-CO-O-$*^B$, $*^A$-CO-$*^B$, $*^A$-CO-O-$*^B$, $*^A$-O-CO-$*^B$, $*^A$-O-$*^B$, $*^A$-S-$*^B$, or $*^A$-$SO_2$-$*^B$.

Note that, when X + Y in the formula (AN1-1) is 1 or more, and $R^{2a}$'s and $R^{2b}$'s in the formula (AN1-1) are all hydrogen atoms, Q represents $*^A$-O-CO-O-$*^B$, $*^A$-CO-$*^B$, $*^A$-O-CO-$*^B$, $*^A$-O-$*^B$, $*^A$-S-$*^B$, or $*^A$-$SO_2$-$*^B$.

$*^A$ represent a bonding site on the $R^3$ side in the formula (AN1) and $*^B$ represent a bonding site on the -$SO_3^-$ side in the formula (AN1).

In the formula (AN1), $R^3$ represents an organic group.

The organic group is not particularly limited as long as it has 1 or more carbon atoms, and may be a linear group (for example, a linear alkyl group), a branched group (for example, a branched alkyl group such as a t-butyl group), or a cyclic group. The organic group may have or may not have a substituent. The organic group may have or may not have a heteroatom (such as an oxygen atom, a sulfur atom, and/or a nitrogen atom).

The organic group may have a group represented by -$SF_4$R, or may not have a group represented by -$SF_4$R.

The organic group may be, for example, an organic group having 1 to 30 carbon atoms.

In particular, $R^3$ is preferably an organic group having a ring structure. The ring structure may be monocyclic or polycyclic, and may have a substituent. In the organic group including a ring structure, the ring is preferably directly bonded to L in the formula (AN1).

The organic group having a ring structure, for example, may have or may not have a heteroatom (such as an oxygen atom, a sulfur atom, and/or a nitrogen atom). The heteroatom may substitute one or more carbon atoms forming the ring structure.

The organic group having a ring structure is preferably, for example, a hydrocarbon group having a ring structure, a lactone ring group, or a sultone ring group. In particular, the organic group having a ring structure is preferably a hydrocarbon group having a ring structure.

The hydrocarbon group having a ring structure is preferably a monocyclic or polycyclic cycloalkyl group. Such groups may have a substituent.

The cycloalkyl group may be monocyclic (such as a cyclohexyl group) or polycyclic (such as an adamantyl group), and preferably has 5 to 12 carbon atoms.

The lactone group and the sultone group are, for example, preferably a group provided by removing, in any one of the above-described structures represented by the formulas (LC1-1) to (LC1-21) and the above-described structures represented by the formulas (SL1-1) to (SL 1-3), one hydrogen atom from a ring-member atom constituting the lactone structure or the sultone structure.

The non-nucleophilic anion may be a benzenesulfonate anion, and is preferably a benzenesulfonate anion substituted with a branched alkyl group or a cycloalkyl group.

The non-nucleophilic anion is also preferably an anion represented by the following formula (AN2).

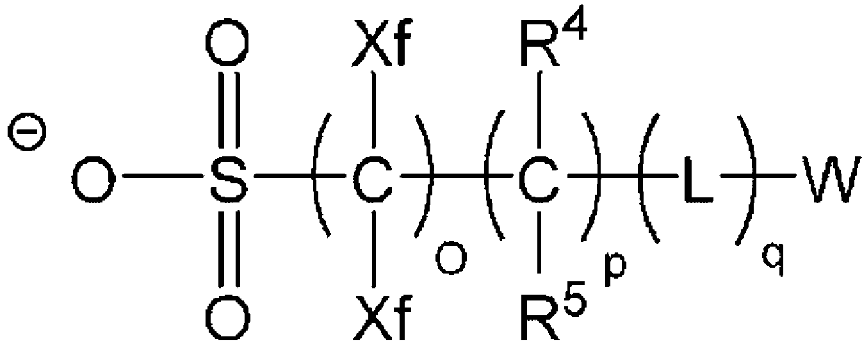

(AN2)

In the formula (AN2), o represents an integer of 1 to 3. p represents an integer of 0 to 10. q represents an integer of 0 to 10.

Xf's represent a hydrogen atom, a fluorine atom, an alkyl group substituted with at least one fluorine atom, an organic group not having fluorine atoms, or a group represented by -$SF_4$R (where R represents a hydrogen atom, a halogen atom, or a monovalent substituent). The alkyl group preferably has 1 to 10 carbon atoms, and more preferably 1 to 4 carbon atoms. The alkyl group substituted with at least one fluorine atom is preferably a perfluoroalkyl group.

Xf's are preferably a fluorine atom or a perfluoroalkyl group having 1 to 4 carbon atoms, and more preferably a fluorine atom or $CF_3$; still more preferably, both of Xf's are fluorine atoms.

$R^4$ and $R^5$ each independently represent a hydrogen atom, a fluorine atom, an alkyl group, or an alkyl group substituted with at least one fluorine atom. When a plurality of $R^4$'s and a plurality of $R^5$'s are present, $R^4$'s and $R^5$'s

may be individually the same or different.

For $R^4$ and $R^5$, the alkyl group preferably has 1 to 4 carbon atoms. The alkyl group may have a substituent. $R_4$ and $R_5$ are preferably a hydrogen atom.

L represents a divalent linking group. L has the same definition as L in the formula (AN1).

W represents an organic group.

The organic group is not particularly limited as long as it has 1 or more carbon atoms, and may be a linear group (for example, a linear alkyl group), a branched group (for example, a branched alkyl group such as a t-butyl group), or a cyclic group. The organic group may have or may not have a substituent. The organic group may have or may not have a heteroatom (such as an oxygen atom, a sulfur atom, and/or a nitrogen atom).

The organic group may have a group represented by $-SF_4R$, or may not have a group represented by $-SF_4R$.

The organic group may be, for example, an organic group having 1 to 30 carbon atoms.

W is preferably an organic group having a ring structure. In particular, preferred is a cyclic organic group.

The cyclic organic group may be, for example, an alicyclic group, an aryl group, or a heterocyclic group.

The alicyclic group may be monocyclic or may be polycyclic. Examples of the monocyclic alicyclic group include monocyclic cycloalkyl groups such as a cyclopentyl group, a cyclohexyl group, and a cyclooctyl group. Examples of the polycyclic alicyclic group include polycyclic cycloalkyl groups such as a norbornyl group, a tricyclodecanyl group, a tetracyclodecanyl group, a tetracyclododecanyl group, and an adamantyl group. In particular, preferred are alicyclic groups having a bulky structure having 7 or more carbon atoms such as a norbornyl group, a tricyclodecanyl group, a tetracyclodecanyl group, a tetracyclododecanyl group, and an adamantyl group.

The aryl group may be monocyclic or polycyclic. Examples of the aryl group include a phenyl group, a naphthyl group, a phenanthryl group, and an anthryl group.

The heterocyclic group may be monocyclic or polycyclic. In particular, in the case of a polycyclic heterocyclic group, diffusion of acid can be further suppressed. The heterocyclic group may have aromaticity or may not have aromaticity. Examples of the heterocycle having aromaticity include a furan ring, a thiophene ring, a benzofuran ring, a benzothiophene ring, a dibenzofuran ring, a dibenzothiophene ring, and a pyridine ring. Examples of the heterocycle not having aromaticity include a tetrahydropyran ring, a lactone ring, a sultone ring, and a decahydroisoquinoline ring. In the heterocyclic group, the heterocycle is preferably a furan ring, a thiophene ring, a pyridine ring, or a decahydroisoquinoline ring.

The cyclic organic group may have a substituent. The substituent may be, for example, an alkyl group (that may be linear or branched and preferably has 1 to 12 carbon atoms), a cycloalkyl group (that may have a monocycle, a polycycle, or a spiro ring, and preferably has 3 to 20 carbon atoms), an aryl group (preferably having 6 to 14 carbon atoms), a hydroxy group, an alkoxy group, an ester group, an amide group, a urethane group, a ureido group, a group represented by $-SF_4R$ (where R represents a hydrogen atom, a halogen atom, or a monovalent substituent), a thioether group, a sulfonamide group, or a sulfonic acid ester group. Note that a carbon constituting the cyclic organic group (carbon contributing to formation of the ring) may be a carbonyl carbon.

The anion represented by the formula (AN2) is preferably $SO_3^-$-$CF_2$-$CH_2$-OCO-$(L)_{q'}$-W, $SO_3^-$-$CF_2$-CHF-$CH_2$-OCO-(L)q-W, $SO_3^-$-$CF_2$-COO-$(L)_{q'}$-W, $SO_3^-$-$CF_2$-$CF_2$-$CH_2$-$CH_2$-$(L)_q$-W, or $SO_3^-$-$CF_2$-CH($CF_3$)-OCO-$(L)_{q'}$-W. Here, L, q, and W are the same as those in the formula (AN2). q' represents an integer of 0 to 10.

The non-nucleophilic anion is also preferably an aromatic sulfonate anion represented by the following formula (AN3).

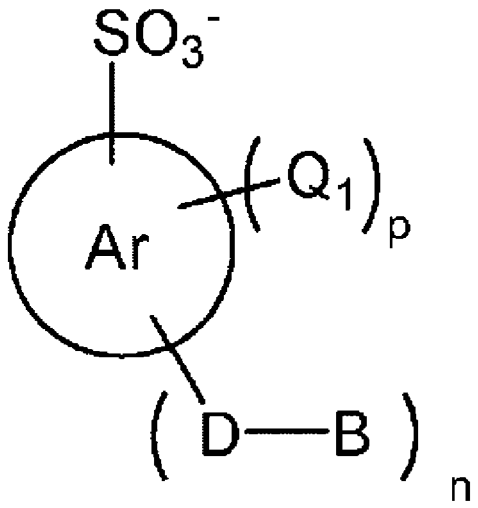

(AN3)

In the formula (AN3), Ar represents an aryl group (such as a phenyl group).

D represents a single bond or a divalent linking group.

B represents a hydrocarbon group.

$Q_1$ represents a group represented by $-SF_4R$ (where R represents a hydrogen atom, a halogen atom, or a monovalent substituent), a fluorine atom, or a hydroxy group.

n represents an integer of 0 or more. When n represents an integer of 2 or more, a plurality of D's may be the same or different. When n represents an integer of 2 or more, a plurality of B's may be the same or different.

p represents an integer of 0 or more. When p represents an integer of 2 or more, a plurality of $Q_1$'s may be the same or different.

D represents a single bond or a divalent linking group. The divalent linking group may be an ether group, a thioether group, a carbonyl group, a sulfoxide group, a sulfo group, a sulfonic acid ester group, an ester group, or a group that is a combination of two or more of the foregoing.

B represents a hydrocarbon group.

B is preferably an aliphatic hydrocarbon group, and more preferably an isopropyl group, a cyclohexyl group, or an aryl group that may further have a substituent (such as a tricyclohexylphenyl group).

n represents an integer of 0 or more. n is preferably 1 or more.

R represents a hydrogen atom, a halogen atom, or a monovalent substituent. For R, specific examples and the like of the halogen atom and the monovalent substituent are the same as those having been described for the ionic compound.

p represents an integer of 0 or more. p is preferably 1 or more.

The non-nucleophilic anion is also preferably a disulfonamide anion.

The disulfonamide anion is, for example, an anion represented by $N^-(SO_2-R^q)_2$.

$R^q$'s represent an alkyl group that may have a substituent, and are preferably a fluoroalkyl group, and more preferably a perfluoroalkyl group. Two $R^q$'s may be bonded together to form a ring. The group formed by bonding together two $R^q$'s is preferably an alkylene group that may have a substituent, preferably a fluoroalkylene group, and more preferably a perfluoroalkylene group. The alkylene group preferably has 2 to 4 carbon atoms.

Other examples of the non-nucleophilic anion include anions represented by the following formulas (d1-1) to (d1-4).

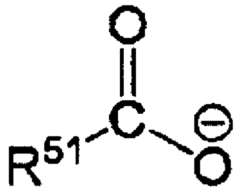

(d1-1)

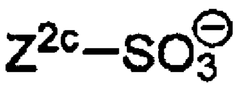

(d1-2)

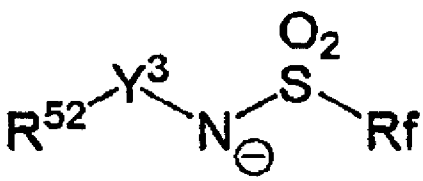

(d1-3)

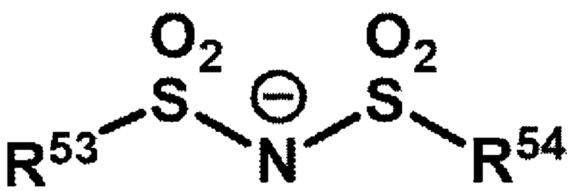

(d1-4)

In the formula (d1-1), $R^{51}$ represents a hydrocarbon group that may have a substituent (such as a hydroxy group) (for example, an aryl group such as a phenyl group).

In the formula (d1-2), $Z^{2c}$ represents a hydrocarbon group that has 1 to 30 carbon atoms and that may have a substituent (provided that the carbon atom adjacent to S is not substituted with a fluorine atom).

In $Z^{2c}$, the hydrocarbon group may be linear or branched, and may have a ring structure. In the hydrocarbon group, a carbon atom (preferably, in a case where the hydrocarbon group has a ring structure, a carbon atom serving as a ring-member atom) may be a carbonyl carbon (-CO-). The hydrocarbon group may be, for example, a group that has a norbornyl group that may have a substituent. A carbon atom forming the norbornyl group may be a carbonyl carbon.

In the formula (d1-2), "$Z^{2c}$-$SO_3^-$" is preferably different from the anions represented by the above-described formulas (AN1) to (AN3). For example, $Z^{2c}$ is preferably not aryl groups. For example, in $Z^{2c}$, the atoms at the $\alpha$ position and the $\beta$ position with respect to -$SO_3^-$ are preferably atoms other than carbon atoms having, as a substituent, a fluorine atom. For example, in $Z^{2c}$, the atom at the $\alpha$ position and/or the atom at the $\beta$ position with respect to -$SO_3^-$is preferably a ring-member atom in a ring group.

In the formula (d1-3), $R^{52}$ represents an organic group (preferably a hydrocarbon group having a fluorine atom); $Y^3$ represents a linear, branched, or cyclic alkylene group, an arylene group, or a carbonyl group; and Rf represents a hydrocarbon group.

In the formula (d1-4), $R^{53}$ and $R^{54}$ each independently represent an organic group (preferably a hydrocarbon group having a fluorine atom). $R^{53}$ and $R^{54}$ may be bonded together to form a ring.

Such organic anions may be used alone or in combination of two or more thereof.

The photoacid generator is also preferably at least one selected from the group consisting of compounds (I) to (II).

Compound (I)

The compound (I) is a compound having one or more structural moieties X described below and one or more structural moieties Y described below, and is a compound that generates, upon irradiation with an actinic ray or a radiation, an acid including a first acidic moiety described below derived from the structural moiety X described below and a second acidic moiety described below derived from the structural moiety Y described below.

Structural moiety X: a structural moiety that is constituted by an anionic moiety $A_1^-$ and a cationic moiety $M_1^+$ and that forms, upon irradiation with an actinic ray or a radiation, the first acidic moiety represented by $HA_1$

Structural moiety Y: a structural moiety that is constituted by an anionic moiety $A_2^-$ and a cationic moiety $M_2^+$ and that forms, upon irradiation with an actinic ray or a radiation, the second acidic moiety represented by $HA_2$

The compound (I) satisfies the following condition I.

Condition I: A compound PI in which the cationic moiety $M_1^+$ in the structural moiety X and the cationic moiety $M_2^+$ in the structural moiety Y in the compound (I) are replaced by $H^+$ has an acid dissociation constant a1 derived from an acidic moiety represented by $HA_1$ in which the cationic moiety $M_1^+$ in the structural moiety X is replaced by $H^+$, and an acid dissociation constant a2 derived from an acidic moiety represented by $HA_2$ in which the cationic moiety $M_2^+$ in the structural moiety Y is replaced by $H^+$, and the acid dissociation constant a2 is larger than the acid dissociation constant a1.

Hereinafter, the condition I will be more specifically described.

When the compound (I) is, for example, a compound that generates an acid having one first acidic moiety derived from the structural moiety X and one second acidic moiety derived from the structural moiety Y, the compound PI corresponds to a "compound having $HA_1$ and $HA_2$" .

The acid dissociation constant a1 and the acid dissociation constant a2 of the compound PI will be more specifically described as follows: in determination of the acid dissociation constants of the compound PI, the pKa at the time when the compound PI turns into a "compound having $A_1^-$ and $HA_2$" is the acid dissociation constant a1, and the pKa at the time when the "compound having $A_1^-$ and $HA_2$" turns into a "compound having $A_1^-$ and $A_2^-$" is the acid dissociation constant a2.

When the compound (I) is, for example, a compound that generates an acid having two first acidic moieties derived from the structural moieties X and one second acidic moiety derived from the structural moiety Y, the compound PI corresponds to a "compound having two $HA_1$ and one $HA_2$".

In determination of the acid dissociation constants of the compound PI, the acid dissociation constant at the time when the compound PI turns into a "compound having one $A_1^-$, one $HA_1$, and one $HA_2$" and the acid dissociation constant at the time when the "compound having one $A_1^-$, one $HA_1$, and one $HA_2$" turns into a "compound having two $A_1^-$ and one $HA_2$" correspond to the above-described acid dissociation constant a1. The acid dissociation constant at the time when the "compound having two $A_1^-$ and one $HA_2$" turns into a "compound having two $A_1^-$ and $A_2^-$" corresponds to the acid dissociation constant a2. In other words, when the compound PI has a plurality of acid dissociation constants derived from the acidic moieties represented by $HA_1$ in which the cationic moiety $M_1^+$ in the structural moiety X is replaced by $H^+$, the value of the acid dissociation constant a2 is larger than the largest value among the plurality of the acid dissociation constants a1. Note that, in a case where the acid dissociation constant at the time when the compound PI turns into the "compound having one $A_1^-$, one $HA_1$, and one $HA_2$" is defined as aa, and the acid dissociation constant at the time when the "compound having one $A_1^-$, one $HA_1$, and one $HA_2$" turns into the "compound having two $A_1^-$ and one $HA_2$" is defined as ab, the relationship between aa and ab satisfies aa < ab.

The acid dissociation constant a1 and the acid dissociation constant a2 can be determined by the above-described method of measuring an acid dissociation constant.

The compound PI corresponds to an acid generated upon irradiation of the compound (I) with an actinic ray or a radiation.

When the compound (I) has two or more structural moieties X, the structural moieties X may be the same or different. The two or more $A_1^-$ and the two or more $M_1^+$ may be individually the same or different.

In the compound (I), $A_1^-$ above and $A_2^-$ above, and $M_1^+$ above and $M_2^+$ above may be individually the same or different, but $A_1^-$ above and $A_2^-$ above are preferably different from each other.

In the compound PI, the difference (absolute value) between the acid dissociation constant a1 (when a plurality of acid dissociation constants a1 are present, the maximum value thereof) and the acid dissociation constant a2 is preferably 0.1 or more, more preferably 0.5 or more, and still more preferably 1.0 or more. Note that the upper limit value of the difference (absolute value) between the acid dissociation constant a1 (when a plurality of acid dissociation constants a1 are present, the maximum value thereof) and the acid dissociation constant a2 is not particularly limited, but is, for example, 16 or less.

In the compound PI, the acid dissociation constant a2 is preferably 20 or less, and more preferably 15 or less. Note that the lower limit value of the acid dissociation constant a2 is preferably -4.0 or more.

In the compound PI, the acid dissociation constant a1 is preferably 2.0 or less, and more preferably 0 or less. Note that the lower limit value of the acid dissociation constant a1 is preferably -20.0 or more.

The anionic moiety $A_1^-$ and the anionic moiety $A_2^-$ are structural moieties including a negatively charged atom or atomic group and are, for example, structural moieties selected from the group consisting of the following formulas (AA-1) to (AA-3) and formulas (BB-1) to (BB-6).

The anionic moiety $A_1^-$ is preferably an anionic moiety that can form an acidic moiety having a small acid dissociation constant, in particular, more preferably any one of the formulas (AA-1) to (AA-3), and still more preferably any one of the formulas (AA-1) and (AA-3).

The anionic moiety $A_2^-$ is preferably an anionic moiety that can form an acidic moiety having a larger acid dissociation constant than the anionic moiety $A_1^-$, more preferably any one of the formulas (BB-1) to (BB-6), and still more preferably any one of the formulas (BB-1) and (BB-4).

Note that, in the formulas (AA-1) to (AA-3) and the formulas (BB-1) to (BB-6) below, * represent a bonding site.

In the formula (AA-2), $R^A$ represent a monovalent organic group. The monovalent organic groups represented by $R^A$ are not particularly limited, but may be, for example, a cyano group, a trifluoromethyl group, or a methanesulfonyl group.

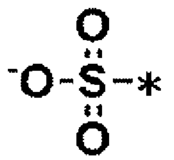

AA-1

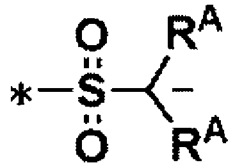

AA-2

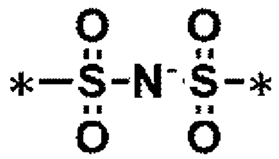

AA-3

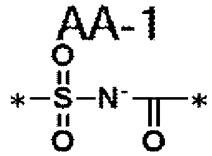

BB-1

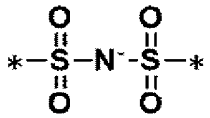

BB-2

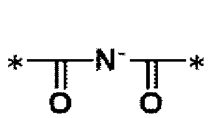

BB-3

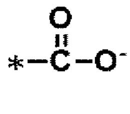

BB-4

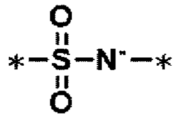

BB-5

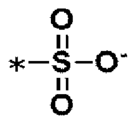

BB-6

The cationic moiety $M_1^+$ and the cationic moiety $M_2^+$ are structural moieties including a positively charged atom or atomic group and are, for example, singly charged organic cations. Note that such an organic cation may be, for example, the above-described organic cation represented by $M^+$.

The cationic moiety $M_1^+$ and the cationic moiety $M_2^+$ may each independently have a group represented by -$SF_4R$, or may not have a group represented by -$SF_4R$.

The anionic moiety $A_1^-$ and the anionic moiety $A_2^-$ may each independently have a group represented by -$SF_4R$, or may not have a group represented by -$SF_4R$.

The compound (I) is not particularly limited in terms of specific structures; examples include compounds represented by a formula (Ia-1) to a formula (Ia-5) described later.

Compound represented by formula (Ia-1)

Hereinafter, first, the compound represented by the formula (Ia-1) will be described.

$$M_{11}^{+}A_{11}^{-}\text{-}L_1\text{-}A_{12}^{-}M_{12}^{+} \qquad \text{(Ia-1)}$$

The compound represented by the formula (Ia-1) generates, upon irradiation with an actinic ray or a radiation, an acid represented by $HA_{11}$-$L_1$-$A_{12}H$.

In the formula (Ia-1), $M_{11}^+$ and $M_{12}^+$ each independently represent an organic cation.

$A_{11}^-$ and $A_{12}^-$ each independently represent a monovalent anionic functional group.

$L_1$ represents a divalent linking group.

$M_{11}^+$ and $M_{12}^+$ may be the same or different.

$A_{11}^-$ and $A_{12}^-$ may be the same or different, but are preferably different from each other.

Note that, in the formula (Ia-1), in a compound PIa ($HA_{11}$-$L_1$-$A_{12}H$) in which the cations represented by $M_{11}^+$ and $M_{12}^+$ are replaced by $H^+$, the acid dissociation constant a2 derived from the acidic moiety represented by $A_{12}H$ is larger than the acid dissociation constant a1 derived from the acidic moiety represented by $HA_{11}$. Note that preferred values of the acid dissociation constant a1 and the acid dissociation constant a2 are the same as those described above. The compound PIa is the same as the acid generated from the compound represented by the formula (Ia-1) upon irradiation with an actinic ray or a radiation.

At least one of $M_{11}^+$, $M_{12}^+$, An', $A_{12}^-$, or $L_1$ may have, as a substituent, an acid-decomposable group.

In the formula (Ia-1), examples of the organic cations represented by $M_{11}^+$ and $M_{12}^+$ include the above-described organic cations represented by $M^+$.

The organic cations represented by $M_{11}^+$ and $M_{12}^+$ may have a group represented by -$SF_4R$, or may not have a group represented by -$SF_4R$.

The monovalent anionic functional group represented by An' means a monovalent group including the above-described anionic moiety $A_1^-$. The monovalent anionic functional group represented by $A_{12}^-$ means a monovalent group including the above-described anionic moiety $A_2^-$.

The monovalent anionic functional groups represented by A11$^-$ and $A_{12}^-$ are preferably monovalent anionic functional groups including the anionic moiety of any one of the above-described formulas (AA-1) to (AA-3) and formulas (BB-1) to (BB-6), and more preferably monovalent anionic functional groups selected from the group consisting of formulas (AX-1) to (AX-3) and formulas (BX-1) to (BX-7). In particular, the monovalent anionic functional group represented by $A_{11}^-$ is preferably the monovalent anionic functional group represented by any one of the formulas (AX-1) to (AX-3). In particular, the monovalent anionic functional group represented by $A_{12}^-$ is preferably the monovalent anionic functional group represented by any one of the formulas (BX-1) to (BX-7), and more preferably the monovalent anionic functional group represented by any one of the formulas (BX-1) to (BX-6).

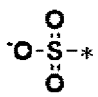
AX-1
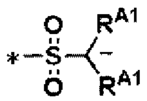
AX-2
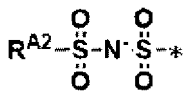
AX-3

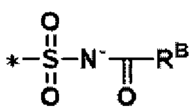
BX-1
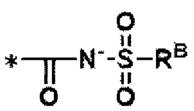
BX-2
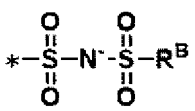
BX-3
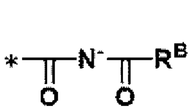
BX-4
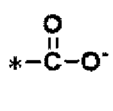
BX-5
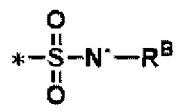
BX-6
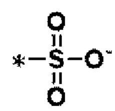
BX-7

In the formulas (AX-1) to (AX-3), $R^{A1}$ and $R^{A2}$ each independently represent a monovalent organic group. * represent a bonding site.

The monovalent organic groups represented by $R^{A1}$ are not particularly limited, but may be, for example, a cyano group, a trifluoromethyl group, or a methanesulfonyl group.

The monovalent organic group represented by $R^{A2}$ is preferably a linear, branched, or cyclic alkyl group, or an aryl group.

The number of carbon atoms of the alkyl group is preferably 1 to 15, more preferably 1 to 10, and still more preferably 1 to 6.

The alkyl group may have a substituent. The substituent is preferably a fluorine atom or a cyano group, and more preferably a fluorine atom. When the alkyl group has, as a substituent, a fluorine atom, it may be a perfluoroalkyl

group

The aryl group is preferably a phenyl group or a naphthyl group, and more preferably a phenyl group.

The aryl group may have a substituent. The substituent is preferably a fluorine atom, an iodine atom, a perfluoroalkyl group (for example, preferably having 1 to 10 carbon atoms, and more preferably 1 to 6 carbon atoms), or a cyano group, and more preferably a fluorine atom, an iodine atom, or a perfluoroalkyl group.

The monovalent organic group represented by $R^{A2}$ may have a group represented by $-SF_4R$, or may not have a group represented by $-SF_4R$.

In the formulas (BX-1) to (BX-4) and the formula (BX-6), $R^B$ represent a monovalent organic group. * represent a bonding site.

The monovalent organic groups represented by $R^B$ are preferably a linear, branched, or cyclic alkyl group, or an aryl group.

The number of carbon atoms of the alkyl group is preferably 1 to 15, more preferably 1 to 10, and still more preferably 1 to 6.

The alkyl group may have a substituent. The substituent is not particularly limited, but the substituent is preferably a group represented by $-SF_4R$, a fluorine atom, or a cyano group, and more preferably a fluorine atom. When the alkyl group has, as a substituent, a fluorine atom, it may be a perfluoroalkyl group.

Note that, in the alkyl group, when a carbon atom serving as a bonding site has a substituent, it is also preferably a substituent other than a fluorine atom and a cyano group. In the alkyl group, the carbon atom serving as the bonding site corresponds to, for example, in the case of the formulas (BX-1) and (BX-4), a carbon atom (in such an alkyl group) directly bonded to -CO- clearly described in the formula; in the case of the formulas (BX-2) and (BX-3), a carbon atom (in such an alkyl group) directly bonded to $-SO_2-$ clearly described in the formula; in the case of the formula (BX-6), a carbon atom (in the alkyl group) directly bonded to $N^-$ clearly described in the formula.

In the alkyl group, a carbon atom may be substituted with a carbonyl carbon.

The aryl group is preferably a phenyl group or a naphthyl group, and more preferably a phenyl group.

The aryl group may have a substituent. The substituent is preferably a fluorine atom, an iodine atom, a perfluoroalkyl group (for example, preferably having 1 to 10 carbon atoms, and more preferably having 1 to 6 carbon atoms), a cyano group, a group represented by $-SF_4R$, an alkyl group (for example, preferably having 1 to 10 carbon atoms, and more preferably having 1 to 6 carbon atoms), an alkoxy group (for example, preferably having 1 to 10 carbon atoms, and more preferably having 1 to 6 carbon atoms), or an alkoxycarbonyl group (for example, preferably having 2 to 10 carbon atoms, and more preferably having 2 to 6 carbon atoms), and more preferably a fluorine atom, an iodine atom, a perfluoroalkyl group, an alkyl group, an alkoxy group, or an alkoxycarbonyl group.

The monovalent organic group represented by $R^{A2}$ may have a group represented by $-SF_4R$, or may not have a group represented by $-SF_4R$.

In the formula (Ia-1), the divalent linking group represented by $L_1$ is not particularly limited, and examples thereof include -CO-, -NR-, -O-, -S-, -SO-, $-SO_2-$, an alkylene group (that preferably has 1 to 6 carbon atoms and may be linear or branched), a cycloalkylene group (preferably having 3 to 15 carbon atoms), an alkenylene group (preferably having 2 to 6 carbon atoms), a divalent aliphatic heterocyclic group (having preferably a 5- to 10-membered ring, more preferably a 5- to 7-membered ring, and still more preferably a 5- to 6-membered ring that have at least one N atom, O atom, S atom, or Se atom in the ring structure), a divalent aromatic heterocyclic group (having preferably a 5- to 10-membered ring, more preferably a 5- to 7-membered ring, and still more preferably a 5- to 6-membered ring that have at least one N atom, O atom, S atom, or Se atom in the ring structure), a divalent aromatic hydrocarbon cyclic group (having preferably a 6- to 10-membered ring, and more preferably a 6-membered ring), and divalent linking groups that are combinations of a plurality of the foregoing. R above may be a hydrogen atom or a monovalent organic group. The monovalent organic group is not particularly limited, but is preferably, for example, an alkyl group (preferably having 1 to 6 carbon atoms).

The alkylene group, the cycloalkylene group, the alkenylene group, the divalent aliphatic heterocyclic group, the divalent aromatic heterocyclic group, and the divalent aromatic hydrocarbon cyclic group may have a substituent. Examples of the substituent include halogen atoms (preferably a fluorine atom).

In particular, the divalent linking group represented by $L_1$ is preferably a divalent linking group represented by a formula (L1).

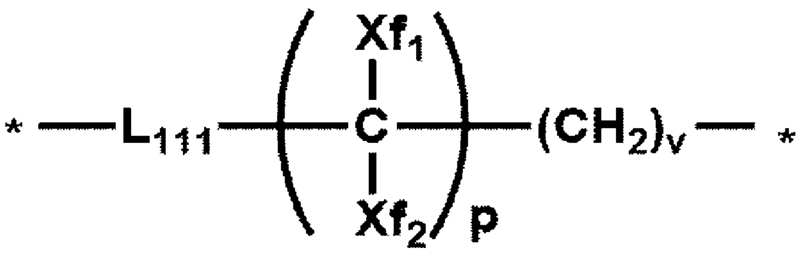

(L1)

In the formula (L1), $L_{111}$ represents a single bond or a divalent linking group.

The divalent linking group represented by $L_{111}$ is not particularly limited, and examples thereof include -CO-, -NH-, -O-, -SO-, $-SO_2-$, an alkylene group that may have a substituent (that more preferably has 1 to 6 carbon atoms and may be linear or branched), a cycloalkylene group that may have a substituent (that preferably has 3 to 15 carbon atoms), an aryl group that may have a substituent (that preferably has 6 to 10 carbon atoms), and divalent linking groups that are combinations of a plurality of the foregoing. The substituent is not particularly limited, and may be, for example, a halogen atom.

p represents an integer of 0 to 3, and preferably represents an integer of 1 to 3.

v represents an integer of 0 or 1.

$Xf_1$ each independently represent a fluorine atom or an alkyl group substituted with at least one fluorine atom. The alkyl group preferably has 1 to 10 carbon atoms, and more preferably 1 to 4 carbon atoms. The alkyl group substituted with at least one fluorine atom is preferably a perfluoroalkyl group.

$Xf_2$ each independently represent a hydrogen atom, an alkyl group that may have, as a substituent, a fluorine atom, or a fluorine atom. The alkyl group preferably has 1 to 10 carbon atoms, and more preferably 1 to 4 carbon atoms. In particular, $Xf_2$ preferably represent a fluorine atom or an alkyl group substituted with at least one fluorine atom, and more preferably a fluorine atom or a perfluoroalkyl group.

In particular, $Xf_1$ and $Xf_2$ are each independently preferably a fluorine atom or a perfluoroalkyl group having 1 to 4 carbon atoms, and more preferably a fluorine atom or $CF_3$. In particular, $Yf_1$ and $Xf_2$ are each still more preferably a fluorine atom.

**[0484]** * represent a bonding site.

When, in the formula (Ia-1), $L_{11}$ represents a divalent linking group represented by the formula (L1), the $L_{111}$-side direct bond (*) in the formula (L1) is preferably bonded to $A_{12}^-$ in the formula (Ia-1).

Compounds represented by formulas (Ia-2) to (Ia-4)

Hereinafter, compounds represented by formulas (Ia-2) to (Ia-4) will be described.

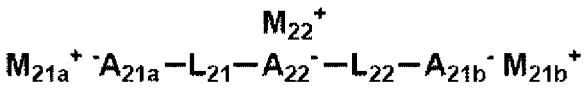

(Ia-2)

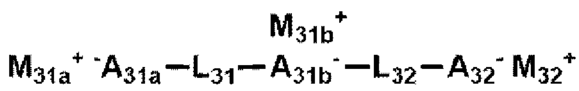

(Ia-3)

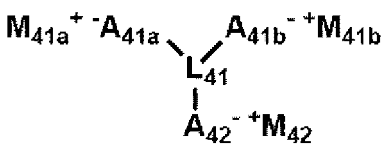

(Ia-4)

In the formula (Ia-2), $A_{21a}^-$ and $A_{21b}^-$ each independently represent a monovalent anionic functional group. For $A_{21a}^-$ and $A_{21b}^-$, the monovalent anionic functional group means a monovalent group including the above-described anionic moiety $A_1^-$. For $A_{21a}^-$ and $A_{21b}^-$, the monovalent anionic functional group is not particularly limited, but may be, for example, a monovalent anionic functional group selected from the group consisting of the above-described formulas (AX-1) to (AX-3).

$A_{22}^-$ represents a divalent anionic functional group. The divalent anionic functional group represented by $A_{22}^-$ means a divalent linking group including the above-described anionic moiety $A_2^-$. For the divalent anionic functional group represented by $A_{22}^-$, examples include divalent anionic functional groups represented by the following formulas (BX-8) to (BX-11).

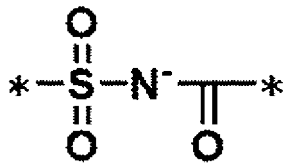

BX-8

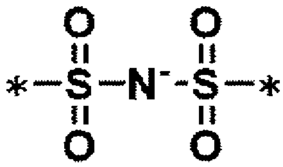

BX-9

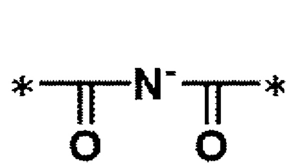

BX-10

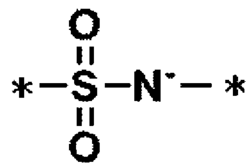

BX-11

$M_{21a}^+$, $M_{21b}^+$, and $M_{22}^+$ each independently represent an organic cation. The organic cations represented by $M_{21a}^+$, $M_{21b}^+$, and $M_{22}^+$ have the same definitions and preferred examples as those of $M_{11}^+$ described above.

$L_{21}$ and $L_{22}$ each independently represent a divalent organic group.

In a compound PIa-2 in which organic cations represented by $M_{21a}^+$, $M_{21b}^+$, and $M_{22}^+$ in the formula (Ia-2) are replaced by $H^+$, the acid dissociation constant a2 derived from the acidic moiety represented by $A_{22}H$ is larger than the acid dissociation constant a 1-1 derived from $A_{21a}H$ and the acid dissociation constant a1-2 derived from the acidic moiety represented by $A_{21b}H$. Note that the acid dissociation constant a1-1 and the acid dissociation constant a1-2 correspond to the above-described acid dissociation constant a1.

Note that $A_{21a}^-$ and $A_{21b}^-$ may be the same or different. $M_{21a}^+$, $M_{21b}^+$, and $M_{22}^+$ may be the same or different.

At least one of $M_{21a}^+$, $M_{21b}^+$, $M_{22}^+$, $A_{21a}^-$, $A_{21b}^-$, $L_{21}$, or $L_{22}$ may have, as a substituent, an acid-decomposable group

In the formula (Ia-3), $A_{31a}^-$ and $A_{32}^-$ each independently represent a monovalent anionic functional group. Note that the monovalent anionic functional group represented by $A_{31a}^-$ has the same definition and preferred examples as the above-described $A_{21a}^-$ and $A_{21b}^-$ in the formula (Ia-2).

The monovalent anionic functional group represented by $A_{32}^-$ means a monovalent group including the above-described anionic moiety $A_2^-$. The monovalent anionic functional group represented by $A_{32}^-$ is not particularly limited, but may be, for example, a monovalent anionic functional group selected from the group consisting of the above-described formulas (BX-1) to (BX-7).

$A_{31b}^-$ represents a divalent anionic functional group. The divalent anionic functional group represented by $A_{31b}^-$ means a divalent linking group including the above-described anionic moiety $A_1^-$. The divalent anionic functional group represented by $A_{31b}^-$ may be, for example, a divalent anionic functional group represented by the following formula (AX-4).

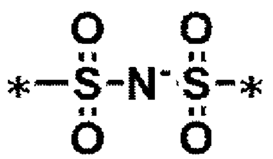

AX-4

$M_{31a}^+$, $M_{31b}^+$, and $M_{32}^+$ each independently represent a monovalent organic cation. The organic cations represented by $M_{31a}^+$, $M_{31b}^+$, and $M_{32}^+$ have the same definitions and preferred examples as $M_{11}^+$ described above.

$L_{31}$ and $L_{32}$ each independently represent a divalent organic group.

In a compound PIa-3 in which organic cations represented by $M_{31a}^+$, $M_{31b}^+$, and $M_{32}^+$ are replaced by $H^+$ in the above-described formula (Ia-3), the acid dissociation constant a2 derived from the acidic moiety represented by $A_{32}H$ is larger than the acid dissociation constant a1-3 derived from the acidic moiety represented by $A_{31a}H$ and the acid dissociation constant a1-4 derived from the acidic moiety represented by $A_{31b}H$. Note that the acid dissociation constant $a_{1-3}$ and the acid dissociation constant a 1-4 correspond to the above-described acid dissociation constant a1.

Note that $A_{31a}^-$ and $A_{32}^-$ may be the same or different. $M_{31a}^+$, $M_{31b}^+$, and $M_{32}^+$ may be the same or different.

At least one of $M_{31a}^+$, $M_{31b}^+$, $M_{32}^+$, $A_{31a}^-$, $A_{32}^-$, $L_{31}$, or $L_{32}$ may have, as a substituent, an acid-decomposable group

In the formula (Ia-4), $A_{41a}^-$, $A_{41b}^-$, and $A_{42}^-$ each independently represent a monovalent anionic functional group. Note that, for $A_{41a}^-$ and $A_{41b}^-$, the monovalent anionic functional group has the same definition as the above-described $A_{21a}^-$ and $A_{21b}^-$ in the formula (Ia-2). The monovalent anionic functional group represented by $A_{42}^-$ has the same definition and preferred examples as the above-described $A_{32}^-$ in the formula (Ia-3).

$M_{41a}^+$, $M_{41b}^+$, and $M_{42}^+$ each independently represent an organic cation. The organic cations represented by $M_{41a}^+$, $M_{41b}^+$, and $M_{42}^+$ have the same definitions and preferred examples as $M_{11}^+$ described above.

The organic cations represented by $M_{41a}^+$, $M_{41b}^+$, and $M_{42}^+$ may have a group represented by $-SF_4R$, or may

not have a group represented by $-SF_4R$.

$L_{41}$ represents a trivalent organic group.

In a compound PIa-4 in which the organic cations represented by $M_{41a}^+$, $M_{41b}^+$, and $M_{42}^+$ in the above-described formula (Ia-4) are replaced by $H^+$, the acid dissociation constant a2 derived from the acidic moiety represented by $A_{42}H$ is larger than the acid dissociation constant a1-5 derived from the acidic moiety represented by $A_{41a}H$ and the acid dissociation constant a1-6 derived from the acidic moiety represented by $A_{41b}H$. Note that the acid dissociation constant a1-5 and the acid dissociation constant a1-6 correspond to the above-described acid dissociation constant a1.

Note that $A_{41a}^-$, $A_{41b}^-$, and $A_{42}^-$ may be the same or different. $M_{41a}^+$, $M_{41b}^+$, and $M_{42}^+$ may be the same or different.

At least one of $M_{41a}^+$, $M_{41b}^+$, $M_{42}^+$, $A_{41a}^-$, $A_{41b}^-$, $A_{42}^-$, or $L_{41}$ may have, as a substituent, an acid-decomposable group.

For $L_{21}$ and $L_{22}$ in the formula (Ia-2) and $L_{31}$ and $L_{32}$ in the formula (Ia-3), the divalent organic group is not particularly limited, and examples thereof include -CO-, -NR-, -O-, -S-, -SO-, $-SO_2-$, an alkylene group (that preferably has 1 to 6 carbon atoms and may be linear or branched), a cycloalkylene group (preferably having 3 to 15 carbon atoms), an alkenylene group (preferably having 2 to 6 carbon atoms), a divalent aliphatic heterocyclic group (having preferably a 5- to 10-membered ring, more preferably a 5- to 7-membered ring, and still more preferably a 5- to 6-membered ring that have at least one N atom, O atom, S atom, or Se atom in the ring structure), a divalent aromatic heterocyclic group (having preferably a 5- to 10-membered ring, more preferably a 5- to 7-membered ring, and still more preferably a 5- to 6-membered ring that have at least one N atom, O atom, S atom, or Se atom in the ring structure), a divalent aromatic hydrocarbon cyclic group (having preferably a 6- to 10-membered ring, and still more preferably a 6-membered ring), and divalent organic groups that are combinations of a plurality of the foregoing. In - NR- above, R may be a hydrogen atom or a monovalent organic group. The monovalent organic group is not particularly limited, but is preferably, for example, an alkyl group (preferably having 1 to 6 carbon atoms).

The alkylene group, the cycloalkylene group, the alkenylene group, the divalent aliphatic heterocyclic group, the divalent aromatic heterocyclic group, and the divalent aromatic hydrocarbon cyclic group may have a substituent. Examples of the substituent include halogen atoms (preferably a fluorine atom).

For $L_{21}$ and $L_{22}$ in the formula (Ia-2) and $L_{31}$ and $L_{32}$ in the formula (Ia-3), the divalent organic group is also preferably, for example, a divalent organic group represented by the following formula (L2).

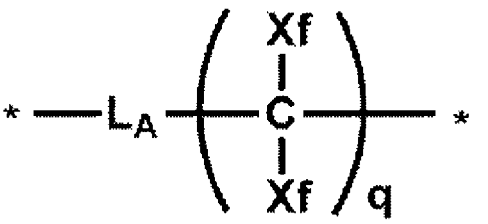

(L2)

In the formula (L2), q represents an integer of 1 to 3. * represent a bonding site.

Xf's each independently represent a fluorine atom or an alkyl group substituted with at least one fluorine atom. The alkyl group preferably has 1 to 10 carbon atoms, and more preferably 1 to 4 carbon atoms. The alkyl group substituted with at least one fluorine atom is preferably a perfluoroalkyl group.

Xf's are preferably a fluorine atom or a perfluoroalkyl group having 1 to 4 carbon atoms, and more preferably a fluorine atom or $CF_3$. In particular, still more preferably, both of Xf's are fluorine atoms.

$L_A$ represents a single bond or a divalent linking group.

The divalent linking group represented by $L_A$ is not particularly limited, and examples thereof include -CO-, -O-, -SO-, $-SO_2-$, an alkylene group (that preferably has 1 to 6 carbon atoms and may be linear or branched), a cycloalkylene group (preferably having 3 to 15 carbon atoms), a divalent aromatic hydrocarbon cyclic group (preferably a 6- to 10-membered ring, and more preferably a 6-membered ring), and divalent linking groups that are combinations of a plurality of the foregoing.

The alkylene group, the cycloalkylene group, and the divalent aromatic hydrocarbon cyclic group may have a substituent. Examples of the substituent include halogen atoms (preferably a fluorine atom).

Examples of the divalent organic group represented by the formula (L2) include $*-CF_2-*$, $*-CF_2-CF_2-*$, $*-CF_2-CF_2-CF_2-*$, $*-Ph-O-SO_2-CF_2-*$, $*-Ph-O-SO_2-CF_2-CF_2-*$, $*-Ph-O-SO_2-CF_2-CF_2-CF_2-*$, and $*-Ph-OCO-CF_2-*$. Note that Ph are a phenylene group that may have a substituent, and preferably a 1,4-phenylene group. The substituent is not particularly limited, but is preferably an alkyl group (for example, preferably having 1 to 10 carbon atoms, and more preferably having 1 to 6 carbon atoms), an alkoxy group (for example, preferably having 1 to 10 carbon atoms, and more preferably having 1 to 6 carbon atoms), or an alkoxycarbonyl group (for example, preferably having 2 to 10 carbon atoms, and more preferably having 2 to 6 carbon atoms).

When $L_{21}$ and $L_{22}$ in the formula (Ia-2) represent the divalent organic group represented by the formula (L2), the $L_A$-side direct bond (*) in the formula (L2) is preferably bonded to $A_{21a}^-$ and $A_{21b}^-$ in the formula (Ia-2).

When $L_{31}$ and $L_{32}$ in the formula (Ia-3) represent the divalent organic group represented by the formula (L2), the $L_A$-side direct bond (*) in the formula (L2) is preferably bonded to $A_{31a}^-$ and $A_{32}^-$ in the formula (Ia-3). Compound represented by formula (Ia-5)

Hereinafter, the formula (Ia-5) will be described.

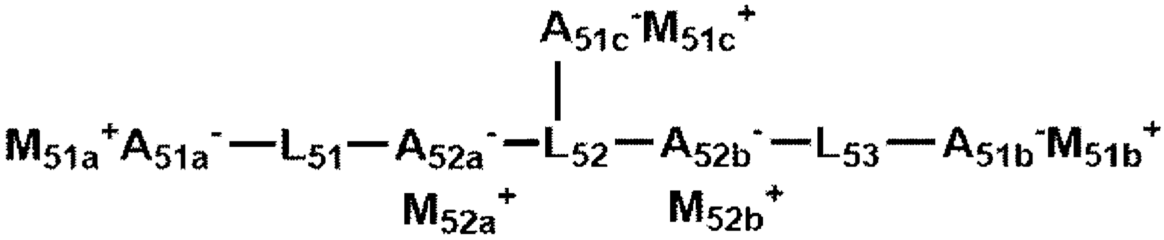

(Ia-5)

In the formula (Ia-5), $A_{51a}^-$, $A_{51b}^-$, and $A_{51c}^-$ each independently represent a monovalent anionic functional group. For $A_{51a}^-$, $A_{51b}^-$, and $A_{51c}^-$, the monovalent anionic functional group means a monovalent group including the above-described anionic moiety $A_1^-$. For $A_{51a}^-$, $A_{51b}^-$, and $A_{51c}^-$, the monovalent anionic functional group is not particularly limited, but may be, for example, a monovalent anionic functional group selected from the group consisting of the above-described formulas (AX-1) to (AX-3).

$A_{52a}^-$ and $A_{52b}^-$ represent a divalent anionic functional group. For $A_{52a}^-$ and $A_{52b}^-$, the divalent anionic functional group means a divalent linking group including the above-described anionic moiety $A_2^-$. The divalent anionic functional group represented by $A_{22}^-$ may be, for example, a divalent anionic functional group selected from the group consisting of the above-described formulas (BX-8) to (BX-11).

$M_{51a}^+$, $M_{51b}^+$, $M_{51c}^+$, $M_{52a}^+$, and $M_{52b}^+$ each independently represent an organic cation. The organic cations represented by $M_{51a}^+$, $M_{51b}^+$, $M_{51c}^+$, $M_{52a}^+$, and $M_{52b}^+$ have the same definitions and preferred examples as $M_{11}^+$ described above.

$L_{51}$ and $L_{53}$ each independently represent a divalent organic group. For $L_{51}$ and $L_{53}$, the divalent organic group has the same definition and preferred examples as the above-described $L_{21}$ and $L_{22}$ in the formula (Ia-2).

$L_{52}$ represents a trivalent organic group. The trivalent organic group represented by $L_{52}$ has the same definition and preferred examples as the above-described $L_{41}$ in the formula (Ia-4).

In a compound PIa-5 in which organic cations represented by $M_{51a}^+$, $M_{51b}^+$, $M_{51c}^+$, $M_{52a}^+$, and $M_{52b}^+$ in the above-described formula (Ia-5) are replaced by $H^+$, the acid dissociation constant a2-1 derived from the acidic moiety represented by $A_{52a}H$ and the acid dissociation constant a2-2 derived from the acidic moiety represented by $A_{52b}H$ are larger than the acid dissociation constant a 1-1 derived from $A_{51a}H$, the acid dissociation constant a1-2 derived from the acidic moiety represented by $A_{51bH,}$ and the acid dissociation constant a1-3 derived from the acidic moiety represented by $A_{51c}H$. Note that the acid dissociation constants a1-1 to a1-3 correspond to the above-described acid dissociation constant a1, and the acid dissociation constants a2-1 and a2-2 correspond to the above-described acid dissociation constant a2.

Note that $A_{51a}^-$, $A_{51b}^-$, and $A_{51c}^-$ may be the same or different. $A_{52a}^-$ and $A_{52b}^-$ may be the same or different.

$M_{51a}^+$, $M_{51b}^+$, $M_{51c}^+$, $M_{52a}^+$, and $M_{52b}^+$ may be the same or different.

At least one of $M_{51b}^+$, $M_{51c}^+$, $M_{52a}^+$, $M_{52b}^+$, $A_{51a}^-$, $A_{51b}^-$, $A_{51c}^-$, $L_{51}$, $L_{52}$, or $L_{53}$ may have, as a substituent, an acid-decomposable group.

Compound (II)

The compound (II) is a compound having two or more structural moieties X described above and one or more structural moieties Z described below, and is a compound that generates an acid including, is a compound that generates, upon irradiation with an actinic ray or a radiation, an acid including two or more first acidic moieties derived from the above-described structural moieties X and the above-described structural moiety Z.

Structural moiety Z: a nonionic moiety that can neutralize acid

In the compound (II), the definition of the structural moiety X and the definitions of $A_1^-$ and $M_1^+$ are the same as the definition of the structural moiety X and the definitions of $A_1^-$ and $M_1^+$ in the above-described compound (I), and preferred examples are also the same.

The cationic moiety $M_1^+$ may have a group represented by $-SF_4R$, or may not have a group represented by $-SF_4R$.

The anionic moieties $A_1^-$ may each independently have a group represented by $-SF_4R$, or may not have a group represented by $-SF_4R$.

In a compound PII in which the cationic moiety $M_1^+$ in the structural moiety X in the compound (II) is replaced by $H^+$, the preferred range of the acid dissociation constant a1 derived from the acidic moiety represented by $HA_1$ in which the cationic moiety $M_1^+$ in the structural moiety X is replaced by $H^+$ is the same as in the acid dissociation constant a1 in the compound PI.

Note that, when the compound (II) is, for example, a compound that generates an acid having two first acidic moieties derived from the structural moiety X and the structural moiety Z, the compound PII corresponds to a "compound having two $HA_1$". In determination of the acid dissociation constants of this compound PII, the acid dissociation constant at the time when the compound PII turns into a "compound having one $A^-$ and one $HA_1$" and the acid dissociation constant at the time when the "compound having one $A_1^-$ and one $HA_1$" turns into a "compound having two $A_1^-$" correspond to the acid dissociation constant a1 .

The acid dissociation constant a1 can be determined by the above-described method of measuring an acid dissociation constant.

The compound PII corresponds to an acid generated upon irradiation of the compound (II) with an actinic ray or a radiation.

Note that the two or more structural moieties X may be the same or different. The two or more $A_1^-$ and the two or more $M_1^+$ may be individually the same or different.

The nonionic moiety that can neutralize acid in the structural moiety Z is not particularly limited, and is preferably, for example, a moiety including a group that can electrostatically interact with a proton or a functional group having an electron.

Examples of the group that can electrostatically interact with a proton or the functional group having an electron include a functional group having a macrocyclic structure such as cyclic polyether, and a functional group having a nitrogen atom having an unshared electron pair that does not contribute to Π-conjugation. Examples of the nitrogen atom having an unshared electron pair that does not contribute to Π-conjugation include nitrogen atoms having partial structures represented by the following formulas.

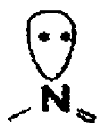
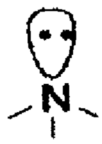
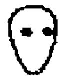

Unshared electron pair

The partial structure of the group that can electrostatically interact with a proton or the functional group having an electron may be, for example, a crown ether structure, an azacrown ether structure, a primary to tertiary amine structure, a pyridine structure, an imidazole structure, or a pyrazine structure; in particular, preferred are primary to tertiary amine structures.

The compound (II) is not particularly limited, but examples thereof include compounds represented by the following formula (IIa-1) and the following formula (IIa-2).

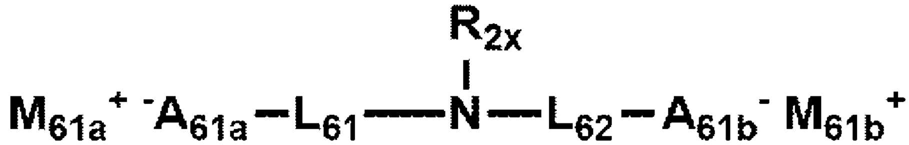

(IIa-1)

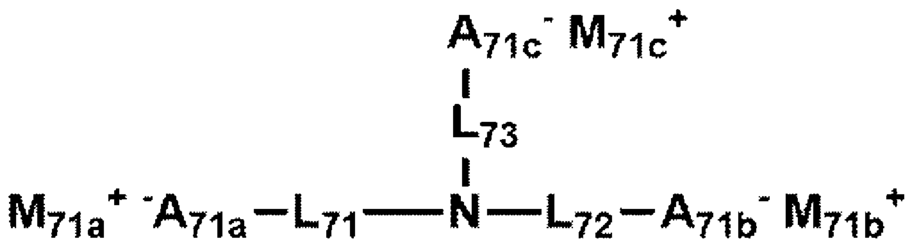

(IIa-2)

In the above-described formula (IIa-1), $A_{61a}^-$ and $A_{61b}^-$ have the same definitions and preferred examples as $A_{11}^-$ in the above-described formula (Ia-1). $M_{61a}^+$ and $M_{61b}^+$ have the same definitions and preferred examples as $M_{11}^+$ in the above-described formula (Ia-1).

In the above-described formula (IIa-1), $L_{61}$ and $L_{62}$ have the same definitions and preferred examples as $L_1$ in the above-described formula (Ia-1).

In the formula (IIa-1), $R_{2X}$ represents a monovalent organic group. The monovalent organic group represented by $R_{2X}$ is not particularly limited and may be an alkyl group (that preferably has 1 to 10 carbon atoms and may be linear or branched), a cycloalkyl group (preferably having 3 to 15 carbon atoms), or an alkenyl group (preferably having 2 to 6 carbon atoms). In the monovalent organic group represented by $R_{2X}$, $-CH_2-$ included in the alkyl group, cycloalkyl group, or alkenyl group may be substituted with one or a combination of two or more selected from the group consisting of -CO-, -NH-, -O-, -S-, -SO-, and $-SO_2-$.

The alkylene group, the cycloalkylene group, and the alkenylene group may have a substituent. The substituent is not particularly limited, but may be, for example, a halogen atom (preferably a fluorine atom).

In a compound PIIa-1 in which the organic cations represented by $M_{61a}^+$ and $M_{61b}^+$ in the above-described formula (IIa-1) are replaced by $H^+$, the acid dissociation constant a1-7 derived from the acidic moiety represented by $A_{61a}H$ and the acid dissociation constant a1-8 derived from the acidic moiety represented by $A_{61b}H$ correspond to the above-described acid dissociation constant a1.

Note that, in the above-described compound (IIa-1), the compound PIIa-1 in which the cationic moieties $M_{61a}^+$ and $M_{61b}^+$ in the structural moieties X are replaced by $H^+$ corresponds to $HA_{61a}-L_{61}-N(R_{2X})-L_{62}-A_{61b}H$. The compound PIIa-1 is the same as the acid generated from the compound represented by the formula (IIa-1) upon irradiation with an actinic ray or a radiation.

At least one of $M_{61a}^+$, $M_{61b}^+$, $A_{61a}^-$, $A_{61b}^-$, $L_{61}$, $L_{62}$, or $R_{2X}$ may have, as a substituent, an acid-decomposable group

In the above-described formula (IIa-2), $A_{71a}^-$, $A_{71b}^-$, and $A_{71c}^-$ have the same definitions and preferred examples as $A_{11}^-$ in the above-described formula (Ia-1). $M_{71a}^+$, $M_{71b}^+$, and $M_{71c}^+$ have the same definitions and preferred examples as $M_{11}^+$ in the above-described formula (Ia-1).

In the above-described formula (IIa-2), $L_{71}$, $L_{72}$, and $L_{73}$ individually have the same definitions and preferred examples as $L_1$ in the above-described formula (Ia-1).

In a compound PIIa-2 in which the organic cations represented by $M_{71a}^+$, $M_{71b}^+$, and $M_{71c}^+$ in the above-described formula (IIa-2) are replaced by $H^+$, the acid dissociation constant a1-9 derived from the acidic moiety represented by $A_{71a}H$, the acid dissociation constant a1-10 derived from the acidic moiety represented by $A_{71b}H$, and the acid dissociation constant a 1-11 derived from the acidic moiety represented by $A_{71c}H$ correspond to the above-described acid dissociation constant a1.

Note that, in the compound (IIa-1), the compound PIIa-2 in which the cationic moieties $M_{71a}^+$, $M_{71b}^+$, and $M_{71c}^+$ in the structural moieties X are replaced by $H^+$ corresponds to $HA_{71a}-L_{71}-N(L_{73}-A_{71c}H)-L_{72}-A_{71b}H$. The compound PIIa-2 is the same as the acid generated from the compound represented by the formula (IIa-2) upon irradiation with an actinic ray or a radiation.

At least one of $M_{71a}^+$, $M_{71b}^+$, $M_{71c}^+$, $A_{71a}^-$, $A_{71b}^-$, $A_{71c}^-$, $L_{71}$, $L_{72}$, or $L_{73}$ may have, as a substituent, an acid-decomposable group.

Examples of the non-cationic moieties that the compound (I) and the compound (II) can have are as follows.

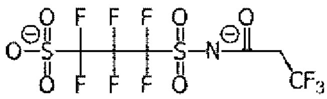
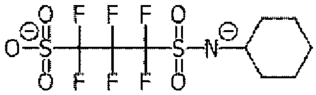
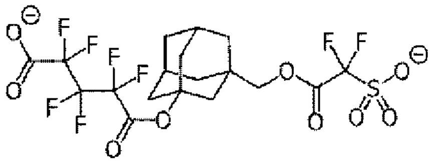
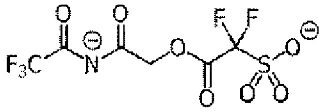
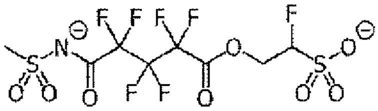
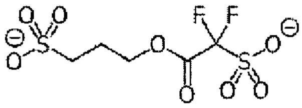
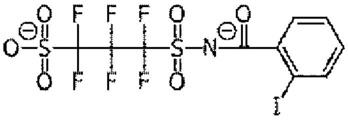
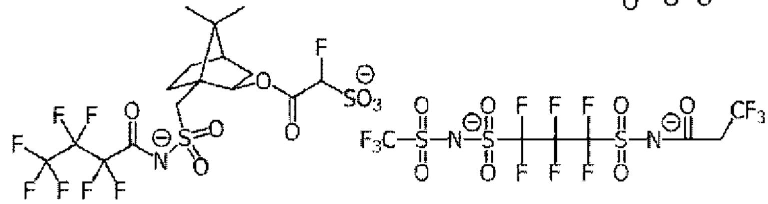
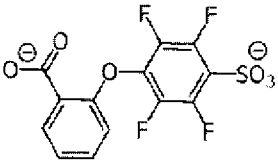
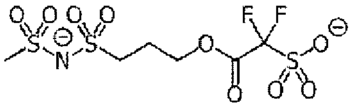
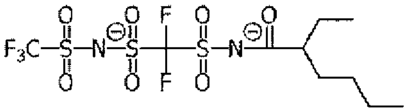
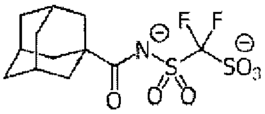
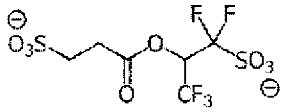
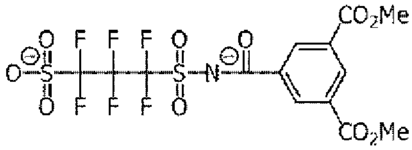
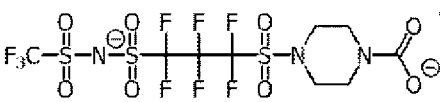
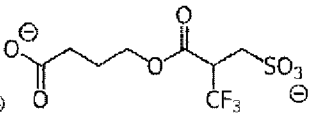
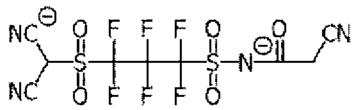
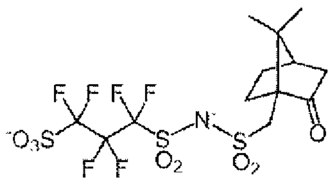
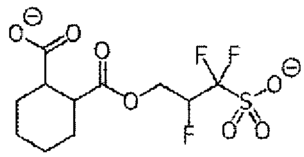
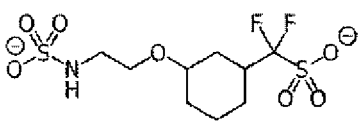
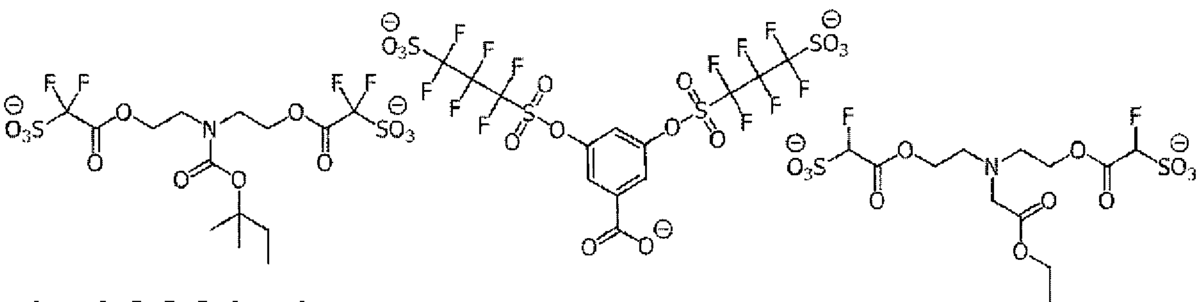
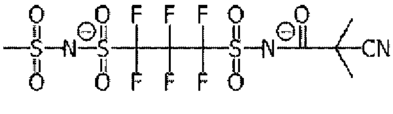

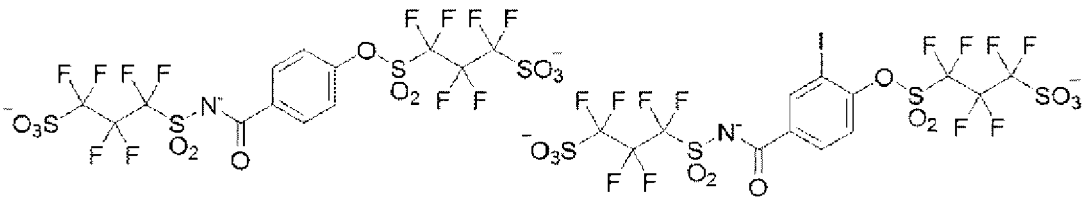

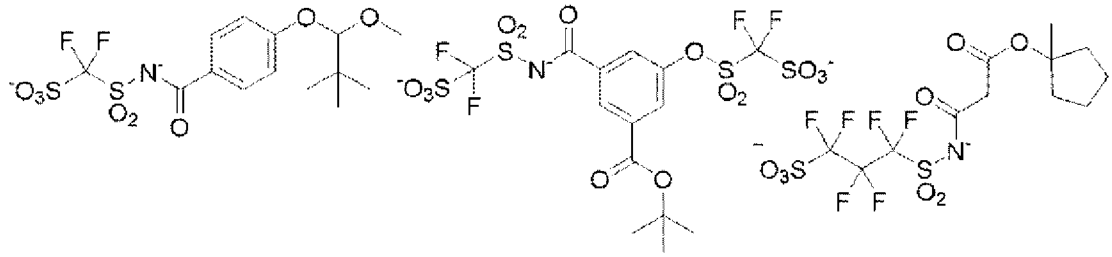

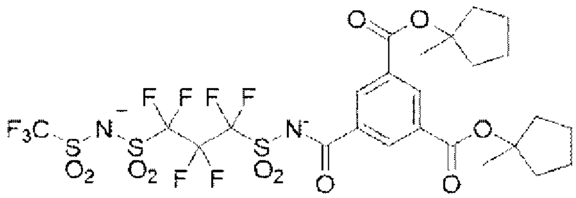

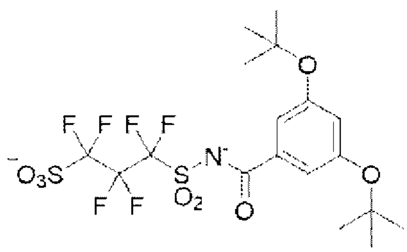

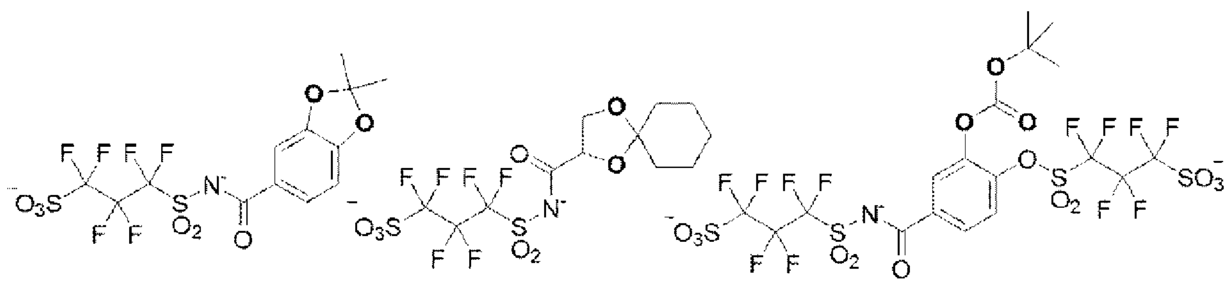

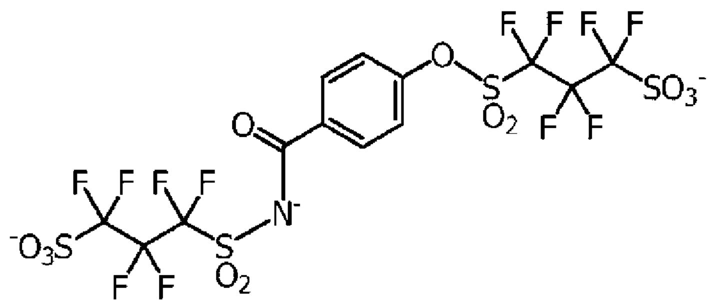

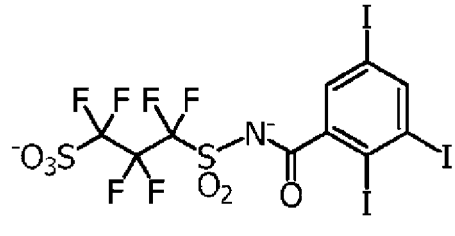

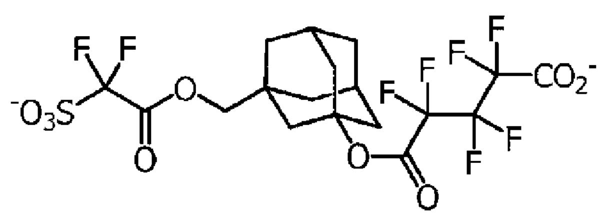

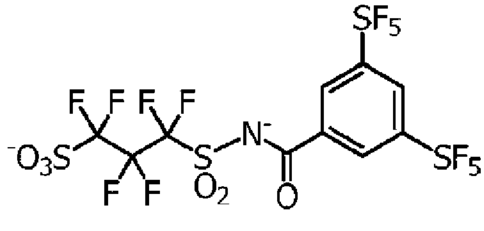

The following are non-limiting specific examples of the photoacid generator.

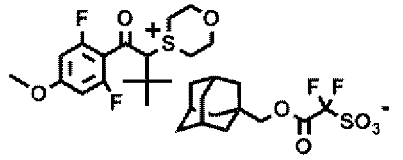

X-1

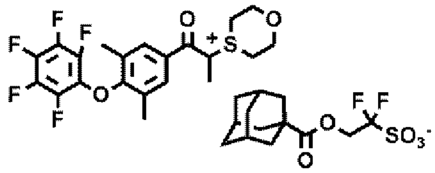

X-2

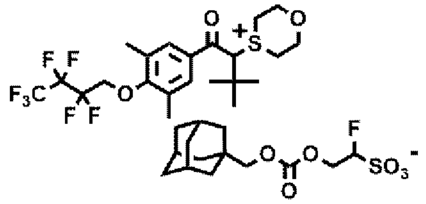

X-3

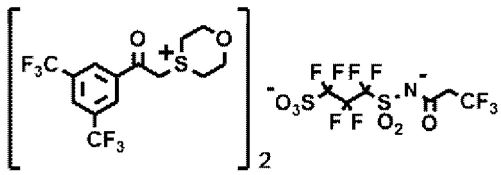

X-4

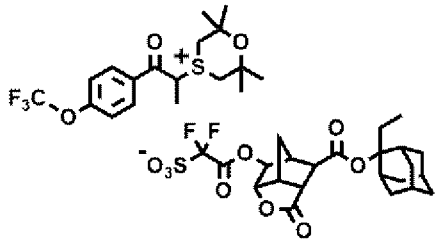

X-5

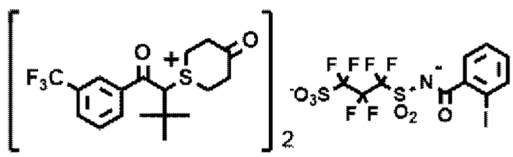

X-6

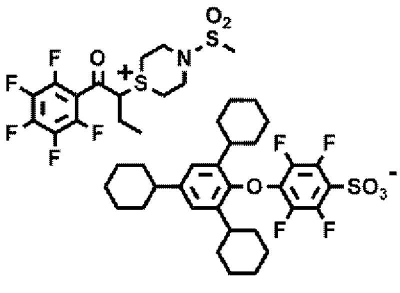

X-7

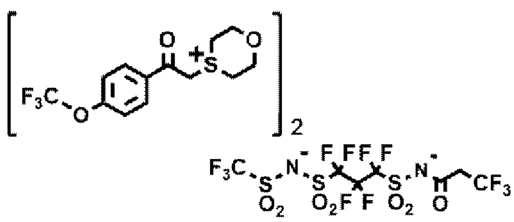

X-8

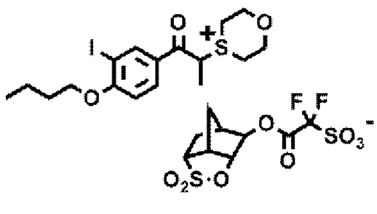

X-9

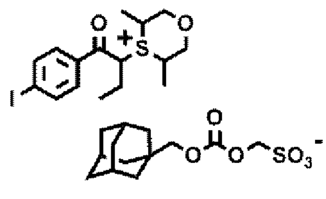

X-10

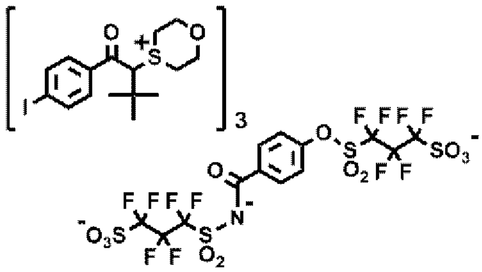

X-11

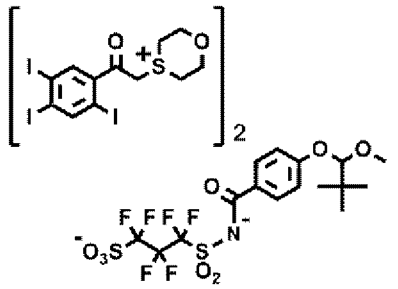

X-12

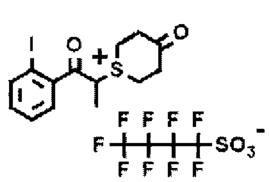

X-13

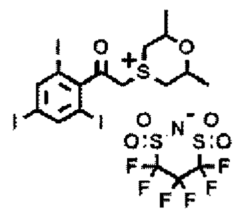

X-14

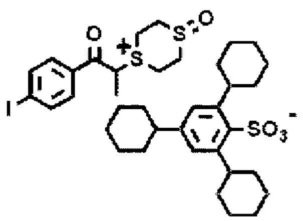

X-15

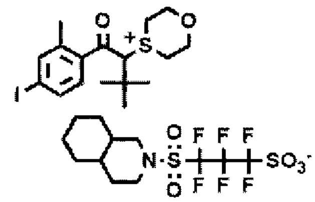

X-16

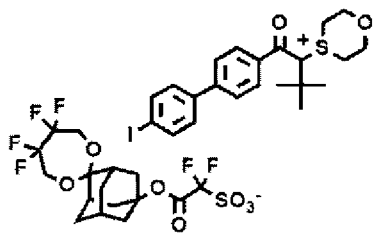

X-17

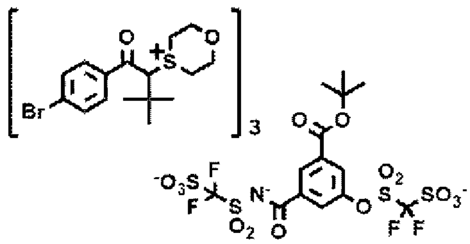

X-18

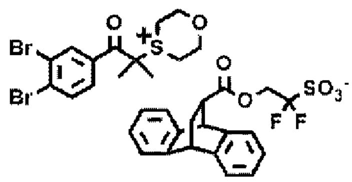

X-19

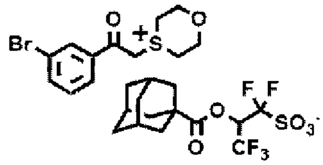

X-20

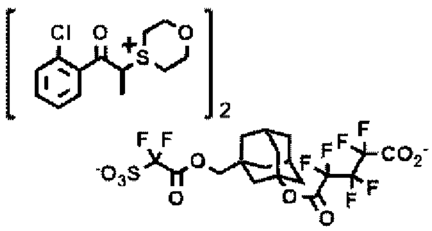

X-21

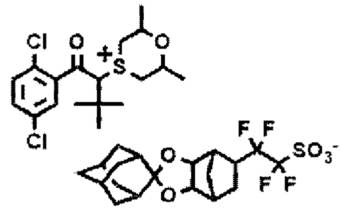

X-22

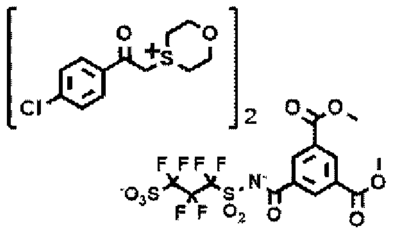

X-23

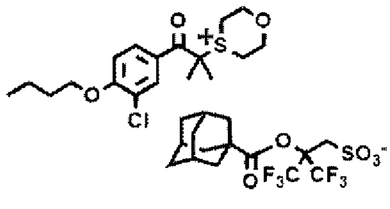

X-24

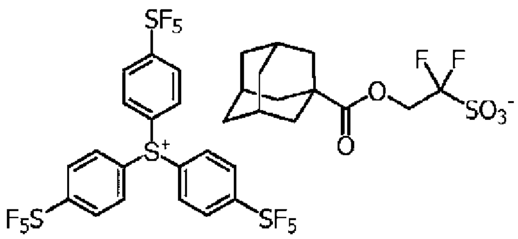

**(I)-1**

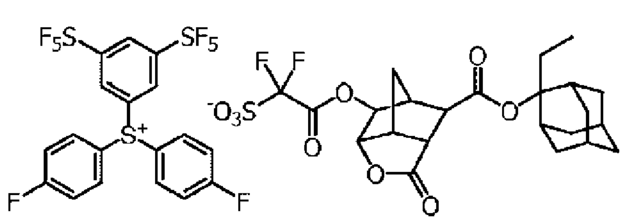

**(I)-2**

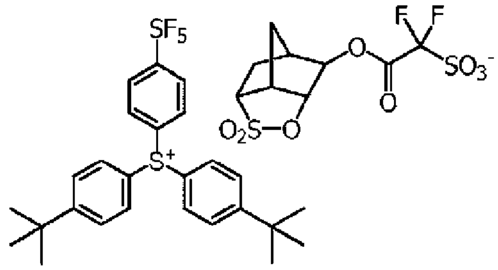

**(I)-3**

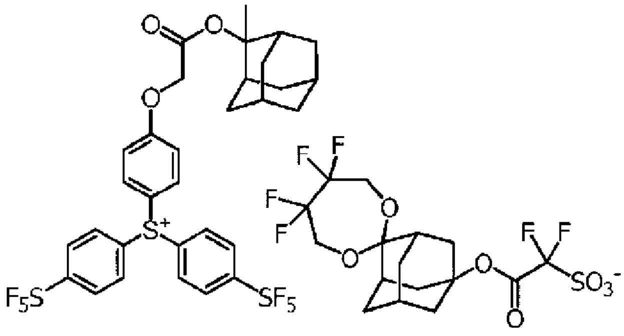

**(I)-4**

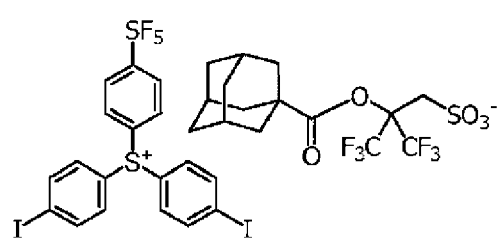

**(I)-5**

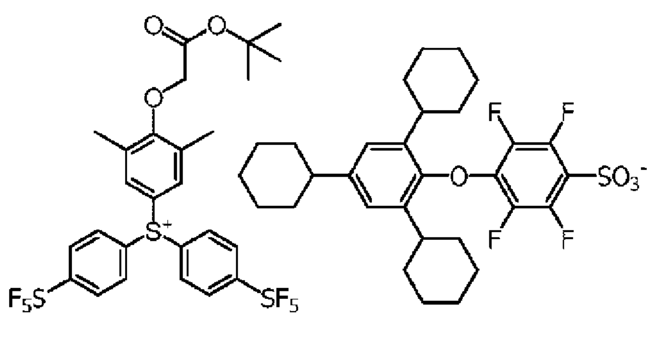

**(I)-6**

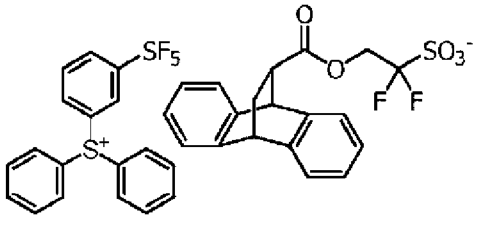

(I)-7

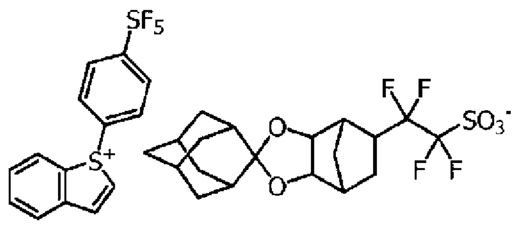

(I)-8

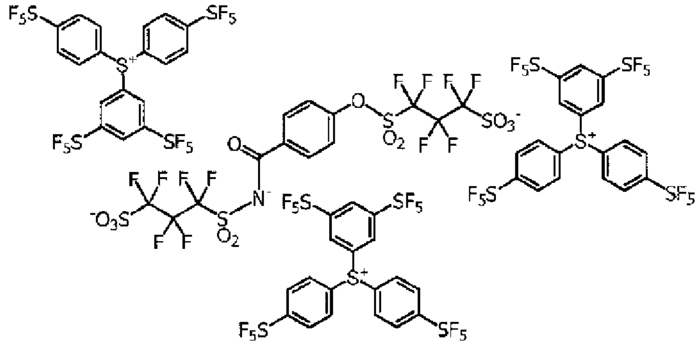

(I)-9

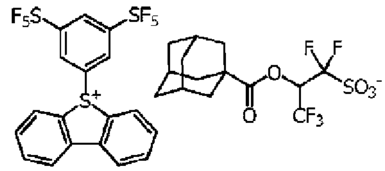

(I)-10

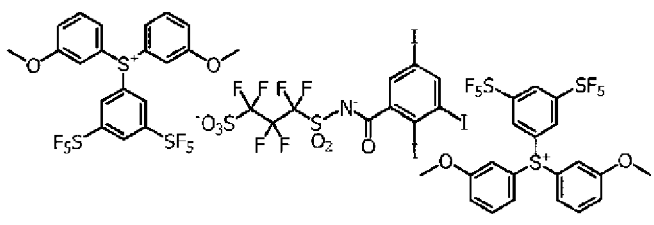

(I)-11

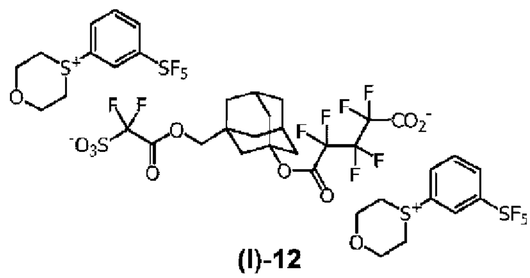

(I)-12

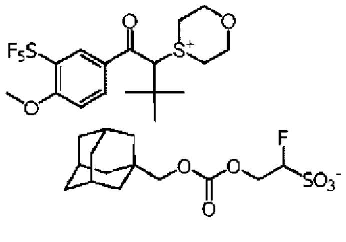

(I)-13

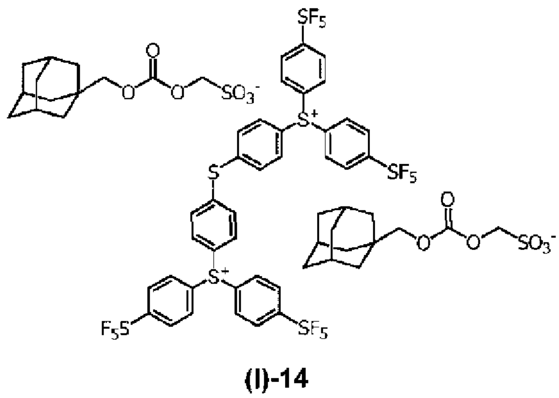

(I)-14

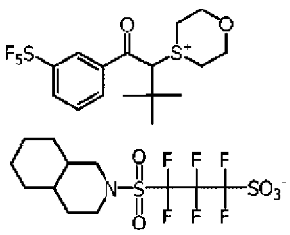

**(I)-15**

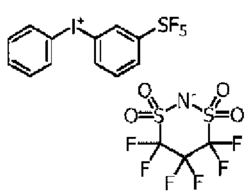

**(I)-16**

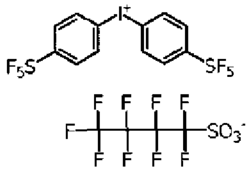

**(I)-17**

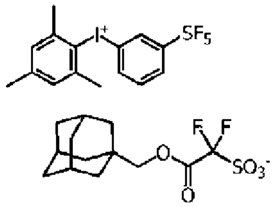

**(I)-18**

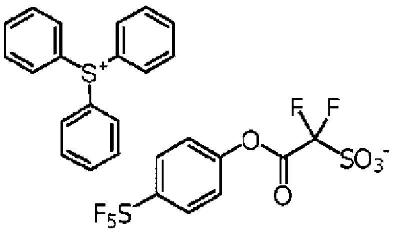

**(I)-19**

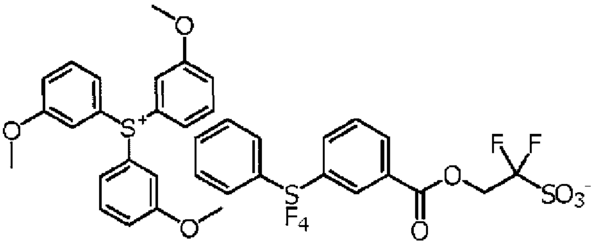

**(I)-20**

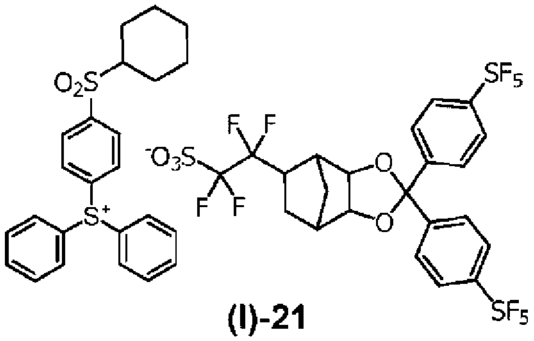

**(I)-21**

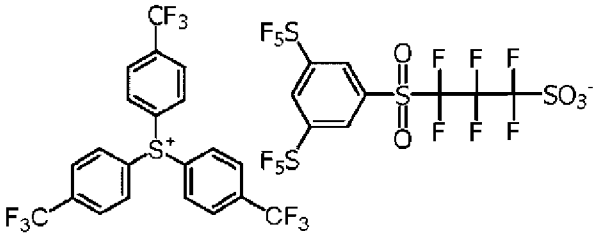

**(I)-22**

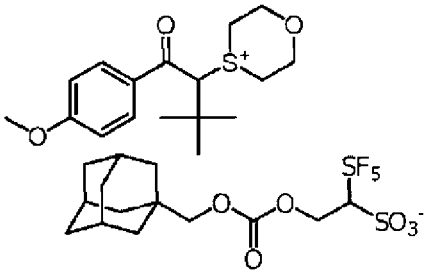

**(I)-23**

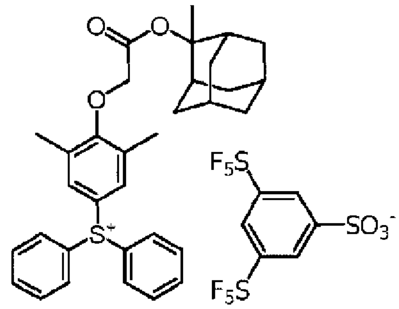

**(I)-24**

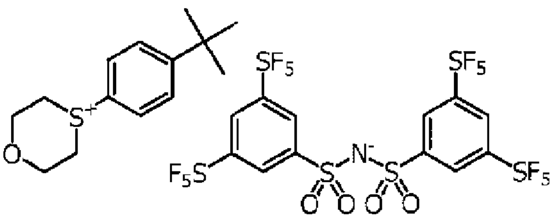

**(I)-25**

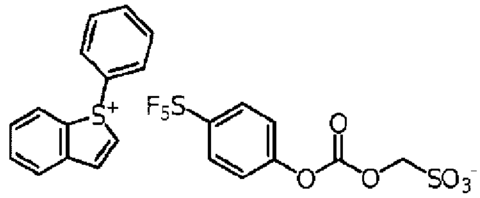

**(I)-26**

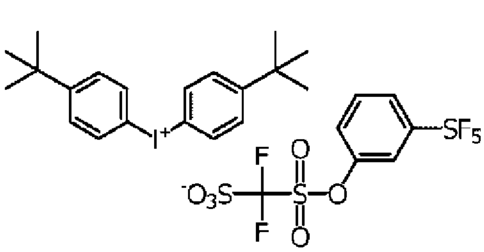

(I)-27

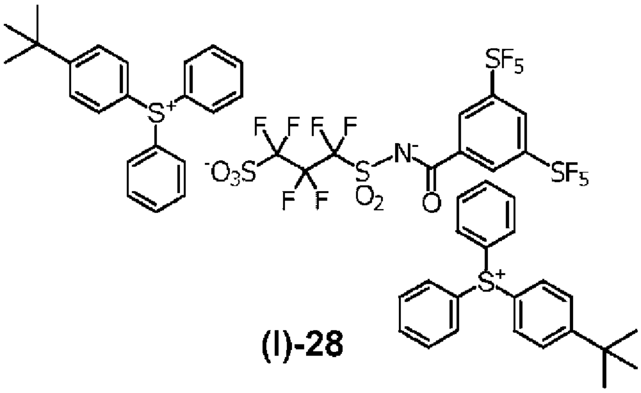

(I)-28

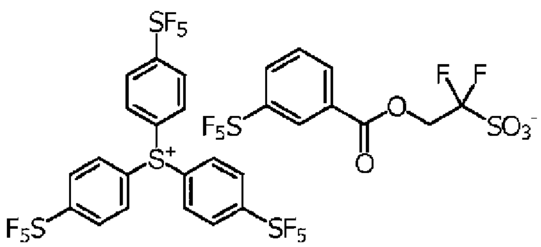

(I)-29

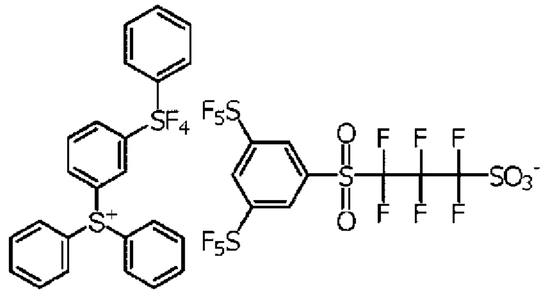

(I)-30

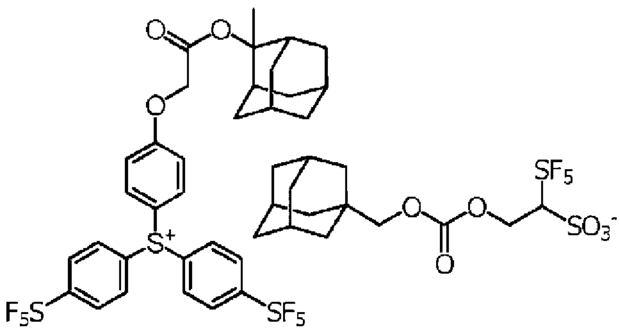

(I)-31

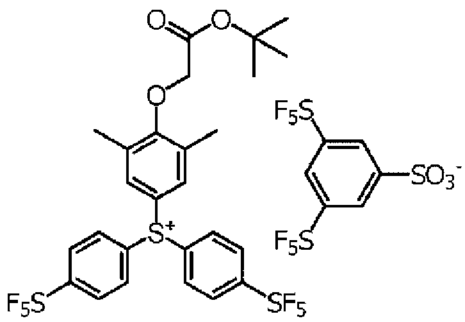

(I)-32

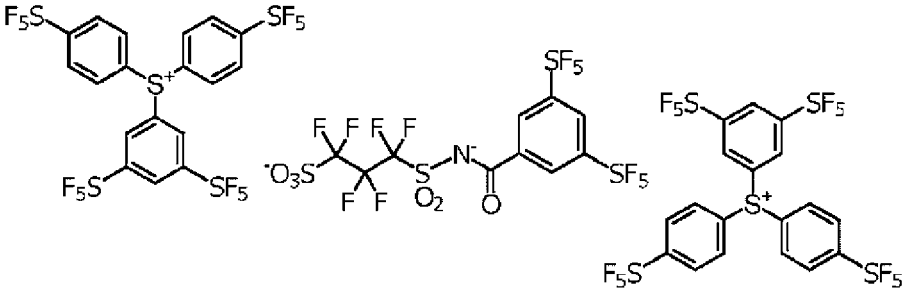

(I)-33

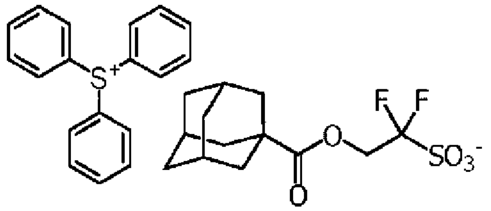

(III)-1

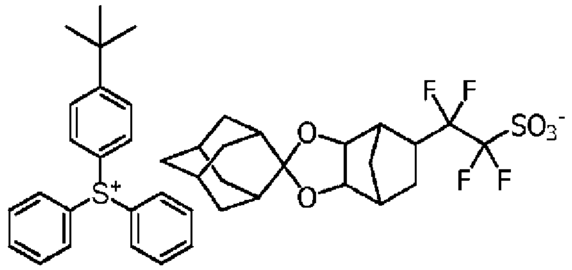

(III)-2

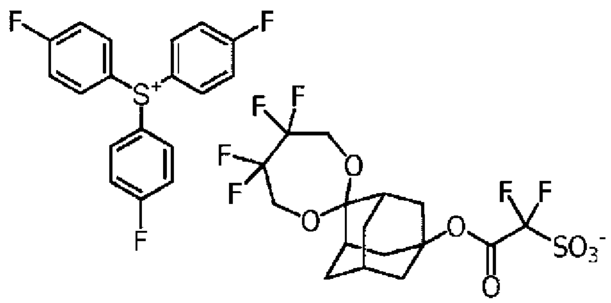

(III)-3

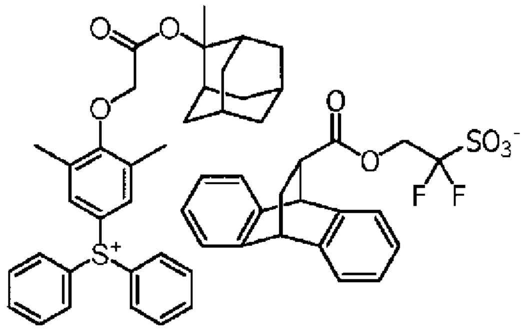

(III)-4

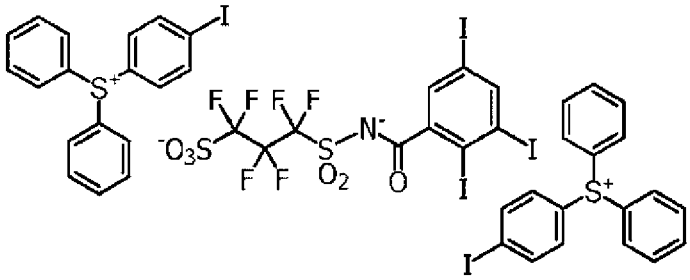

(III)-5

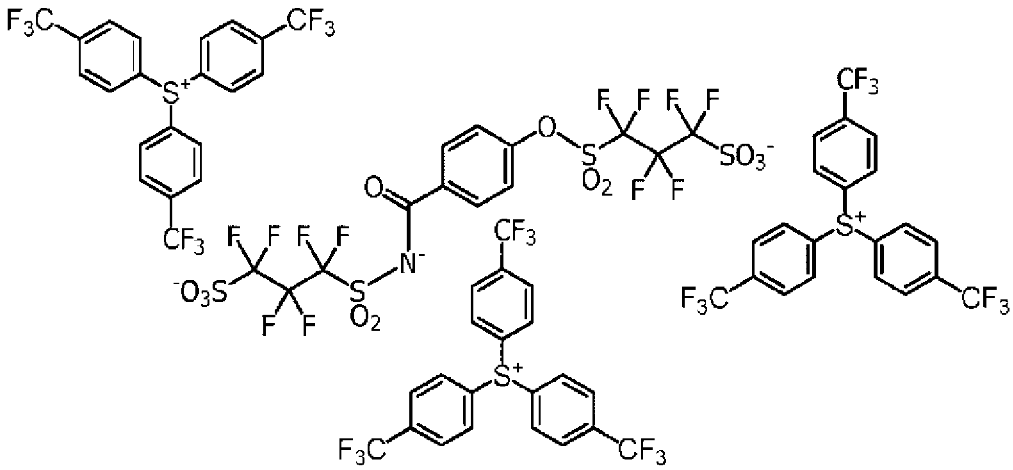

(III)-6

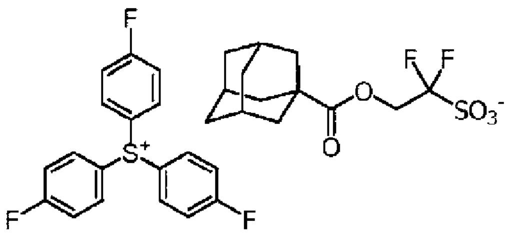

(Z)-1

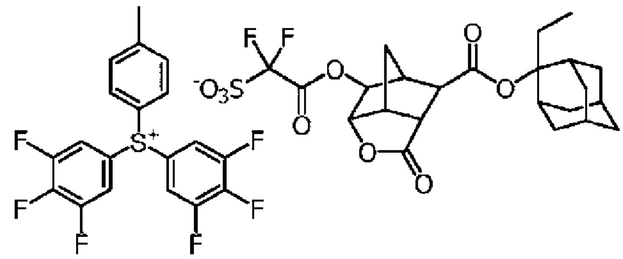

(Z)-2

When the composition of the present invention includes the photoacid generator (B), its content is not particularly limited, but, relative to the total solid content of the composition, preferably 0.5 mass% or more, and more preferably 1.0 mass% or more. The content relative to the total solid content of the composition is preferably 80.0 mass% or less, more preferably 70.0 mass% or less, and still more preferably 60.0 mass% or less.
Such photoacid generators (B) may be used alone or in combination of two or more thereof.

Acid diffusion control agent (C)

The composition of the present invention may include an acid diffusion control agent.
The acid diffusion control agent serves as a quencher that traps the acid generated from the photoacid generator or the like upon exposure and that suppresses the reaction of the acid-decomposable resin, in the unexposed region, caused by an excess of generated acid.
The type of the acid diffusion control agent is not particularly limited, and examples thereof include a basic compound (CA), a low-molecular-weight compound (CB) having a nitrogen atom and having a group that leaves by the action of an acid, and a compound (CC) whose acid diffusion control ability is reduced or lost upon irradiation with an actinic ray or a radiation.
Examples of the compound (CC) include an onium salt compound (CD) that becomes a weak acid relative to the photoacid generator, and a basic compound (CE) whose basicity is reduced or lost upon irradiation with an actinic ray or a radiation.
Specific examples of the basic compound (CA) include, for example, those described in Paragraphs [0132] to [0136] of WO2020/066824A; specific examples of the basic compound (CE) whose basicity is reduced or lost upon irradiation with an actinic ray or a radiation include those described in Paragraphs [0137] to [0155] of WO2020/066824A; specific examples of the low-molecular-weight compound (CB) having a nitrogen atom and having a group that leaves by the action of an acid include those described in Paragraphs [0156] to [0163] of WO2020/066824A; and specific examples of the basic compound (CE) whose basicity is reduced or lost upon irradiation with an actinic ray or a radiation include those described in Paragraph [0164] of WO2020/066824A.
Specific examples of the onium salt compound (CD) that becomes a weak acid relative to the photoacid generator include, for example, those described in Paragraphs [0305] to [0314] of WO2020/158337A.
In addition to those described above, for example, the publicly known compounds disclosed in Paragraphs [0627] to [0664] in US2016/0070167A1, Paragraphs [0095] to [0187] in US2015/0004544A1, Paragraphs [0403] to [0423] in US2016/0237190A1, and Paragraphs [0259] to [0328] in US2016/0274458A1 can be suitably used as acid diffusion control agents.
In a preferred embodiment, the acid diffusion control agent is preferably an onium salt compound (specifically, the onium salt compound (CD)).
The acid diffusion control agent may have a group represented by $-SF_4R$, or may not have a group represented by $-SF_4R$. Here, R represents a hydrogen atom, a halogen atom, or a monovalent substituent. For R, specific examples and the like of the halogen atom and the monovalent substituent are the same as those having been described for the ionic compound.
In the acid diffusion control agent, the cationic moiety may have a group represented by $-SF_4R$, or may not have a group represented by $-SF_4R$. The anionic moiety may have a group represented by $-SF_4R$, or may not have a group represented by $-SF_4R$. The cationic moiety and the anionic moiety may have a group represented by $-SF_4R$.
In the acid diffusion control agent, a compound having a group represented by $-SF_4R$ in at least one of the cationic moiety or the anionic moiety is included in the above-described ionic compound (Y).
The following are non-limiting specific examples of the acid diffusion control agent.

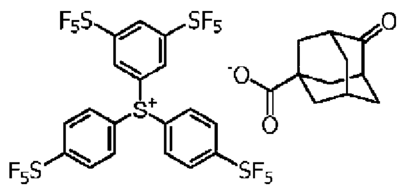

(II)-1

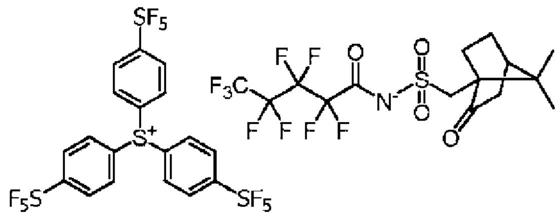

(II)-2

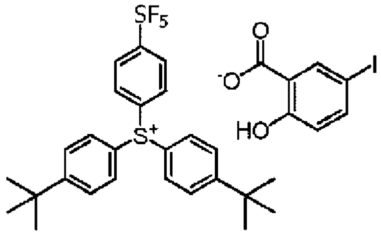

(II)-3

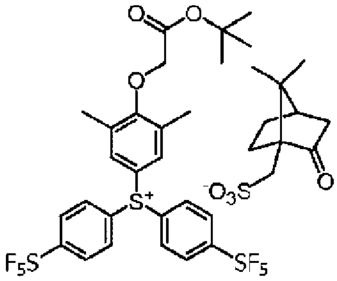

(II)-4

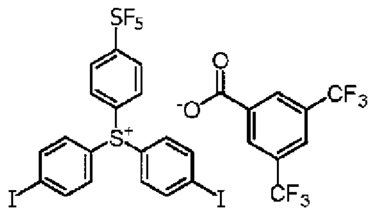

(II)-5

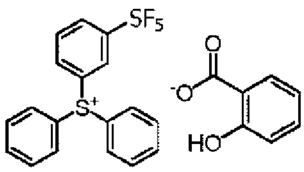

(II)-6

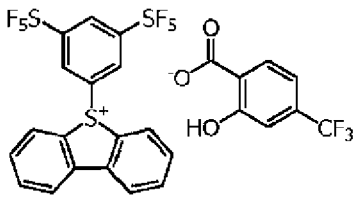

(II)-7

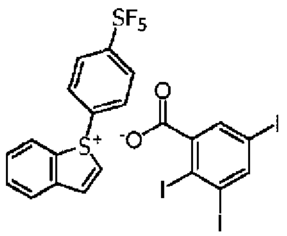

(II)-8

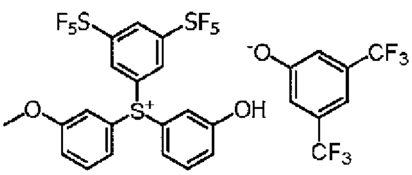

(II)-9

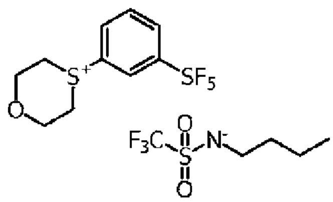

(II)-10

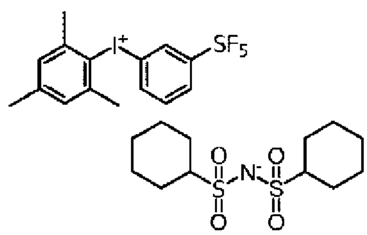

(II)-11

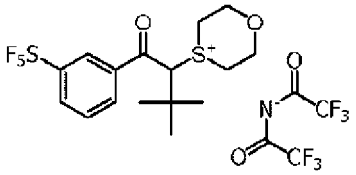

(II)-12

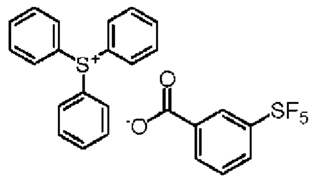

(II)-13

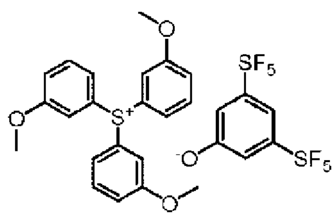

(II)-14

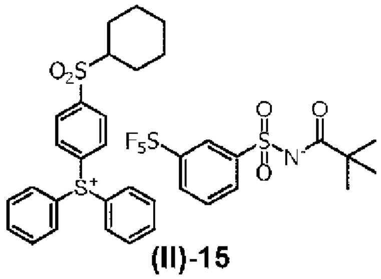

(II)-15

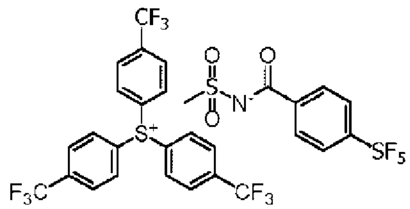

(II)-16

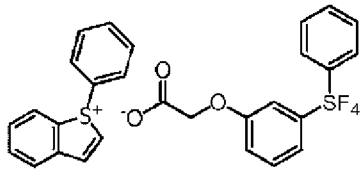

(II)-17

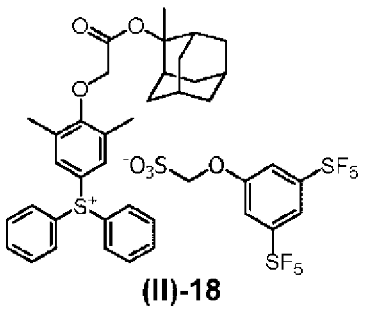

(II)-18

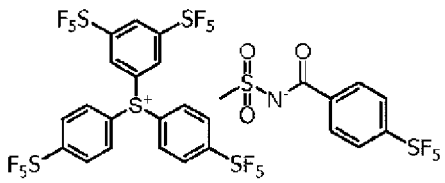

(II)-19

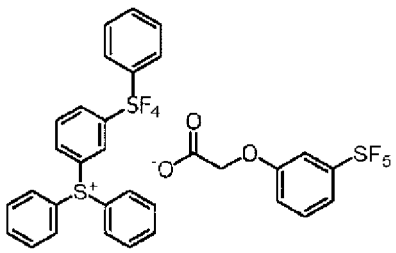

(II)-20

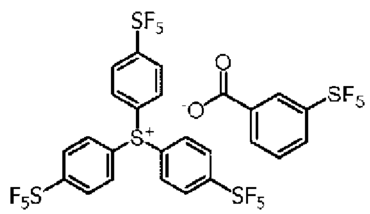

(II)-21

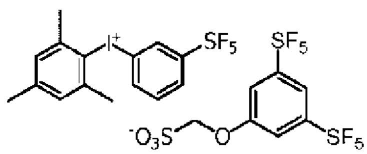

(II)-22

When the composition of the present invention includes an acid diffusion control agent, the content of the acid diffusion control agent (when a plurality of acid diffusion control agents are present, the total content thereof) relative to the total solid content of the composition is preferably 0.1 to 30.0 mass%, and more preferably 1.0 to 25.0 mass%.

In the composition of the present invention, such acid diffusion control agents may be used alone or in combination of two or more thereof.

In a preferred embodiment, the ionic compound (Y) preferably has an anionic moiety represented by any one of the following formulas (N1) to (N3).

As described above, the ionic compound may be an ionic compound having, in the cationic moiety, a group represented by $-SF_4R$, or may be an ionic compound having, in the anionic moiety, a group represented by $-SF_4R$.

In a preferred embodiment, when the ionic compound has, in the anionic moiety, a group represented by $-SF_4R$, the anionic moiety is preferably represented by any one of the following formulas (N1) to (N3). In this case, the anionic moiety represented by any one of the following formulas (N1) to (N3) preferably has a group represented by $-SF_4R$.

$$R^{N2}-C(R^{N1})(R^{N3})-SO_3^- \quad (N1) \qquad R^{N4}-CO_2^- \quad (N2) \qquad R^{N5}-L^{N1}-N^--L^{N2}-R^{N6} \quad (N3)$$

In the formula (N1),

$R^{N1}$ represents a hydrogen atom, a chlorine atom, a bromine atom, an iodine atom, a hydroxy group, a group represented by $-SF_4R$, or an organic group. R represents a hydrogen atom, a halogen atom, or a monovalent substituent.

$R^{N2}$ to $R^{N3}$ each independently represent a hydrogen atom, a fluorine atom, a chlorine atom, a bromine atom, an iodine atom, a hydroxy group, a group represented by $-SF_4R$, or an organic group. R represents a hydrogen atom, a halogen atom, or a monovalent substituent.

$R^{N1}$ to $R^{N3}$ may be bonded together to form a ring.

In the formula (N2),

$R^{N4}$ represents an organic group.

In the formula (N3),

$L^{N1}$ and $L^{N2}$ each independently represent $-SO_2-$, -CO-, or an alkylene group.

$R^{N5}$ and $R^{N6}$ each independently represent an organic group. $R^{N5}$ and $R^{N6}$ may be bonded together to form a ring.

R represents a hydrogen atom, a halogen atom, or a monovalent substituent. For R, specific examples and the like of the halogen atom and the monovalent substituent are the same as those having been described for the ionic compound.

The organic group of $R^{N1}$ is not particularly limited as long as the organic group has one or more carbon atoms. The organic group may have or may not have a heteroatom (such as an oxygen atom, a sulfur atom, and/or a nitrogen atom). The organic group may have a group represented by $-SF_4R$, or may not have a group represented by - $SF_4R$.

The organic group may be, for example, an organic group having 1 to 30 carbon atoms.

The organic group is also preferably a group represented by a formula (Y1) below.

$$-L^{P1}-W^{p} \qquad (Y1)$$

In the formula (Y1),

$L^{p1}$ represents a single bond or a divalent linking group.

$W^p$ represents an organic group.

For $L^{p1}$, the divalent linking group is, for example, -O-CO-O-, -COO-, -CONH-, -CO-, -O-, -S-, -SO-, $-SO_2-$, an alkylene group (preferably having 1 to 6 carbon atoms), a cycloalkylene group (that may be monocyclic or may be polycyclic and preferably has 3 to 15 carbon atoms), an alkenylene group (preferably having 2 to 6 carbon atoms), a lactone ring group, a sultone ring group, and a divalent linking group that is a combination of a plurality of the foregoing. In particular, the divalent linking group is preferably -O-CO-O-, -COO-, -CONH-, -CO-, -O-, $-SO_2-$, -O-CO-O-alkylene group-, -COO-alkylene group-, or -CONH-alkylene group-, and more preferably -O-CO-O-, -O-COO-alkylene group-, -COO-, -CONH-, $-SO_2-$, or -COO-alkylene group-.

The lactone ring group and the sultone ring group are, for example, preferably a group provided by removing two hydrogen atoms from a ring-member atom constituting the lactone structure or the sultone structure in any one of the above-described structures represented by the formulas (LC1-1) to (LC1-21) and the above-described structures represented by the formulas (SL 1 -1) to (SL 1-3).

The alkylene group, the cycloalkylene group, and the alkenylene group may have a substituent.

$W^p$ represents an organic group.

The organic group is not particularly limited as long as it has 1 or more carbon atoms, and may be a linear group (for example, a linear alkyl group), a branched group (for example, a branched alkyl group such as a t-butyl group), or a cyclic group. The organic group may have or may not have a substituent. The organic group may have or may not have a heteroatom (such as an oxygen atom, a sulfur atom, and/or a nitrogen atom).

The organic group may have a group represented by $-SF_4R$, or may not have a group represented by $-SF_4R$.

The organic group may be, for example, an organic group having 1 to 30 carbon atoms.

The cyclic organic group may be, for example, an alicyclic group, an aryl group, or a heterocyclic group.

The alicyclic group may be monocyclic or may be polycyclic. Examples of the monocyclic alicyclic group include monocyclic cycloalkyl groups such as a cyclopentyl group, a cyclohexyl group, and a cyclooctyl group. Examples of the polycyclic alicyclic group include polycyclic cycloalkyl groups such as a norbornyl group, a tricyclodecanyl group, a tetracyclodecanyl group, a tetracyclododecanyl group, and an adamantyl group. In particular, preferred are alicyclic groups having a bulky structure having 7 or more carbon atoms such as a norbornyl group, a tricyclodecanyl group, a tetracyclodecanyl group, a tetracyclododecanyl group, and an adamantyl group.

The aryl group may be monocyclic or polycyclic. Examples of the aryl group include a phenyl group, a naphthyl group, a phenanthryl group, and an anthryl group.

The heterocyclic group may be monocyclic or polycyclic. In particular, in the case of a polycyclic heterocyclic group, diffusion of acid can be further suppressed. The heterocyclic group may have aromaticity or may not have

aromaticity. Examples of the heterocycle having aromaticity include a furan ring, a thiophene ring, a benzofuran ring, a benzothiophene ring, a dibenzofuran ring, a dibenzothiophene ring, and a pyridine ring. Examples of the heterocycle not having aromaticity include a tetrahydropyran ring, a lactone ring, a sultone ring, and a decahydroisoquinoline ring. In the heterocyclic group, the heterocycle is preferably a furan ring, a thiophene ring, a pyridine ring, or a decahydroisoquinoline ring.

The cyclic organic group may have a substituent. The substituent may be, for example, an alkyl group (that may be linear or branched and preferably has 1 to 12 carbon atoms), a cycloalkyl group (that may be have a monocycle, a polycycle, or a spiro ring and preferably has 3 to 20 carbon atoms), an aryl group (preferably having 6 to 14 carbon atoms), a hydroxy group, an alkoxy group, an ester group, an amide group, a urethane group, a ureido group, a group represented by $-SF_4R$ (where R represents a hydrogen atom, a halogen atom, or a monovalent substituent), a thioether group, a sulfonamide group, or a sulfonic acid ester group. Note that a carbon constituting the cyclic organic group (carbon contributing to formation of the ring) may be a carbonyl carbon.

For $R^{N2}$ to $R^{N3}$, the organic group is not particularly limited as long as it has one or more carbon atoms. The organic group may have or may not have a substituent. The organic group may have or may not have a heteroatom (such as an oxygen atom, a sulfur atom, and/or a nitrogen atom).

The organic group is not particularly limited, but is preferably an organic group having 1 to 30 carbon atoms. Of these, preferred is an alkyl group having 1 to 10 carbon atoms. The organic group may have a substituent.

When the ionic compound (Y) has, in the anionic moiety, a group represented by $-SF_4R$, in the formula (N1), at least one of $R^{N1}$, $R^{N2}$, or $R^{N3}$ represents a group represented by $-SF_4R$ or an organic group having a group represented by $-SF_4R$.

In the formula (N2), $R^{N4}$ represents an organic group.

For the organic group of $R^{N4}$, the organic group is not particularly limited as long as it has one or more carbon atoms. It may be a linear group (for example, a linear alkyl group), a branched group (for example, a branched alkyl group such as a t-butyl group), or a cyclic group. The organic group may have or may not have a substituent. The organic group may have or may not have a heteroatom (such as an oxygen atom, a sulfur atom, and/or a nitrogen atom).

The organic group may be, for example, an organic group having 1 to 30 carbon atoms.

Examples of the organic group include an alkyl group. The alkyl group is not particularly limited, but may be linear or may be branched, and may be, for example, an alkyl group having 1 to 10 carbon atoms. The alkyl group may have a substituent.

In a preferred embodiment, the substituent may be, for example, an aryloxy group or a group represented by $-SF_4R$ (where R represents a hydrogen atom, a halogen atom, or a monovalent substituent). Such substituents may further have a substituent.

The cyclic organic group may be, for example, an alicyclic group, an aryl group, or a heterocyclic group.

The alicyclic group may be monocyclic or may be polycyclic. Examples of the monocyclic alicyclic group include monocyclic cycloalkyl groups such as a cyclopentyl group, a cyclohexyl group, and a cyclooctyl group. Examples of the polycyclic alicyclic group include polycyclic cycloalkyl groups such as a norbornyl group, a tricyclodecanyl group, a tetracyclodecanyl group, a tetracyclododecanyl group, and an adamantyl group. In particular, preferred are alicyclic groups having a bulky structure having 7 or more carbon atoms such as a norbornyl group, a tricyclodecanyl group, a tetracyclodecanyl group, a tetracyclododecanyl group, and an adamantyl group.

The aryl group may be monocyclic or polycyclic. Examples of the aryl group include a phenyl group, a naphthyl group, a phenanthryl group, and an anthryl group.

The heterocyclic group may be monocyclic or polycyclic. In particular, in the case of a polycyclic heterocyclic group, diffusion of acid can be further suppressed. The heterocyclic group may have aromaticity or may not have aromaticity. Examples of the heterocycle having aromaticity include a furan ring, a thiophene ring, a benzofuran ring, a benzothiophene ring, a dibenzofuran ring, a dibenzothiophene ring, and a pyridine ring. Examples of the heterocycle not having aromaticity include a tetrahydropyran ring, a lactone ring, a sultone ring, and a decahydroisoquinoline ring. In the heterocyclic group, the heterocycle is preferably a furan ring, a thiophene ring, a pyridine ring, or a decahydroisoquinoline ring.

The cyclic organic group may have a substituent. The substituent may be, for example, an alkyl group (that may be linear or branched and preferably has 1 to 12 carbon atoms), a cycloalkyl group (that may have a monocycle, a polycycle, or a spiro ring and preferably has 3 to 20 carbon atoms), an aryl group (preferably having 6 to 14 carbon atoms), a hydroxy group, an alkoxy group, an ester group, an amide group, a urethane group, a ureido group, a halogen atom, a group represented by $-SF_4R$ (where R represents a hydrogen atom, a halogen atom, or a monovalent substituent), a thioether group, a sulfonamide group, or a sulfonic acid ester group. Note that a carbon constituting the cyclic organic group (carbon contributing to formation of the ring) may be a carbonyl carbon.

When the ionic compound (Y) has, in the anionic moiety, a group represented by $-SF_4R$, in the formula (N2), $R^{N4}$ represents an organic group having a group represented by $-SF_4R$.

In the formula (N3), $L^{N1}$ and $L^{N2}$ each independently represent $-SO_2-$, -CO-, or an alkylene group.

$R^{N5}$ and $R^{N6}$ each independently represent an organic group. $R^{N5}$ and $R^{N6}$ may be bonded together to form a ring.
For $L^{N1}$ and $L^{N2}$, the alkylene group is not particularly limited, but is preferably an alkylene group having 1 to 6 carbon atoms.
$L^{N1}$ preferably represents $-SO_2-$ or -CO-, and $L^{N2}$ preferably represents $-SO_2-$ or -CO-.
For the organic groups of $R^{N5}$ and $R^{N6}$, such an organic group is not particularly limited as long as it has one or more carbon atoms. It may be a linear group (for example, a linear alkyl group), a branched group (for example, a branched alkyl group such as a t-butyl group), or a cyclic group. The organic group may have or may not have a substituent. The organic group may have or may not have a heteroatom (such as an oxygen atom, a sulfur atom, and/or a nitrogen atom).
The organic group may be, for example, an organic group having 1 to 30 carbon atoms.
The organic group may be, for example, an alkyl group. The alkyl group is not particularly limited, but may be linear or may be branched, and may be, for example, an alkyl group having 1 to 10 carbon atoms. The alkyl group may have a substituent.
In a preferred embodiment, the substituent may be, for example, a halogen atom or a group represented by $-SF_4R$ (where R represents a hydrogen atom, a halogen atom, or a monovalent substituent). Such substituents may further have a substituent.
The cyclic organic group may be, for example, an alicyclic group, an aryl group, or a heterocyclic group.
The alicyclic group may be monocyclic or may be polycyclic. Examples of the monocyclic alicyclic group include monocyclic cycloalkyl groups such as a cyclopentyl group, a cyclohexyl group, and a cyclooctyl group. Examples of the polycyclic alicyclic group include polycyclic cycloalkyl groups such as a norbornyl group, a tricyclodecanyl group, a tetracyclodecanyl group, a tetracyclododecanyl group, and an adamantyl group. In particular, preferred are alicyclic groups having a bulky structure having 7 or more carbon atoms such as a norbornyl group, a tricyclodecanyl group, a tetracyclodecanyl group, a tetracyclododecanyl group, and an adamantyl group.
The aryl group may be monocyclic or polycyclic. Examples of the aryl group include a phenyl group, a naphthyl group, a phenanthryl group, and an anthryl group.
The heterocyclic group may be monocyclic or polycyclic. In particular, in the case of a polycyclic heterocyclic group, diffusion of acid can be further suppressed. The heterocyclic group may have aromaticity or may not have aromaticity. Examples of the heterocycle having aromaticity include a furan ring, a thiophene ring, a benzofuran ring, a benzothiophene ring, a dibenzofuran ring, a dibenzothiophene ring, and a pyridine ring. Examples of the heterocycle not having aromaticity include a tetrahydropyran ring, a lactone ring, a sultone ring, and a decahydroisoquinoline ring. In the heterocyclic group, the heterocycle is preferably a furan ring, a thiophene ring, a pyridine ring, or a decahydroisoquinoline ring.
The cyclic organic group may have a substituent. The substituent may be, for example, an alkyl group (that may be linear or branched and preferably has 1 to 12 carbon atoms), a cycloalkyl group (that may have a monocycle, a polycycle, or a spiro ring, and preferably has 3 to 20 carbon atoms), an aryl group (preferably having 6 to 14 carbon atoms), a hydroxy group, an alkoxy group, an ester group, an amide group, a urethane group, a ureido group, a halogen atom, a group represented by $-SF_4R$ (where R represents a hydrogen atom, a halogen atom, or a monovalent substituent), a thioether group, a sulfonamide group, or a sulfonic acid ester group. Note that a carbon constituting the cyclic organic group (carbon contributing to formation of the ring) may be a carbonyl carbon.
When the ionic compound (Y) has, in the anionic moiety, a group represented by $-SF_4R$, in the formula (N3), at least one of $R^{N5}$ or $R^{N6}$ represents an organic group having a group represented by $-SF_4R$.

Hydrophobic resin (D)

The composition of the present invention may further include a hydrophobic resin different from the resin (A).
The hydrophobic resin is preferably designed so as to be localized in the surface of a resist film; however, unlike surfactants, the hydrophobic resin does not necessarily need to have intramolecularly a hydrophilic group, and does not necessarily contribute to homogeneous mixing of a polar substance and a nonpolar substance.
Advantages due to addition of the hydrophobic resin may be control of static and dynamic contact angles (for water) at the surface of the resist film, and suppression of outgassing.
The hydrophobic resin, from the viewpoint of localization in the surface layer of the film, preferably has one or more species, more preferably two or more species, selected from the group consisting of a fluorine atom, a silicon atom, and a $CH_3$ moiety included in the side chain moiety of the resin. The hydrophobic resin preferably has a hydrocarbon group having 5 or more carbon atoms. The resin may have such a group in the main chain or, as a substituent, in a side chain.
Examples of the hydrophobic resin include the compounds described in Paragraphs [0275] to [0279] in WO2020/004306A.
When the composition of the present invention includes a hydrophobic resin, the content of the hydrophobic

resin relative to the total solid content of the resist composition is preferably 0.01 to 20.0 mass%, and more preferably 0.1 to 15.0 mass%.

Surfactant (E)

The composition of the present invention may include a surfactant. In the case of including a surfactant, a pattern having higher adhesiveness and a less number of development defects can be formed.
The surfactant is preferably a fluorine-based and/or silicone-based surfactant.
Examples of the fluorine-based and/or silicone-based surfactant include the surfactants disclosed in Paragraphs [0218] and [0219] of WO2018/193954A.
Such surfactants may be used alone or in combination of two or more thereof.
When the composition of the present invention includes a surfactant, the surfactant content relative to the total solid content of the resist composition is preferably 0.0001 to 2.0 mass%, more preferably 0.0005 to 1.0 mass%, and still more preferably 0.1 to 1.0 mass%.

Solvent (F)

The composition of the present invention preferably includes a solvent.
The solvent preferably includes at least one of a component (M1) or (M2), which is (M1) a propylene glycol monoalkyl ether carboxylate or (M2) at least one selected from the group consisting of a propylene glycol monoalkyl ether, a lactate, an acetate, an alkoxypropionate, a chain ketone, a cyclic ketone, a lactone, and an alkylene carbonate. Note that the solvent may further include a component other than the components (M1) and (M2).
A combination of the above-described solvent and the above-described resin is preferred from the viewpoint of improving the coatability of the resist composition and reducing the number of pattern development defects. The above-described solvent is well-balanced in terms of solubility of the above-described resin, boiling point, and viscosity, to thereby suppress, for example, unevenness of the film thickness of the resist film and generation of deposit during spin-coating.
Details of the component (M1) and the component (M2) are described in Paragraphs [0218] to [0226] in WO2020/004306A, and these contents are incorporated herein by reference.
When the solvent further includes a component other than the components (M1) and (M2), the content of the component other than the components (M1) and (M2) relative to the total amount of the solvent is preferably 5 to 30 mass%.
The content of the solvent in the composition of the present invention is determined such that the solid-content concentration is preferably 0.5 to 30 mass%, and more preferably 1 to 20 mass%. This further improves the coatability of the composition.
Note that the solid content means all the components other than the solvent, and, as described above, means components that form the actinic ray-sensitive or radiation-sensitive film.
The solid-content concentration is a mass percentage of the mass of other components excluding the solvent relative to the total mass of the composition of the present invention.
The "total solid content" refers to the total mass of the components excluding the solvent from all the components of the composition of the present invention. As described above, the "solid content" is components excluding the solvent, and may be a solid or a liquid at 25°C, for example.

Other additives

The composition of the present invention may further include a dissolution-inhibiting compound, a dye, a plasticizer, a photosensitizer, a light absorbent, and/or a compound that promotes solubility in a developer (for example, a phenol compound having a molecular weight of 1000 or less, or an alicyclic or aliphatic compound including a carboxyl group).
The "dissolution-inhibiting compound" is a compound that is decomposed by the action of an acid to undergo a decrease in the degree of solubility in organic-based developers, and has a molecular weight of 3000 or less.
The composition in this Specification is suitably used as a photosensitive composition for EUV exposure.
The EUV light has a wavelength of 13.5 nm, which is a shorter wavelength than in the ArF (having a wavelength of 193 nm) light and the like, and hence provides, upon exposure at the same sensitivity, a smaller number of incident photons. Thus, "photon shot noise", which is random variations in the number of photons, exerts a strong effect, which leads to degradation of LER and bridge defects. In order to reduce the photon shot noise, a method of increasing the exposure dose to increase the number of incident photons may be employed; however, there is a tradeoff between this method and the demand for an increase in the sensitivity.
In a preferred embodiment, the composition of the present invention does not have a perfluoroalkyl group.

The present invention also relates to the following compound:

A sulfonium salt compound having, in at least one of a cationic moiety or an anionic moiety, a group represented by $-SF_4R$, where R represents a hydrogen atom, a halogen atom, or a monovalent substituent.

For R, specific examples and the like of the halogen atom and the monovalent substituent are the same as those having been described for the ionic compound.

The sulfonium salt compound is one of preferred embodiments of the ionic compound (Y).

Examples of the cationic moiety include organic cations, specifically, sulfonium cations. The sulfonium cations are the same as the cation represented by the above-described formula (ZaI). The same applies to specific examples and preferred examples.

The sulfonium salt compound is preferably a compound having, in the cationic moiety, a group represented by $-SF_4R$.

The anionic moiety is not particularly limited, but may be the same as the anion $X^-$ described above in the photoacid generator in the composition of the present invention. The same applies to specific examples and preferred examples.

Other examples of the anionic moiety include anions represented by any one of the following formulas (N1) to (N3).

The sulfonium salt compound is preferably a compound having, in the anionic moiety, a group represented by $-SF_4R$.

The group represented by $-SF_4R$ is preferably $-SF_5$.

Examples of the sulfonium salt also include compounds corresponding to the sulfonium salt in the compound (I) and the compound (II) described in the photoacid generator in the composition of the present invention.

Specific examples of the sulfonium salt include, but are not particularly limited to, those corresponding to the sulfonium salt among those described above in the photoacid generator and the acid diffusion control agent in the composition of the present invention.

The present invention also relates to the following compound:

An iodonium salt compound having, in at least one of a cationic moiety or an anionic moiety, a group represented by $-SF_4R$, wherein the anionic moiety is represented by any one of the following formulas (N1) to (N3), where R represents a hydrogen atom, a halogen atom, or a monovalent substituent,

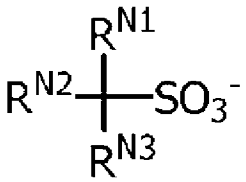
(N1)
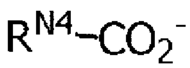
(N2)
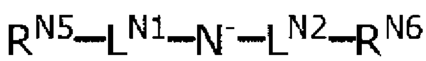
(N3)

in the formula (N1),

$R^{N1}$ represents a hydrogen atom, a chlorine atom, a bromine atom, an iodine atom, a hydroxy group, a group represented by $-SF_4R$, or an organic group, where R represents a hydrogen atom, a halogen atom, or a monovalent substituent,
$R^{N2}$ to $R^{N3}$ each independently represent a hydrogen atom, a fluorine atom, a chlorine atom, a bromine atom, an iodine atom, a hydroxy group, a group represented by $-SF_4R$, or an organic group, where R represents a hydrogen atom, a halogen atom, or a monovalent substituent,
$R^{N1}$ to $R^{N3}$ may be bonded together to form a ring,
in the formula (N2),
$R^{N4}$ represents an organic group,
in the formula (N3),
$L^{N1}$ and $L^{N2}$ each independently represent $-SO_2-$, $-CO-$, or an alkylene group,
$R^{N5}$ and $R^{N6}$ each independently represent an organic group, and $R^{N5}$ and $R^{N6}$ may be bonded together to form a ring.

For R, specific examples and the like of the halogen atom and the monovalent substituent are the same as those having been described for the ionic compound.

The iodonium salt compound is one of preferred embodiments of the ionic compound (Y).

For $R^{N1}$ in the formula (N1), the organic group may be the same as the above-described organic group serving as $R^{N1}$ in the formula (N1) in the composition of the present invention, and specific examples and preferred ranges thereof are also the same.

For $R^{N2}$ and $R^{N3}$ in the formula (N2), the organic groups may be the same as the above-described organic groups serving as $R^{N2}$ and $R^{N3}$ in the formula (N2) in the composition of the present invention, and specific examples and preferred ranges thereof are also the same.

For $L^{N1}$ and $L^{N2}$ in the formula (N3), the alkylene groups may be the same as the above-described alkylene groups serving as $L^{N1}$ and $L^{N2}$ in the formula (N3) in the composition of the present invention, and specific examples and preferred ranges thereof are also the same.

For $R^{N5}$ and $R^{N6}$ in the formula (N3), the organic groups may be the same as the above-described organic groups serving as $R^{N5}$ and $R^{N6}$ in the formula (N3) in the composition of the present invention, and specific examples and preferred ranges thereof are also the same.

Examples of the cationic moiety include organic cations, specifically, sulfonium cations. The sulfonium cations are the same as the above-described cation represented by the formula (ZaI). The same applies to specific examples and preferred examples.

The iodonium salt compound is preferably a compound having, in the cationic moiety, a group represented by $-SF_4R$.

The iodonium salt compound is preferably a compound having, in the anionic moiety, a group represented by $-SF_4R$.

The group represented by $-SF_4R$ is preferably $-SF_5$.

Specific examples of the iodonium salt include, but are not particularly limited to, those corresponding to the iodonium salt among those described above in the photoacid generator and the acid diffusion control agent in the composition of the present invention.

The present invention also relates to the following compound:

An iodonium salt compound having, in at least one of a cationic moiety or an anionic moiety, a group represented by $-SF_4R$,
where R represents a hydrogen atom, a halogen atom, or a monovalent substituent.

For R, specific examples and the like of the halogen atom and the monovalent substituent are the same as those having been described for the ionic compound.

The iodonium salt compound is one of preferred embodiments of the ionic compound (Y).

Examples of the cationic moiety include organic cations, specifically, sulfonium cations. The sulfonium cations are the same as the above-described cation represented by the formula (ZaI). The same applies to specific examples and preferred examples.

The iodonium salt compound is preferably a compound having, in the cationic moiety, a group represented by $-SF_4R$.

The anionic moiety is not particularly limited, but examples thereof include the same as the above-described anion $X^-$ described in the photoacid generator in the composition of the present invention. The same applies to specific examples and preferred examples.

The iodonium salt compound is preferably a compound having, in the anionic moiety, a group represented by $-SF_4R$.

Examples of the iodonium salt also include compounds corresponding to the iodonium salt in the compound (I) and the compound (II) described in the photoacid generator in the composition of the present invention.

Specific examples of the iodonium salt include, but are not particularly limited to, those corresponding to the iodonium salt among those described above in the photoacid generator and the acid diffusion control agent in the composition of the present invention.

Actinic ray-sensitive or radiation-sensitive film and pattern forming method

The procedures of the pattern forming method using the composition are not particularly limited, but preferably have the following steps.

Step 1: a step of using the actinic ray-sensitive or radiation-sensitive resin composition to form an actinic ray-sensitive or radiation-sensitive film on a substrate
Step 2: a step of exposing the actinic ray-sensitive or radiation-sensitive film
Step 3: a step of developing the exposed actinic ray-sensitive or radiation-sensitive film using a developer

Hereinafter, procedures of the steps will be individually described in detail.

Step 1: actinic ray-sensitive or radiation-sensitive film formation step

The step 1 is a step of using the actinic ray-sensitive or radiation-sensitive resin composition to form an actinic ray-sensitive or radiation-sensitive film on a substrate.

Examples of the method of using the actinic ray-sensitive or radiation-sensitive resin composition to form an actinic ray-sensitive or radiation-sensitive film (preferably, a resist film) on a substrate include a method of applying the composition of the present invention onto a substrate.

Note that the composition of the present invention is preferably filtered through a filter before application as needed. The filter preferably has a pore size of 0.1 μm or less, more preferably 0.05 μm or less, and still more preferably 0.03 μm or less. In the filter filtration, filters having such pore sizes are also preferably used in combination. The filter is preferably formed of polytetrafluoroethylene, polyethylene, or nylon.

The composition of the present invention can be applied onto a substrate (such as a silicon, silicon dioxide-coated substrate) used in the production of an integrated circuit element, by an appropriate application process using a spinner, a coater, or the like. The application process is preferably spin-coating using a spinner. The spin-coating using a spinner is preferably performed at a rotation rate of 1000 to 3000 rpm.

After application of the composition of the present invention, the substrate may be dried to form an actinic ray-sensitive or radiation-sensitive film. Note that, as needed, as underlayers of the actinic ray-sensitive or radiation-sensitive film, various underlying films (an inorganic film, an organic film, or an antireflection film) may be formed.

The drying process may be, for example, a process of performing heating to achieve drying. The heating can be performed using means included in an ordinary exposure device and/or an ordinary development device, or may alternatively be performed using a hot plate, for example. The heating temperature is preferably 80 to 150°C, more preferably 80 to 140°C, and still more preferably 80 to 130°C. The heating time is preferably 30 to 1000 seconds, more preferably 60 to 800 seconds, and still more preferably 60 to 600 seconds.

The film thickness of the actinic ray-sensitive or radiation-sensitive film is not particularly limited, but is, from the viewpoint of enabling formation of more precise fine patterns, preferably 10 to 120 nm. In particular, in the case of employing EUV exposure, the film thickness of the actinic ray-sensitive or radiation-sensitive film is more preferably 10 to 65 nm, and still more preferably 15 to 50 nm. In the case of employing ArF liquid immersion exposure, the film thickness of the actinic ray-sensitive or radiation-sensitive film is more preferably 10 to 120 nm, and still more preferably 15 to 90 nm.

Note that, for an overlying layer of the actinic ray-sensitive or radiation-sensitive film, a topcoat composition may be used to form a topcoat.

The topcoat composition preferably does not mix with the actinic ray-sensitive or radiation-sensitive film, and can be uniformly applied for an overlying layer of the actinic ray-sensitive or radiation-sensitive film. The topcoat is not particularly limited; a publicly known topcoat can be formed by a publicly known process; for example, on the basis of descriptions of Paragraphs [0072] to [0082] in JP2014-059543A, a topcoat can be formed.

For example, a topcoat including a basic compound and described in JP2013-61648A is preferably formed on the actinic ray-sensitive or radiation-sensitive film. Specific examples of the basic compound that can be included in the topcoat include basic compounds that may be included in the resist composition.

The topcoat also preferably includes a compound including at least one group or bond selected from the group consisting of an ether bond, a thioether bond, a hydroxy group, a thiol group, a carbonyl bond, and an ester bond.

Step 2: Exposure step

The step 2 is a step of exposing the actinic ray-sensitive or radiation-sensitive film.

The exposure process may be a process of irradiating the formed actinic ray-sensitive or radiation-sensitive film, through a predetermined mask, with an actinic ray or a radiation.

Examples of the actinic ray or the radiation include infrared light, visible light, ultraviolet light, far-ultraviolet light, extreme ultraviolet light, X-rays, and an electron beam; preferred is 250 nm or less, more preferred is 220 nm or less, and particularly preferred are far-ultraviolet light having a wavelength of 1 to 200 nm and specifically KrF excimer laser (248 nm), ArF excimer laser (193 nm), $F_2$ excimer laser (157 nm), EUV (13.5 nm), X-rays, and an electron beam.

After the exposure, before development, baking (heating) is preferably performed. The baking accelerates the reaction in the exposed regions, to provide higher sensitivity and a better pattern profile.

The heating temperature is preferably 80 to 150°C, more preferably 80 to 140°C, and still more preferably 80 to 130°C.

The heating time is preferably 10 to 1000 seconds, more preferably 10 to 180 seconds, and still more preferably 30 to 120 seconds.

The heating can be performed using means included in an ordinary exposure device and/or an ordinary development device, and may alternatively be performed using a hot plate, for example.

This step is also referred to as post-exposure baking.

Step 3: Development step

The step 3 is a step of using a developer to develop the exposed actinic ray-sensitive or radiation-sensitive film, to form a pattern.

The developer may be an alkali developer or may be a developer containing an organic solvent (hereafter, also referred to as organic-based developer).

Examples of the development process include a process of immersing, for a predetermined time, the substrate in a tank filled with the developer (dipping process), a process of puddling, with the developer, the surface of the substrate using surface tension and leaving the developer at rest for a predetermined time to achieve development (puddling process), a process of spraying the developer to the surface of the substrate (spraying process), and a process of scanning, at a constant rate, over the substrate rotated at a constant rate, a developer ejection nozzle to continuously eject the developer (dynamic dispensing process).

After the step of performing development, a step of performing exchange with another solvent to stop the development may be performed.

The development time is not particularly limited as long as the resin in the unexposed regions is sufficiently dissolved in the time, and is preferably 10 to 300 seconds, and more preferably 20 to 120 seconds.

The temperature of the developer is preferably 0 to 50°C, and more preferably 15 to 35°C.

The alkali developer employed is preferably an alkali aqueous solution including an alkali. The type of the alkali aqueous solution is not particularly limited, but may be, for example, an alkali aqueous solution including a quaternary ammonium salt represented by tetramethylammonium hydroxide, an inorganic alkali, a primary amine, a secondary amine, a tertiary amine, an alcoholamine, a cyclic amine, or the like. In particular, the alkali developer is preferably an aqueous solution of a quaternary ammonium salt represented by tetramethylammonium hydroxide (TMAH). To the alkali developer, an appropriate amount of an alcohol, a surfactant, or the like may be added. The alkali developer ordinarily preferably has an alkali concentration of 0.1 to 20 mass%. The alkali developer ordinarily preferably has a pH of 10.0 to 15.0.

The organic-based developer is preferably a developer containing at least one organic solvent selected from the group consisting of ketone-based solvents, ester-based solvents, alcohol-based solvents, amide-based solvents, ether-based solvents, and hydrocarbon-based solvents.

A plurality of such solvents may be mixed together, or such a solvent may be mixed with a solvent other than those described above or water. The developer as a whole has a moisture content of preferably less than 50 mass%, more preferably less than 20 mass%, still more preferably less than 10 mass%, and particularly preferably contains substantially no moisture.

In the organic-based developer, the content of the organic solvent relative to the total amount of the developer is preferably 50 mass% or more and 100 mass% or less, more preferably 80 mass% or more and 100 mass% or less, still more preferably 90 mass% or more and 100 mass% or less, and particularly preferably 95 mass% or more and 100 mass% or less.

Other step

The pattern forming method preferably includes a step of, after the step 3, using a rinse liquid to perform rinsing.

After the development step using an alkali developer, in the rinsing step, the rinse liquid employed may be, for example, pure water. Note that, to the pure water, an appropriate amount of surfactant may be added.

To the rinse liquid, an appropriate amount of surfactant may be added.

After the development step using an organic-based developer, in the rinsing step, the rinse liquid employed is not particularly limited as long as it does not dissolve the pattern, and may be a solution including an ordinary organic solvent. The rinse liquid employed is preferably a rinse liquid containing at least one organic solvent selected from the group consisting of hydrocarbon-based solvents, ketone-based solvents, ester-based solvents, alcohol-based solvents, amide-based solvents, and ether-based solvents.

The process of performing the rinsing step is not particularly limited; examples include a process of continuously ejecting, onto the substrate rotated at a constant rate, the rinse liquid (spin-coating process), a process of immersing, in a tank filled with the rinse liquid, the substrate for a predetermined time (dipping process), and a process of spraying, to the surface of the substrate, the rinse liquid (spraying process).

The pattern forming method may include a heating step (Post Bake) performed after the rinsing step. In this step, baking removes the developer and the rinse liquid remaining between and within the patterns. In addition, this step also provides an effect of annealing the resist pattern to address the rough surface of the pattern. The heating step after

the rinsing step is performed ordinarily at 40 to 250°C (preferably 90 to 200°C) for ordinarily 10 seconds to 3 minutes (preferably 30 seconds to 120 seconds).

The formed pattern may be used as a mask for subjecting the substrate to etching treatment. Specifically, the pattern formed in the step 3 may be used as a mask for processing the substrate (or the underlayer film and the substrate), to form a pattern in the substrate.

The process of processing the substrate (or the underlayer film and the substrate) is not particularly limited, but is preferably a process of using the pattern formed in the step 3 as a mask for subjecting the substrate (or the underlayer film and the substrate) to dry etching, to thereby form a pattern in the substrate. The dry etching is preferably oxygen plasma etching.

Various materials used in the composition of the present invention and the pattern forming method of this Specification (for example, a solvent, a developer, a rinse liquid, an antireflection film-forming composition, and a topcoat-forming composition) preferably do not include impurities such as metals. The content of impurities included in such materials is preferably 1 mass ppm or less, more preferably 10 mass ppb or less, still more preferably 100 mass ppt or less, particularly preferably 10 mass ppt or less, and most preferably 1 mass ppt or less. The lower limit is not particularly limited, but is preferably 0 mass ppt or more. Examples of the metallic impurities include Na, K, Ca, Fe, Cu, Mg, Al, Li, Cr, Ni, Sn, Ag, As, Au, Ba, Cd, Co, Pb, Ti, V, W, and Zn.

The process of removing, from the various materials, impurities such as metals may be, for example, filtration using a filter. The details of filtration using a filter are described in Paragraph [0321] in WO2020/004306A.

Examples of the process of reducing the amount of impurities such as metals included in the various materials include a process of selecting, as raw materials constituting the various materials, raw materials having lower metal content, a process of subjecting raw materials constituting the various materials to filtration using a filter, and a process of performing distillation under conditions under which contamination is minimized by, for example, lining the interior of the apparatuses with TEFLON (registered trademark).

Instead of the filtration using a filter, an adsorption material may be used to remove impurities; alternatively, the filtration using a filter may be used in combination with an adsorption material. Such adsorption materials can be publicly known adsorption materials, and examples include inorganic-based adsorption materials such as silica gel and zeolite, and organic-based adsorption materials such as active carbon. In order to reduce the amount of impurities such as metals included in the various materials, ingress of metallic impurities in the production steps needs to be prevented. Whether or not metallic impurities are sufficiently removed from the production apparatuses can be determined by measuring the content of metallic components included in the washing liquid having been used for washing the production apparatuses. The content of metallic components included in the washing liquid having been used is preferably 100 mass ppt (parts per trillion) or less, more preferably 10 mass ppt or less, and still more preferably 1 mass ppt or less. The lower limit is not particularly limited, but is preferably 0 mass ppt or more.

To organic-based treatment liquids such as the rinse liquid, in order to prevent electrostatic buildup and the subsequent electrostatic discharge causing failure of the chemical solution pipe and various parts (such as a filter, an O-ring, and a tube), a conductive compound may be added. The conductive compound is not particularly limited, but may be, for example, methanol. The amount of addition is not particularly limited, but is, from the viewpoint of maintaining preferred development performance or rinsing performance, preferably 10 mass% or less, and more preferably 5 mass% or less. The lower limit is not particularly limited, but is preferably 0.01 mass% or more.

Examples of the chemical solution pipe include various pipes formed of SUS (stainless steel), or coated with polyethylene, polypropylene, or a fluororesin (such as polytetrafluoroethylene or a perfluoroalkoxy resin) treated so as to be antistatic. Similarly for the filter and the O-ring, polyethylene, polypropylene, or a fluororesin (such as polytetrafluoroethylene or a perfluoroalkoxy resin) treated so as to be antistatic can be used.

Method for producing electronic device

This Specification also relates to a method for producing an electronic device, the method including the above-described pattern forming method, and an electronic device produced by the production method.

The electronic device in this Specification is, in a preferred embodiment, mounted on electric or electronic devices (such as household appliances, OA (Office Automation), media-related devices, optical devices, and communication devices).

EXAMPLES

Hereinafter, the present invention will be described further in detail with reference to Examples. In the following Examples, materials, usage amounts, ratios, details of treatments, and orders of treatments can be appropriately changed without departing from the spirit and scope of the present invention. Thus, the scope of the present invention should not be construed as being limited to the following Examples.

Various components of actinic ray-sensitive or radiation-sensitive resin composition

Resin (A)

As the resin (A), resins A-1 to A-35 were used.

Table 1 describes the content (mol%) of each repeating unit included in each resin, the weight-average molecular weight (Mw), and the dispersity (Mw/Mn). The content of each repeating unit is the ratio (molar ratio) of the repeating unit to all the repeating units included in each resin. Each repeating unit is described using the structure of the corresponding monomer.

The weight-average molecular weight (Mw) and dispersity (Mw/Mn) of the resin were measured by GPC (carrier: tetrahydrofuran (THF)) (polystyrene-equivalent values). The contents of the repeating units were measured by $^{13}$C-NMR (nuclear magnetic resonance).

Table 1

| Table 1 | Repeating unit 1 | | Repeating unit 2 | | Repeating unit 3 | | Repeating unit 4 | | Mw | Mw/Mn |
|---|---|---|---|---|---|---|---|---|---|---|
| | Type | Content (mol%) | Type | Content (mol%) | Type | Content (mol%) | Type | Content (mol%) | | |
| Resin A-1 | MB-10 | 50 | MA-16 | 50 | - | - | - | - | 8500 | 1.60 |
| Resin A-2 | MB-15 | 40 | MA-7 | 60 | - | - | - | - | 9000 | 1.70 |
| Resin A-3 | MB-7 | 30 | MB-14 | 10 | MA-6 | 60 | - | - | 7000 | 1.55 |
| Resin A-4 | MB-5 | 50 | MB-12 | 10 | MA-15 | 40 | - | - | 7500 | 1.55 |
| Resin A-5 | MB-3 | 20 | MB-20 | 40 | MA-4 | 40 | - | - | 7000 | 1.60 |
| Resin A-6 | MB-4 | 20 | MB-14 | 30 | MA-2 | 50 | - | - | 6500 | 1.63 |
| Resin A-7 | MB-6 | 30 | MB-19 | 10 | MA-3 | 60 | - | - | 9500 | 1.45 |
| Resin A-8 | MB-9 | 30 | MB-20 | 10 | MA-13 | 60 | - | - | 12000 | 1.65 |
| Resin A-9 | MB-4 | 20 | MB-19 | 20 | MA-2 | 60 | - | - | 6000 | 1.55 |
| Resin A-10 | MB-16 | 30 | MA-17 | 70 | - | - | - | - | 8000 | 1.40 |
| Resin A-11 | MB-4 | 30 | MB-31 | 30 | MA-4 | 40 | - | - | 6500 | 1.65 |
| Resin A-12 | MB-30 | 20 | MA-8 | 80 | - | - | - | - | 5500 | 1.65 |
| Resin A-13 | MB-13 | 50 | MA-10 | 50 | - | - | - | - | 15000 | 1.75 |
| Resin A-14 | MB-8 | 30 | MA-20 | 70 | - | - | - | - | 9000 | 1.60 |
| Resin A-15 | MB-18 | 30 | MB-29 | 30 | MA-14 | 40 | - | - | 8000 | 1.55 |
| Resin A-16 | MB-3 | 30 | MB-20 | 20 | MA-2 | 50 | - | - | 7500 | 1.70 |

(continued)

| Table 1 | Repeating unit 1 | | Repeating unit 2 | | Repeating unit 3 | | Repeating unit 4 | | Mw | Mw/Mn |
|---|---|---|---|---|---|---|---|---|---|---|
| | Type | Content (mol%) | Type | Content (mol%) | Type | Content (mol%) | Type | Content (mol%) | | |
| Resin A-17 | MB-1 | 30 | MB-26 | 30 | MA-11 | 40 | - | - | 18000 | 1.80 |
| Resin A-18 | MB-27 | 60 | MA-5 | 40 | - | - | - | - | 7500 | 1.65 |
| Resin A-19 | MB-17 | 30 | MB-24 | 10 | MA-19 | 60 | - | - | 8000 | 1.70 |
| Resin A-20 | MB-11 | 30 | MB-28 | 40 | MA-9 | 30 | - | - | 9500 | 1.80 |
| Resin A-21 | MB-3 | 20 | MB-20 | 10 | MB-32 | 10 | MA-2 | 60 | 10000 | 1.70 |
| Resin A-22 | MB-2 | 60 | MA-1 | 40 | - | - | - | - | 11000 | 1.65 |
| Resin A-23 | MB-21 | 50 | MA-12 | 50 | - | - | - | - | 6500 | 1.60 |
| Resin A-24 | MB-23 | 40 | MA-10 | 60 | - | - | - | - | 8000 | 1.55 |
| Resin A-25 | MB-1 | 20 | MB-19 | 20 | MA-2 | 60 | - | - | 8000 | 1.55 |
| Resin A-26 | MB-25 | 30 | MA-2 | 70 | - | - | - | - | 7500 | 1.60 |
| Resin A-27 | MB-3 | 40 | MA-3 | 60 | - | - | - | - | 9500 | 1.60 |
| Resin A-28 | MB-22 | 40 | MA-4 | 60 | - | - | - | - | 10000 | 1.70 |
| Resin A-29 | MB-3 | 30 | MB-33 | 20 | MA-2 | 50 | - | - | 6600 | 1.63 |
| Resin A-30 | MB-7 | 30 | MB-14 | 10 | MA-18 | 60 | - | - | 8100 | 1.65 |
| Resin A-31 | MB-1 | 20 | MB-19 | 20 | MA-21 | 60 | - | - | 6600 | 1.65 |
| Resin A-32 | MB-17 | 60 | MA-24 | 40 | - | - | - | - | 8200 | 1.71 |
| Resin A-33 | MB-4 | 20 | MB-19 | 20 | MA-23 | 60 | - | - | 6500 | 1.60 |
| Resin A-34 | MB-14 | 30 | MA-22 | 70 | - | - | - | - | 7200 | 1.58 |
| Resin A-35 | MB-19 | 25 | MB-34 | 25 | MA-2 | 50 | - | - | 8400 | 1.59 |

The structural formulas of the monomers described in Table 1 (monomers corresponding to the repeating units) will be described below.

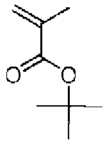
MA-1

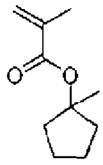
MA-2

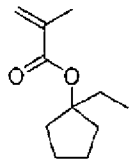
MA-3

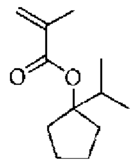
MA-4

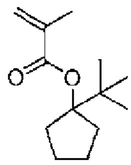
MA-5

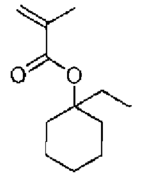
MA-6

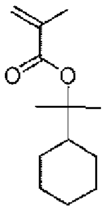
MA-7

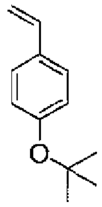
MA-8

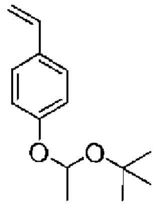
MA-9

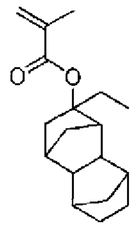
MA-10

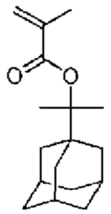
MA-11

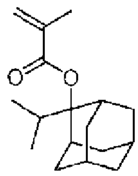
MA-12

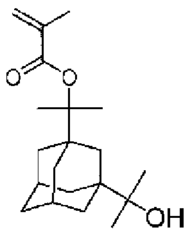
MA-13

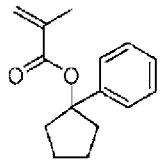
MA-14

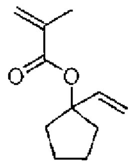
MA-15

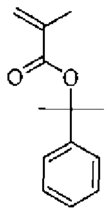
MA-16

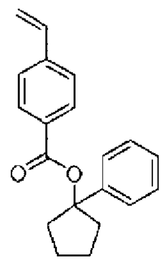
MA-17

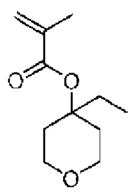
MA-18

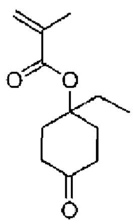
MA-19

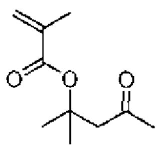
MA-20

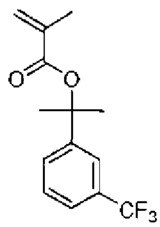
MA-21

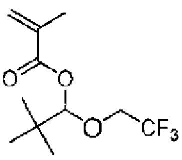
MA-22

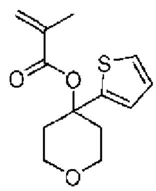
MA-23

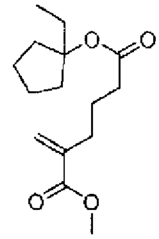
MA-24

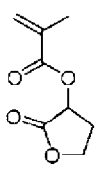
MB-1

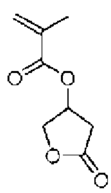
MB-2

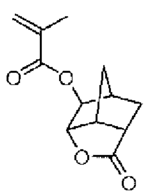
MB-3

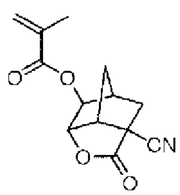
MB-4

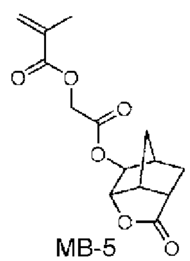
MB-5

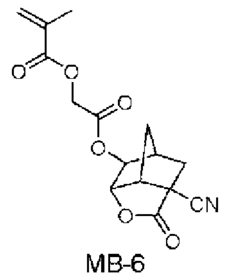

MB-6

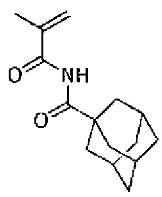

MB-7

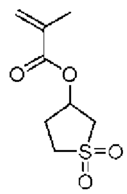

MB-8

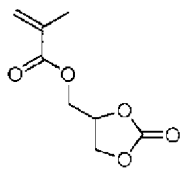

MB-9

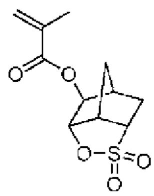

MB-10

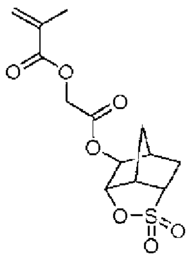

MB-11

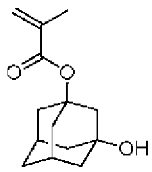

MB-12

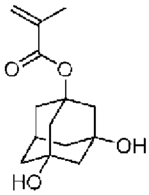

MB-13

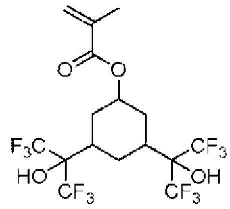

MB-14

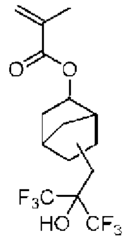

MB-15

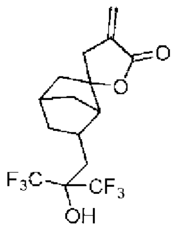

MB-16

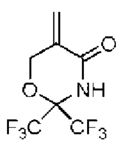

MB-17

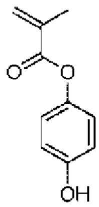

MB-18

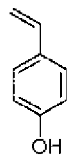

MB-19

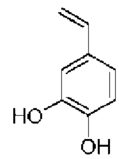

MB-20

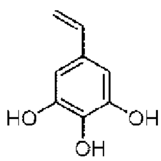

MB-21

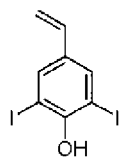

MB-22

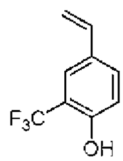

MB-23

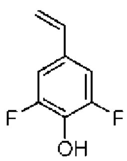

MB-24

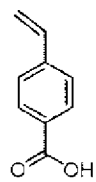

MB-25

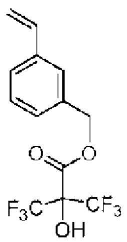

MB-26

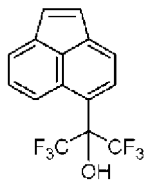

MB-27

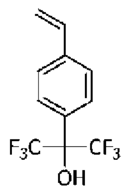

MB-28

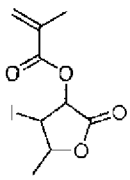

MB-29

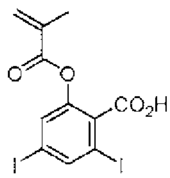

MB-30

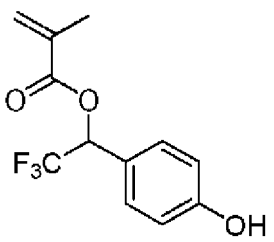

MB-31

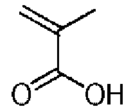

MB-32

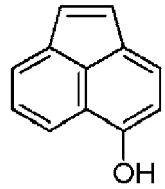

MB-33

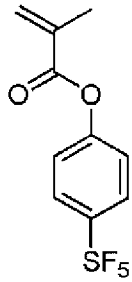

MB-34

Synthesis of Resin A-1

Propylene glycol monomethyl ether acetate (28 g) was heated to 80°C under a nitrogen stream. To this liquid under stirring, a mixed solution of a monomer (30 g) represented by MA-16, a monomer (38 g) represented by MB-10, propylene glycol monomethyl ether acetate (112 g), and dimethyl 2,2'-azobisisobutyrate [V-601, manufactured by FUJIFILM Wako Pure Chemical Corporation] (5.7 g) was added dropwise over 6 hours to obtain a reaction solution. After completion of the dropwise addition, the reaction solution was further stirred at 80°C for 2 hours. The obtained reaction solution was left to cool, and subsequently subjected to reprecipitation with a mixed solvent of a large amount of heptane and ethyl acetate (heptane:ethyl acetate = 9:1, mass ratio) and subsequently to filtration; and the obtained solid was vacuum-dried to thereby obtain 58 g of a resin A-1. The obtained resin A-1 was subjected to GPC (carrier: tetrahydrofuran (THF)) and found to have a weight-average molecular weight (Mw: polystyrene-equivalent) of 8500, and a dispersity (Mw/Mn) of 1.60. The molar content ratio of the repeating units measured by $^{13}C$-NMR (nuclear magnetic resonance) was 50/50.

The other resins (A) used in EXAMPLES were also synthesized in the same manner as described above.

Photoacid generator

The structures of the photoacid generators used will be described below.

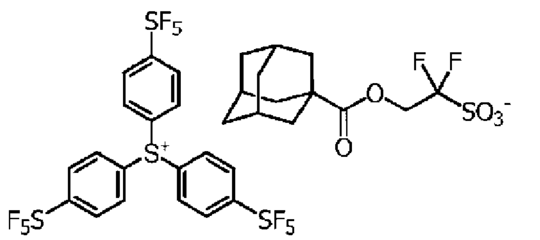

**(I)-1**

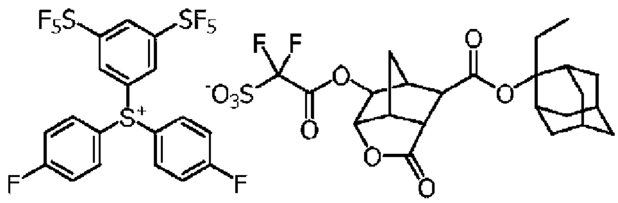

**(I)-2**

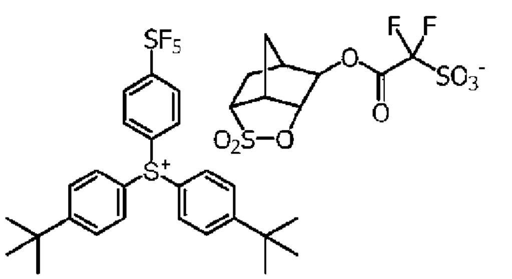

**(I)-3**

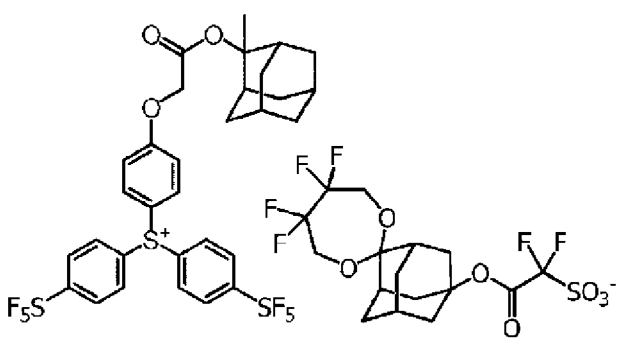

**(I)-4**

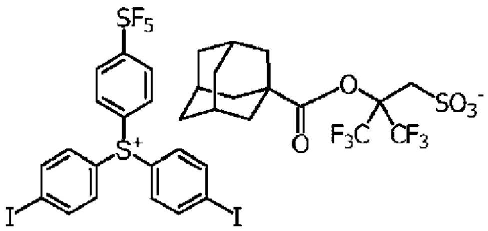

(I)-5

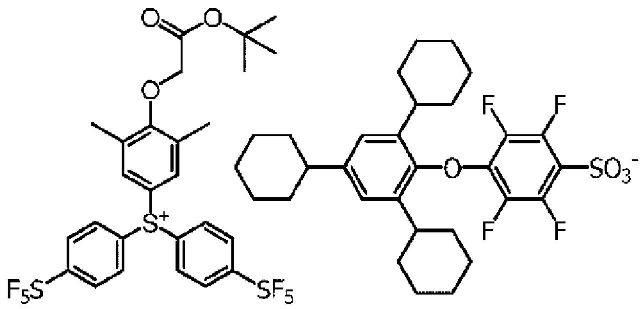

(I)-6

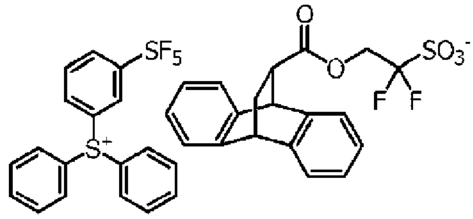

(I)-7

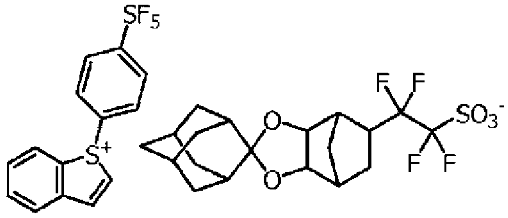

(I)-8

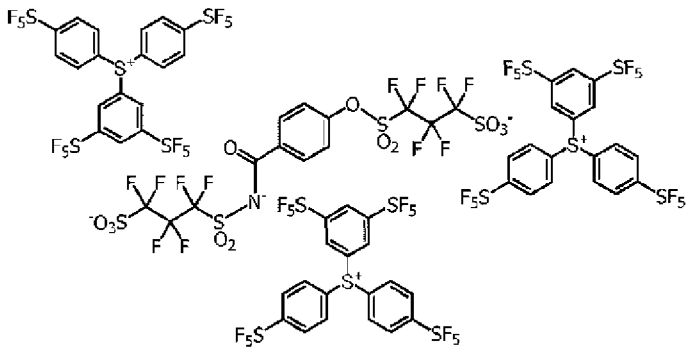

(I)-9

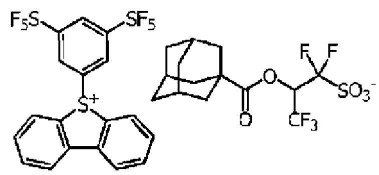

(I)-10

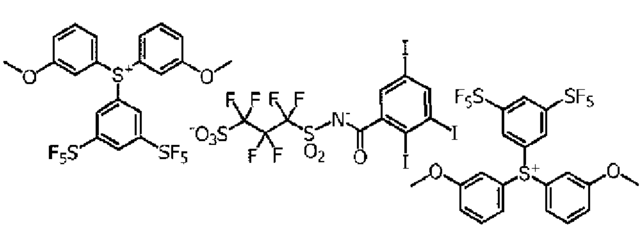

(I)-11

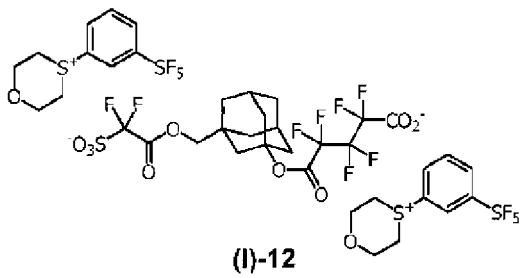

(I)-12

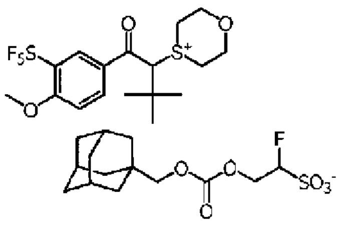

(I)-13

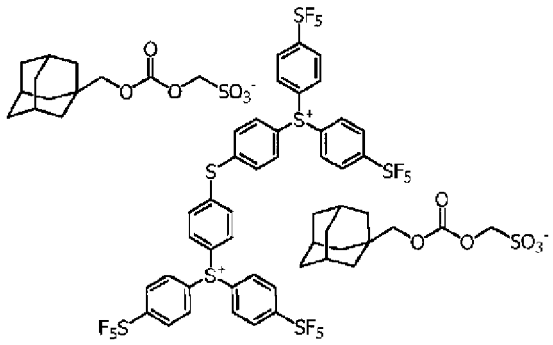

(I)-14

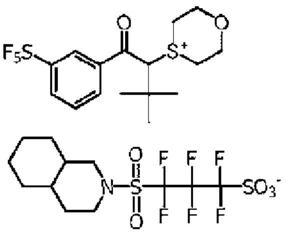

(I)-15

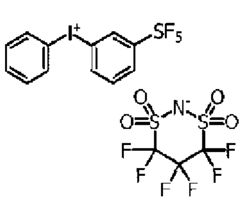

(I)-16

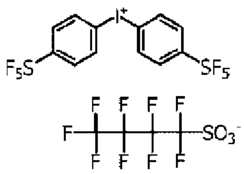

(I)-17

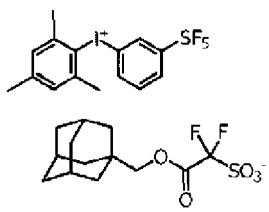

(I)-18

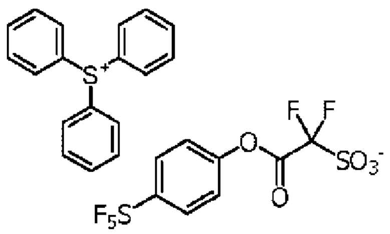

(I)-19

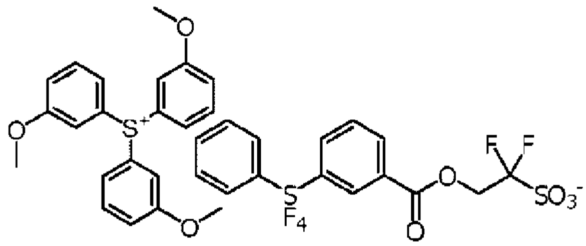

(I)-20

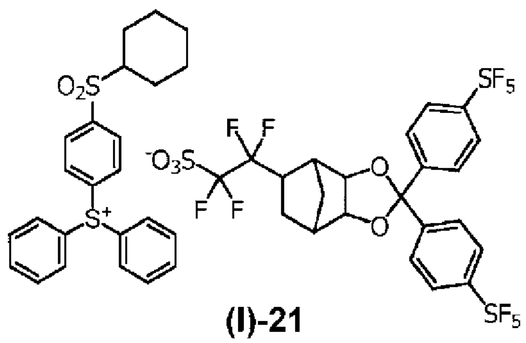

(I)-21

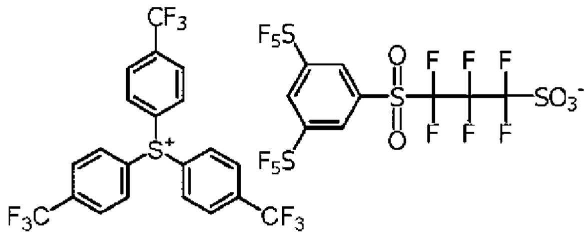

(I)-22

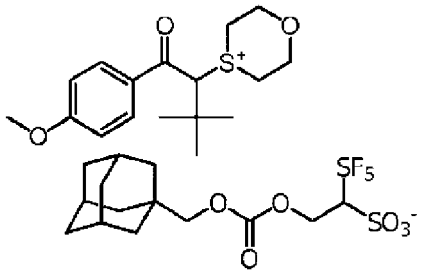

(I)-23

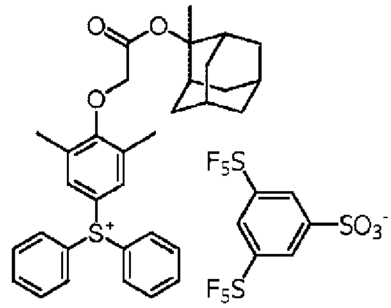

(I)-24

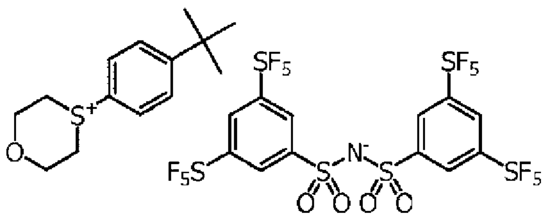

(I)-25

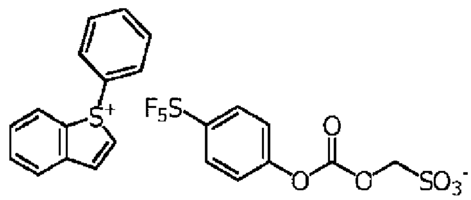

(I)-26

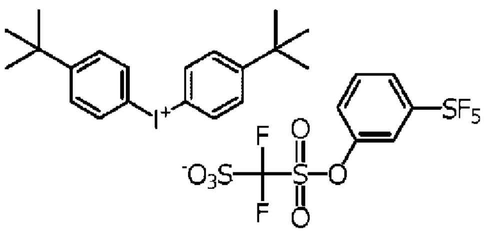

(I)-27

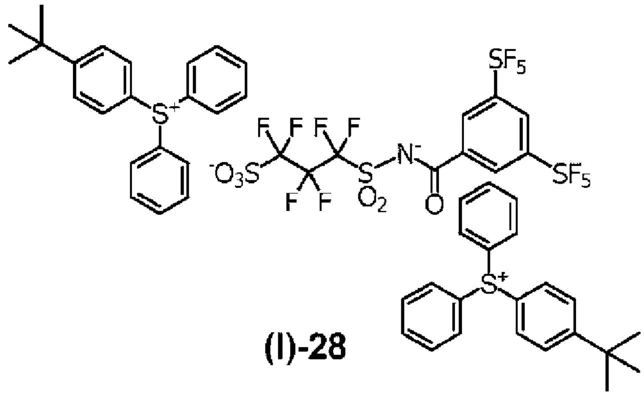

(I)-28

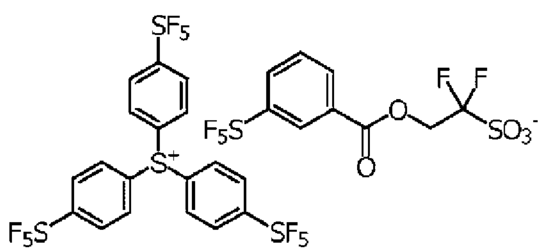

(I)-29

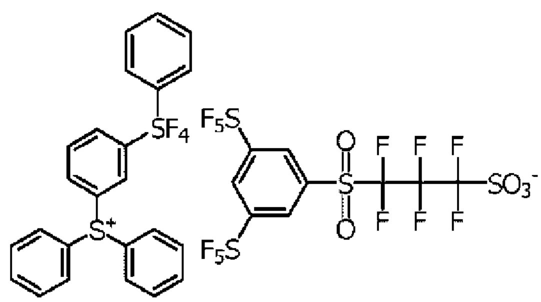

(I)-30

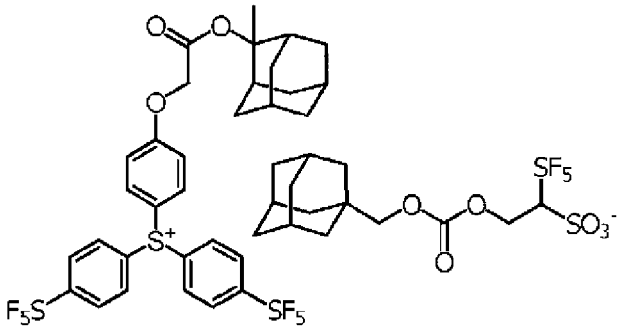

(I)-31

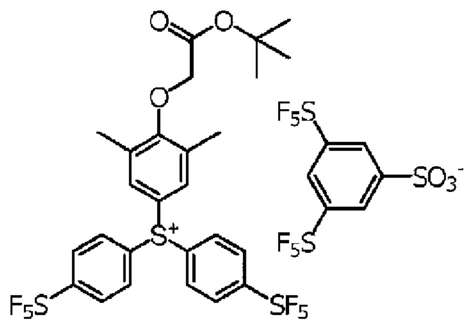

(I)-32

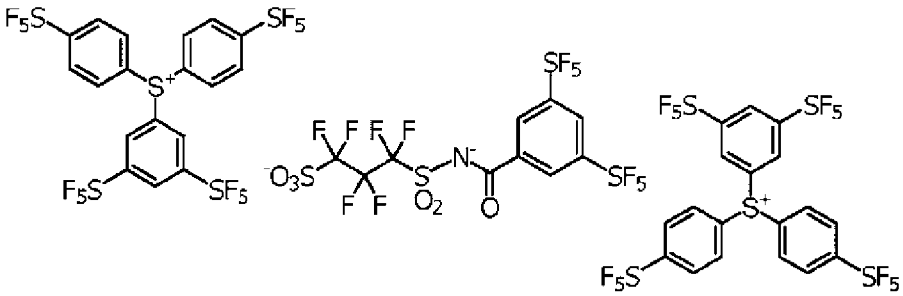

(I)-33

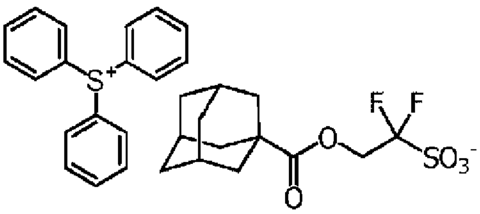

(III)-1

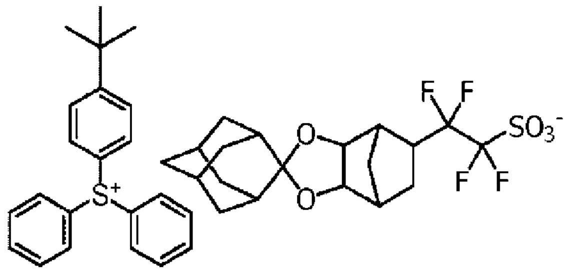

(III)-2

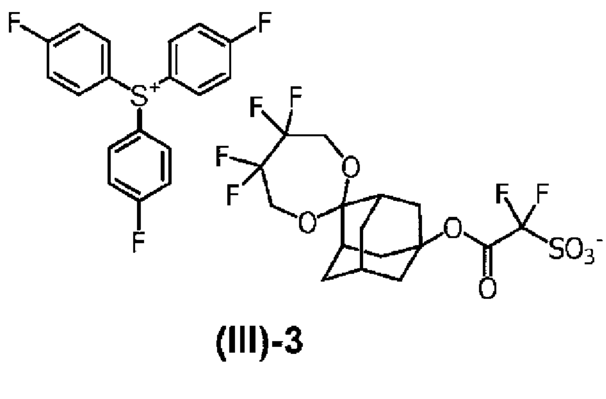

(III)-3

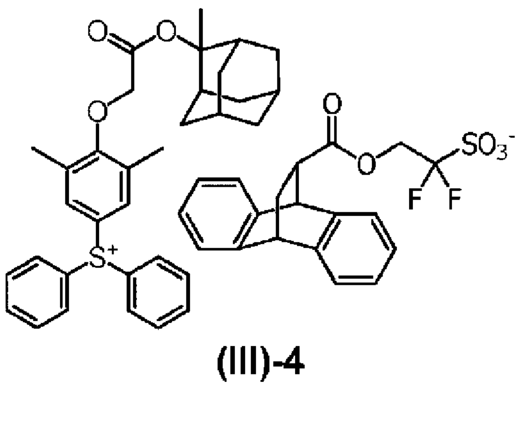

(III)-4

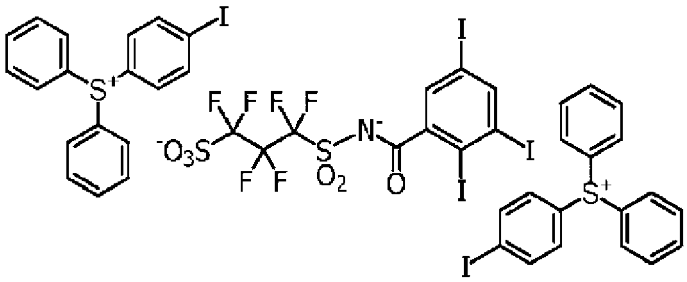

(III)-5

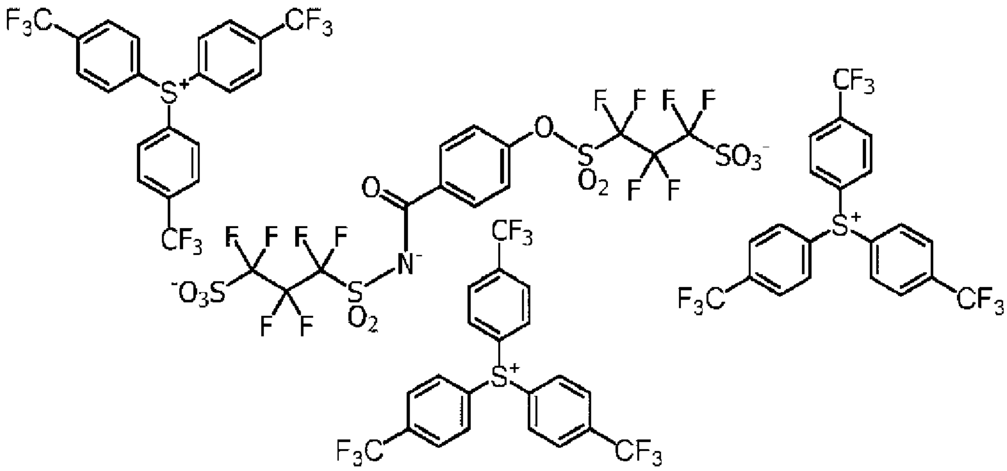

(III)-6

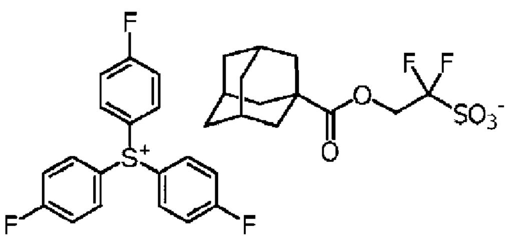

(Z)-1

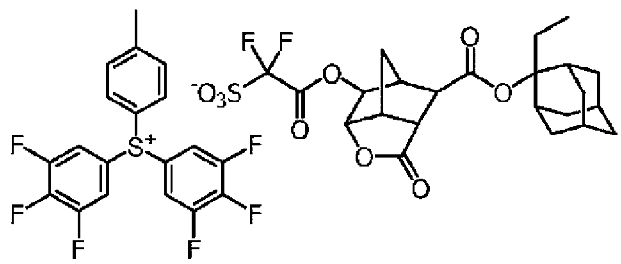

(Z)-2

Synthesis of compound (I)-1

(1) Synthesis of compound (I)-1-A

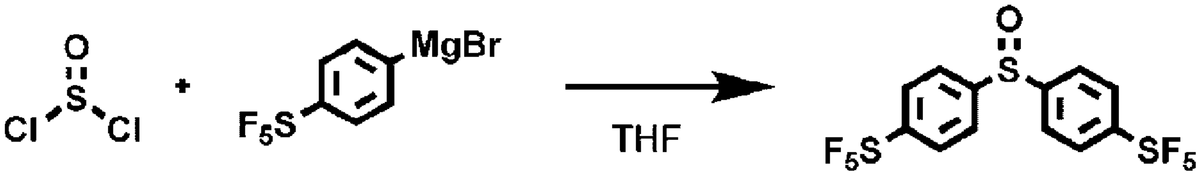


(I)-1-1

(I)-1-A

Magnesium (180 g) was added to tetrahydrofuran (500 mL) to obtain a mixture. To the obtained mixture, 4-bromophenylsulfur pentafluoride (190.6 g) was added dropwise. Subsequently, the mixture was stirred for 1 hour to

prepare (I)-1-1.

Thionyl chloride (37.7 g) was added to tetrahydrofuran (THF) (500 mL) to obtain a mixed solution. The obtained mixed solution was cooled to 0°C, and to the mixed solution, the previously prepared (I)-1-1 was added dropwise. The mixed solution was stirred for 1 hour; subsequently, to the mixed solution, 1 N hydrochloric acid (600 mL) was added while the temperature of the mixed solution was maintained at 0°C.

The reaction product generated in the mixed solution was extracted with ethyl acetate (600 mL). The resultant organic phase was washed with a saturated aqueous sodium hydrogen carbonate solution (500 mL) and water (500 mL), and subsequently the solvent was distilled off from the organic phase. The obtained concentrated product was washed with hexane (300 mL) and filtered to thereby obtain (I)-1-A (71.9 g) as a residue (yield: 50%).

(2) Synthesis of compound (I)-1-B

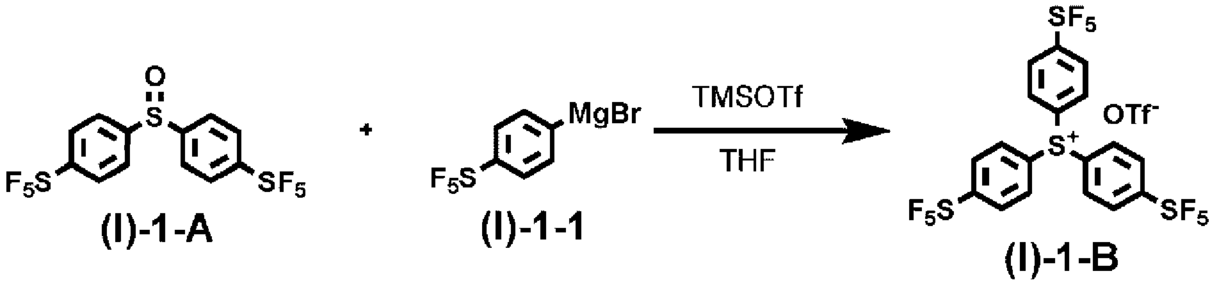


Magnesium (5.0 g) was added to tetrahydrofuran (170 mL) to obtain a mixture. To the obtained mixture, 4-bromophenylsulfur pentafluoride (50.2 g) was added dropwise. Subsequently, the mixture was stirred for 1 hour to prepare (I)-1-1.

The above-described (I)-1-A (26.9 g) was added to tetrahydrofuran (170 mL) to obtain a mixed solution. The obtained mixed solution was cooled to 0°C, and to the mixed solution, trimethylsilyl trifluoromethanesulfonate (TMSOTf) (91.9 g) was added dropwise. Subsequently, the previously prepared (I)-1-1 was added dropwise to the mixed solution, and subsequently the mixed solution was stirred for 1 hour.

To the mixed solution, water (500 mL) was added while the temperature of the mixed solution was maintained at 0°C, and subsequently the reaction product generated in the mixed solution was extracted with methylene chloride (500 mL). The obtained organic phase was washed with water (500 mL), and subsequently the solvent was distilled off from the organic phase. The obtained concentrated product was washed with diisopropyl ether (300 mL) and filtered to thereby obtain (I)-1-B (19.4 g) as a residue (yield: 47%).

(3) Synthesis of compound (I)-1

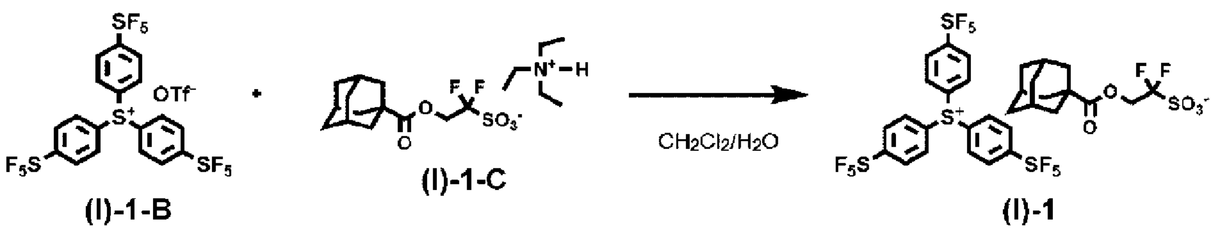


Methylene chloride (150 mL) and water (150 mL) were mixed together to obtain a mixed solution. To the mixed solution, (I)-1-B (100 g) and (I)-1-C (6.63 g) were added. The mixed solution was stirred for 1 hour and subsequently the aqueous phase was removed from the mixed solution. The remaining organic layer was washed with 0.01 N hydrochloric acid (100 mL) and water (100 mL). The solvent was distilled off from the organic phase to thereby obtain (I)-1 (14.7 g) (yield: 98%).

The obtained (I)-1 was subjected to $^{1}$H NMR measurement.

$^{1}$H NMR (400 MHz, acetone-d6) d 1.71-1.79 (m, 6H), 1.93-1.94 (m, 6H), 2.01 (s, 3H), 4.65 (d, J= 15.2 Hz, 2H), 8.19 (d,

J = 8.5 Hz, 6H), 8.39 (d, J = 8.5 Hz, 6H)

The other photoacid generators used in EXAMPLES were also synthesized in the same manner as described above.

Acid diffusion control agent

The structures of the acid diffusion control agents used are as follows.

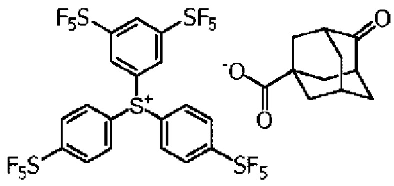

(II)-1

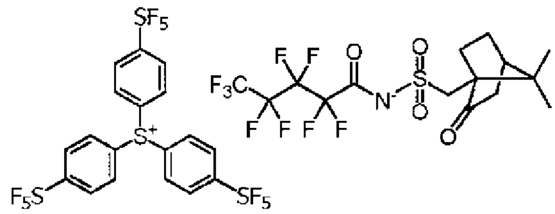

(II)-2

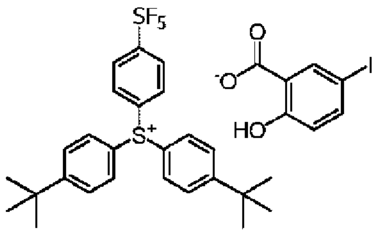

(II)-3

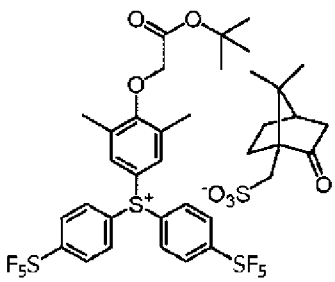

(II)-4

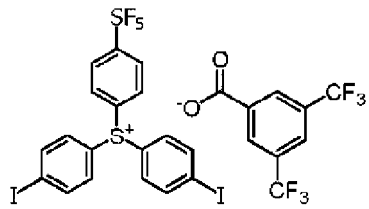

(II)-5

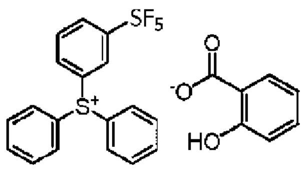

(II)-6

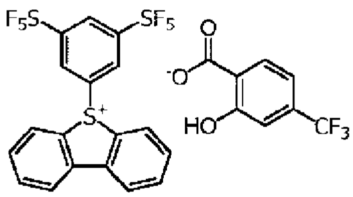

(II)-7

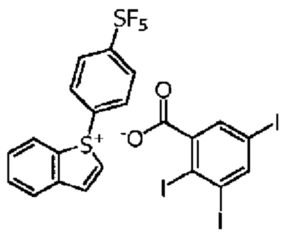

(II)-8

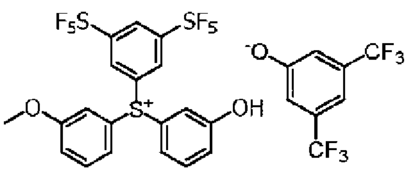

(II)-9

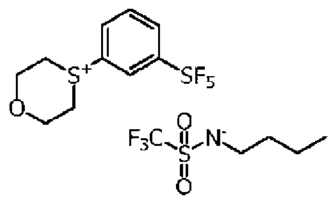

(II)-10

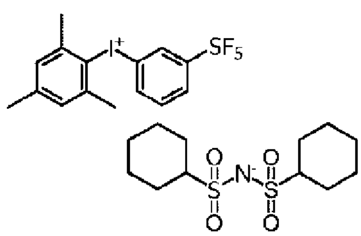

(II)-11

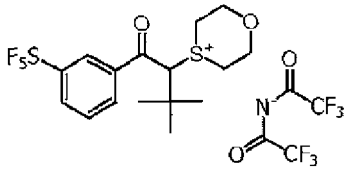

(II)-12

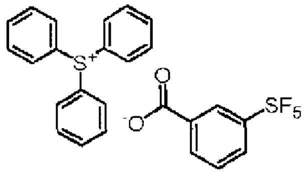

(II)-13

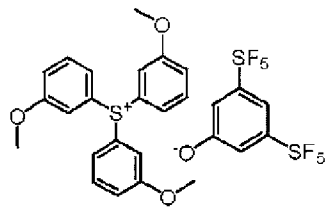

(II)-14

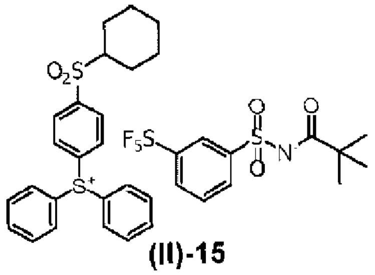

(II)-15

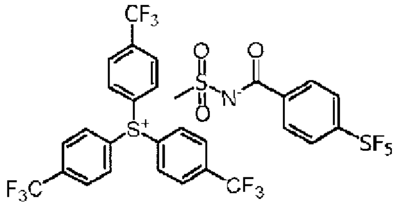

(II)-16

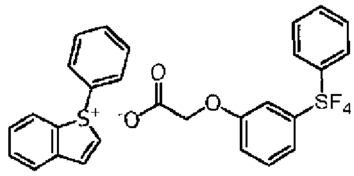

(II)-17

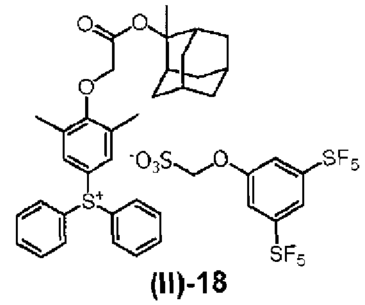

(II)-18

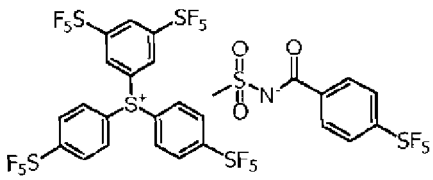

(II)-19

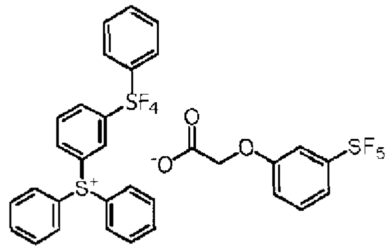

(II)-20

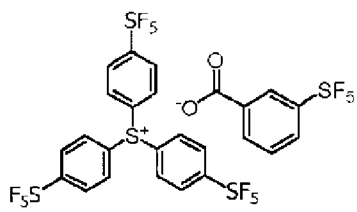

(II)-21

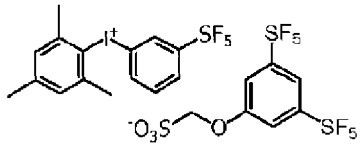

(II)-22

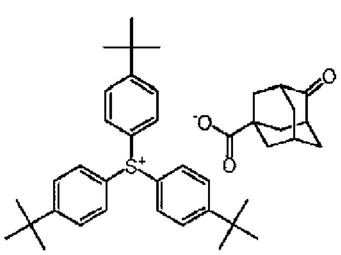

(IV)-1

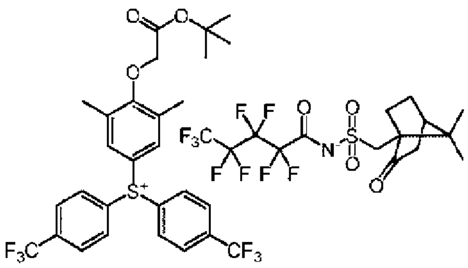

(IV)-2

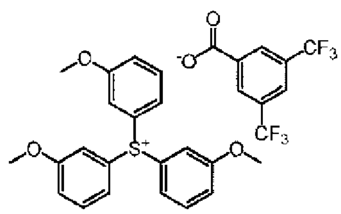

(IV)-3

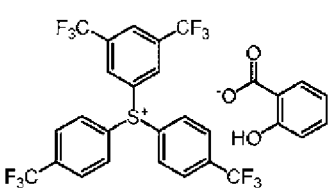

(IV)-4

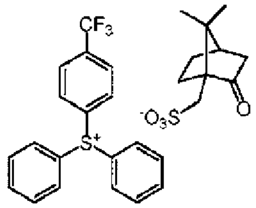

(IV)-5

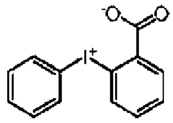

(IV)-6

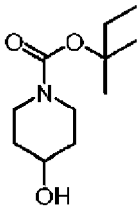

B-1

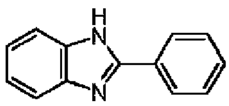

B-2

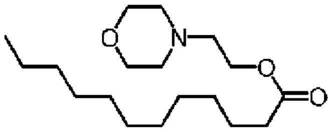

B-3

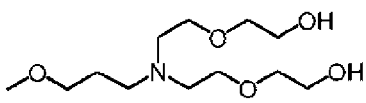

B-4

(Y) Ionic compound

The compounds (I)-1 to (I)-33 and (II)-1 to (II)-22 are the ionic compounds (Y).

Note that the compounds (III)-1 to (III)-6, (IV)-1 to (IV)-6, and (Z)-1 to (Z)-2 are not the ionic compounds (Y).

Hydrophobic resin

The structural formulas of resins C-1 to C-8 used as the hydrophobic resin will be described below.

Table 2 describes the content (mol%) of each repeating unit included in each resin, the weight-average molecular weight (Mw), and the dispersity (Mw/Mn). The content of each repeating unit is the ratio (molar ratio) of the repeating unit to all the repeating units included in each resin. The values of the contents of the repeating units described in Table 2 correspond to the following structural formulas in order from the left. The repeating units are described using the structures of the corresponding monomers.

The weight-average molecular weight (Mw) and dispersity (Mw/Mn) of the resin were measured by GPC (carrier: tetrahydrofuran (THF)) (polystyrene-equivalent values). The contents of the repeating units were measured by $^{13}$C-NMR (nuclear magnetic resonance).

Table 2

| Table 2 | Molar ratio of repeating unit 1 | | Molar ratio of repeating unit 2 | | Molar ratio of repeating unit 3 | | Molar ratio of repeating unit 4 | | Mw | Mw/Mn |
|---|---|---|---|---|---|---|---|---|---|---|
| | Type | Content (mol%) | Type | Content (mol%) | Type | Content (mol%) | Type | Content (mol%) | | |
| Resin C-1 | MC-9 | 50 | MC-1 | 50 | - | - | - | - | 12000 | 1.5 |
| Resin C-2 | MC-2 | 40 | MC-8 | 50 | MC-5 | 5 | MC-11 | 5 | 6000 | 1.3 |
| Resin C-3 | MC-6 | 50 | MC-2 | 50 | - | - | - | - | 15000 | 1.5 |
| Resin C-4 | MC-3 | 100 | - | - | - | - | - | - | 23000 | 1.7 |
| Resin C-5 | MC-8 | 10 | MC-10 | 85 | MC-5 | 5 | - | - | 11000 | 1.4 |
| Resin C-6 | MC-4 | 80 | MC-7 | 20 | - | - | - | - | 13000 | 1.4 |

(continued)

| Table 2 | Molar ratio of repeating unit 1 | | Molar ratio of repeating unit 2 | | Molar ratio of repeating unit 3 | | Molar ratio of repeating unit 4 | | Mw | Mw/Mn |
|---|---|---|---|---|---|---|---|---|---|---|
| | Type | Content (mol%) | Type | Content (mol%) | Type | Content (mol%) | Type | Content (mol%) | | |
| Resin C-7 | MC-3 | 50 | MC-12 | 50 | - | - | - | - | 12000 | 1.5 |
| Resin C-8 | MC-2 | 50 | MC-13 | 50 | - | - | - | - | 10000 | 1.6 |

The structural formulas of the monomers described in Table 2 (monomers corresponding to the repeating units) are as follows.

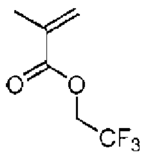

**MC-1**

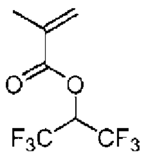

**MC-2**

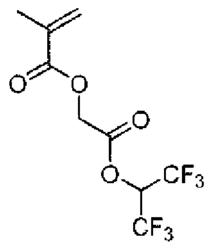

**MC-3**

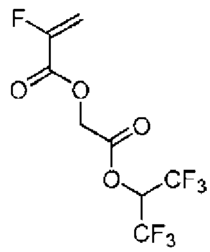

**MC-4**

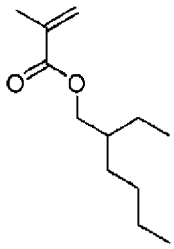

**MC-5**

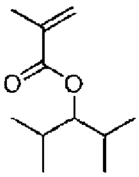

**MC-6**

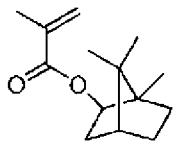

**MC-7**

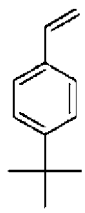

**MC-8**

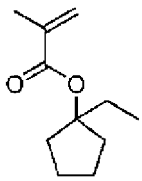

**MC-9**

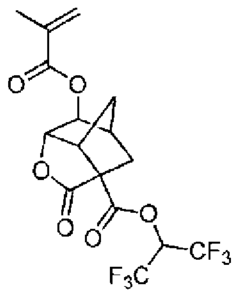

**MC-10**

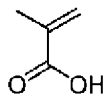

**MC-11**

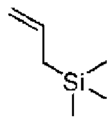

**MC-12**

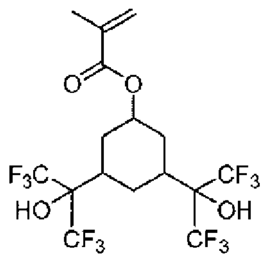

**MC-13**

Surfactant

The surfactants used are as follows.

E-1: MEGAFACE F176 (manufactured by DIC Corporation, fluorine-based surfactant)
E-2: MEGAFACE R08 (manufactured by DIC Corporation, fluorine-based and silicone-based surfactant)
E-3: PF656 (manufactured by OMNOVA SOLUTIONS INC., fluorine-based surfactant) Solvent

The solvents used are as follows.

F-1: propylene glycol monomethyl ether acetate (PGMEA)
F-2: propylene glycol monomethyl ether (PGME)
F-3: propylene glycol monoethyl ether (PGEE)
F-4: cyclohexanone
F-5: cyclopentanone
F-6: 2-heptanone
F-7: ethyl lactate
F-8: γ-butyrolactone
F-9: propylene carbonate

Preparation of resist compositions

The components described in Tables 3 to 5 were mixed together so that the solid-content concentration became 2.0 mass%. Subsequently, the obtained mixed solution was filtered by being passed through first a polyethylene filter having a pore size of 50 nm, subsequently a nylon filter having a pore size of 10 nm, and finally a polyethylene filter having a pore size of 5 nm in this order; in this way, resist compositions (Re-1 to Re-73 and Re-C1 to Re-C2) were prepared
Note that the solid content means all components other than the solvent. The obtained resist compositions were used in Examples and Comparative Examples
In Tables 3 to 5, the columns "mass%" indicate the content (mass%) of each component relative to the total solid content in the resist composition.

Table 3

| Resist composition | Resin (A) | | Photoacid generator (B) | | Acid diffusion control agent | | Hydrophobic resin | | Surfactant | | Solvent | |
|---|---|---|---|---|---|---|---|---|---|---|---|---|
| | Type | mass% | Type | mass% | Type | mass% | Type | mass% | Type | mass% | Type | Mixing ratio (mass ratio) |
| Re-1 | A-34 | 63.0 | (I)-1 | 26.0 | (IV)-1 | 11.0 | - | - | - | - | F-1 | 100 |
| Re-2 | A-3 | 60.4 | (I)-2 | 29.5 | (IV)-1 | 9.0 | C-8 | 1.1 | - | - | F-1 | 100 |
| Re-3 | A-6 | 59.1 | (I)-3 | 30.0 | (IV)-1 | 9.0 | C-5 | 1.9 | - | - | F-1/F-3 | 80/20 |
| Re-4 | A-20 | 67.1 | (I)-4 | 22.5 | (IV)-2 | 8.0 | C-4 | 2.4 | - | - | F-1/F-9 | 90/10 |
| Re-5 | A-12 | 74.5 | (I)-5 | 18.0 | (IV)-2 | 7.5 | - | - | - | - | F-1/F-7 | 80/20 |
| Re-6 | A-9 | 51.8 | (I)-6 | 35.0 | (IV)-2 | 13.0 | - | - | E-1/E-2 | 0.1/0.1 | F-1/F-8 | 85/15 |
| Re-7 | A-15 | 57.5 | (I)-7 | 32.0 | (IV)-3 | 10.5 | - | - | - | - | F-4 | 100 |
| Re-8 | A-30 | 69.5 | (I)-8 | 22.0 | (IV)-3 | 7.0 | C-3 | 1.5 | - | - | F-1/F-5 | 50/50 |
| Re-9 | A-33 | 58.0 | (I)-9 | 40.0 | (IV)-3 | 2.0 | - | - | - | - | F-1 | 100 |
| Re-10 | A-12 | 73.0 | (I)-10 | 20.0 | (IV)-4 | 7.0 | - | - | - | - | F-1/F-2/F-8 | 70/25/5 |
| Re-11 | A-1 | 62.0 | (I)-11 | 38.0 | - | - | - | - | - | - | F-1/F-6 | 40/60 |
| Re-12 | A-2 | 67.0 | (I)-12 | 29.0 | (IV)-4 | 2.0 | C-1 | 2.0 | - | - | F-1/F-5 | 50/50 |
| Re-13 | A-27 | 58.0 | (I)-13 | 32.0 | (IV)-5 | 10.0 | - | - | - | - | F-1 | 100 |
| Re-14 | A-31 | 62.0 | (I)-14 | 32.0 | (IV)-5 | 6.0 | - | - | - | - | F-1/F-2/F-8 | 70/25/5 |
| Re-15 | A-5 | 57.9 | (I)-15 | 33.0 | (IV)-5 | 9.0 | - | - | E-1 | 0.1 | F-7 | 100 |
| Re-16 | A-23 | 65.5 | (I)-16 | 29.5 | (IV)-6 | 5.0 | - | - | - | - | F-1/F-8 | 85/15 |

(continued)

| Resist composition | Resin (A) | | Photoacid generator (B) | | Acid diffusion control agent | | Hydrophobic resin | | Surfactant | | Solvent | |
|---|---|---|---|---|---|---|---|---|---|---|---|---|
| | Type | mass% | Type | mass% | Type | mass% | Type | mass% | Type | mass% | Type | Mixing ratio (mass ratio) |
| Re-17 | A-29 | 52.0 | (I)-17 | 41.0 | (IV)-6 | 7.0 | - | - | - | - | F-1/F-2 | 70/30 |
| Re-18 | A-17 | 68.9 | (I)-18 | 26.0 | (IV)-6 | 5.0 | - | - | E-2 | 0.1 | F-1/F-5 | 50/50 |
| Re-19 | A-10 | 52.6 | (I)-19 | 31.0 | (IV)-1 | 13.0 | C-6 | 3.4 | - | - | F-1/F-2 | 70/30 |
| Re-20 | A-18 | 66.0 | (I)-20 | 30.0 | B-1 | 4.0 | - | - | - | - | F-1/F-6 | 40/60 |
| Re-21 | A-8 | 57.0 | (I)-21 | 39.0 | B-2 | 4.0 | - | - | - | - | F-4 | 100 |
| Re-22 | A-14 | 81.5 | (I)-22 | 16.0 | B-3 | 2.5 | - | - | - | - | F-4 | 100 |
| Re-23 | A-22/A-24 | 32.0/32.0 | (I)-23 | 31.0 | B-4 | 5.0 | - | - | - | - | F-1 | 100 |
| Re-24 | A-11 | 65.5 | (I)-24 | 26.0 | (IV)-1 | 8.5 | - | - | - | - | F-1/F-8 | 85/15 |
| Re-25 | A-16 | 41.0 | (I)-25 | 41.0 | (IV)-2 | 18.0 | - | - | - | - | F-7 | 100 |

Table 4

| Resist composition | Resin (A) | | Photoacid generator (B) | | Acid diffusion control agent | | Hydrophobic resin | | Surfactant | | Solvent | |
|---|---|---|---|---|---|---|---|---|---|---|---|---|
| | Type | mass% | Type | mass% | Type | mass% | Type | mass% | Type | mass% | Type | Mixing ratio (mass ratio) |
| Re-26 | A-26 | 60.5 | (I)-26 | 26.5 | (IV)-3 | 13.0 | - | - | - | - | F-1/F-8 | 85/15 |
| Re-27 | A-13 | 50.0 | (I)-27 | 37.0 | (IV)-4 | 13.0 | - | - | - | - | F-1/F-2 | 70/30 |
| Re-28 | A-21 | 49.9 | (I)-28 | 50.0 | - | - | - | - | E-3 | 0.1 | F-1/F-3 | 90/10 |
| Re-29 | A-28 | 63.0 | (I)-29 | 29.0 | (IV)-4 | 8.0 | - | - | - | - | F-4 | 100 |
| Re-30 | A-1 | 57.5 | (I)-30 | 32.0 | (IV)-4 | 10.5 | - | - | - | - | F-1/F-8 | 85/15 |
| Re-31 | A-21 | 64.0 | (I)-31 | 31.0 | (IV)-5 | 5.0 | - | - | - | - | F-1/F-3 | 90/10 |
| Re-32 | A-28 | 66.0 | (I)-32 | 29.0 | (IV)-5 | 5.0 | - | - | - | - | F-4 | 100 |
| Re-33 | A-1 | 47.0 | (I)-33 | 53.0 | - | - | - | - | - | - | F-1/F-8 | 85/15 |
| Re-34 | A-32 | 63.1 | (III) -1 | 20.0 | (II)-1 | 14.0 | C-7 | 2.9 | - | - | F-1 | 100 |
| Re-35 | A-19 | 59.9 | (III) -1 | 25.0 | (II)-2 | 14.0 | C-2 | 1.1 | - | - | F-4 | 100 |
| Re-36 | A-25 | 53.0 | (III) -1 | 30.0 | (II)-3 | 17.0 | - | - | - | - | F-1/F-6 | 40/60 |
| Re-37 | A-7 | 54.5 | (III)-2 | 30.0 | (II)-4 | 15.5 | - | - | - | - | F-4 | 100 |
| Re-38 | A-4 | 61.0 | (III)-2 | 26.0 | (II)-5 | 13.0 | - | | - | - | F-1/F-8 | 85/15 |
| Re-39 | A-32 | 72.1 | (III)-2 | 19.0 | (II) -6 | 6.0 | C-7 | 2.9 | - | - | F-1 | 100 |
| Re-40 | A-19 | 55.4 | (III)-3 | 31.0 | (II) -7 | 12.5 | C-2 | 1.1 | - | - | F-4 | 100 |
| Re-41 | A-35 | 61.0 | (III)-3 | 27.0 | (II)-8 | 12.0 | - | - | - | - | F-1/F-2 | 70/30 |
| Re-42 | A-31 | 53.0 | (III)-3 | 33.0 | (II)-9 | 14.0 | - | - | - | - | F-1/F-2/F-8 | 70/25/5 |
| Re-43 | A-22/A-24 | 36.0/36.5 | (III)-4 | 22.0 | (II)-10 | 5.5 | - | - | - | - | F-7 | 100 |
| Re-44 | A-26 | 48.5 | (III)-4 | 38.0 | (II)-11 | 13.5 | - | - | - | - | F-1/F-8 | 85/15 |
| Re-45 | A-26 | 67.0 | (III)-4 | 25.0 | (II)-12 | 8.0 | - | - | - | - | F-1/F-8 | 85/15 |
| Re-46 | A-13 | 67.0 | (III)-5 | 28.0 | (II) -13 | 5.0 | - | - | - | - | F-1/F-2 | 70/30 |
| Re-47 | A-21 | 58.0 | (III)-5 | 35.0 | (II)-14 | 7.0 | - | - | - | - | F-1/F-3 | 90/10 |
| Re-48 | A-28 | 71.0 | (III)-5 | 21.0 | (II)-15 | 8.0 | - | - | - | - | F-4 | 100 |
| Re-49 | A-1 | 61.0 | (III)-6 | 31.0 | (II)-16 | 8.0 | - | - | - | - | F-1/F-8 | 85/15 |

(continued)

| Resist composition | Resin (A) | | Photoacid generator (B) | | Acid diffusion control agent | | Hydrophobic resin | | Surfactant | | Solvent | |
|---|---|---|---|---|---|---|---|---|---|---|---|---|
| | Type | mass% | Type | mass% | Type | mass% | Type | mass% | Type | mass% | Type | Mixing ratio (mass ratio) |
| Re-50 | A-21 | 64.0 | (III)-6 | 35.0 | (II)-17 | 1.0 | - | - | - | - | F-1/F-3 | 90/10 |

Table 5

| Resist composition | Resin (A) | | Photoacid generator (B) | | Acid diffusion control agent | | Hydrophobic resin | | Surfactant | | Solvent | |
|---|---|---|---|---|---|---|---|---|---|---|---|---|
| | Type | mass % | Type | mass% | Type | mas s% | Type | mass% | Type | mass % | Type | Mixing ratio (mass ratio) |
| Re-51 | A-28 | 71.0 | (III)-6 | 27.0 | (II)-18 | 2.0 | - | - | - | - | F-4 | 100 |
| Re-52 | A-33 | 38.0 | (III) -1 | 36.0 | (II)-19 | 26.0 | - | - | - | - | F-1 | 100 |
| Re-53 | A-12 | 45.5 | (III)-2 | 39.0 | (II)-20 | 15.5 | - | - | - | - | F-1/F-2/F-8 | 70/25/5 |
| Re-54 | A-1 | 54.0 | (III)-3 | 30.0 | (II)-21 | 16.0 | - | - | - | - | F-1/F-6 | 40/60 |
| Re-55 | A-5 | 61.9 | (III)-4 | 27.0 | (II)-22 | 11.0 | - | - | E-1 | 0.1 | F-7 | 100 |
| Re-56 | A-23 | 51.0 | (I)-1 | 33.0 | (II)-19 | 16.0 | - | - | - | - | F-1/F-8 | 85/15 |
| Re-57 | A-29 | 48.0 | (I)-2 | 38.0 | (II)-21 | 14.0 | - | - | - | - | F-1/F-2 | 70/30 |
| Re-58 | A-17 | 64.9 | (I)-3 | 27.0 | (II) -13 | 8.0 | - | - | E-2 | 0.1 | F-1/F-5 | 50/50 |
| Re-59 | A-1 | 68.0 | (I)-6 | 25.0 | (II)-15 | 7.0 | - | - | - | - | F-1/F-8 | 85/15 |
| Re-60 | A-21 | 53.0 | (I)-7 | 32.0 | (II)-16 | 15.0 | - | - | - | - | F-1/F-3 | 90/10 |
| Re-61 | A-28 | 62.0 | (I)-9 | 36.0 | (II)-1 | 2.0 | - | - | - | - | F-4 | 100 |
| Re-62 | A-11 | 57.0 | (I)-19 | 27.0 | (II)-2 | 16.0 | - | - | - | - | F-1/F-8 | 85/15 |
| Re-63 | A-16 | 76.5 | (I)-22 | 17.0 | (II)-4 | 6.5 | - | - | - | - | F-7 | 100 |
| Re-64 | A-7 | 54.0 | (I)-27 | 31.0 | (II)-5 | 15.0 | - | - | - | - | F-4 | 100 |
| Re-65 | A-34 | 50.0 | (I)-28 | 41.0 | (II)-6 | 9.0 | - | - | - | - | F-1 | 100 |
| Re-66 | A-3 | 67.9 | (I)-32 | 24.0 | (II)-9 | 7.0 | C-8 | 1.1 | - | - | F-1 | 100 |
| Re-67 | A-6 | 61.1 | (I)-33 | 35.0 | (II)-11 | 2.0 | C-5 | 1.9 | - | - | F-1/F-3 | 80/20 |
| Re-68 | A-20 | 59.6 | (I)-9/(I)-11 | 30.0/8.0 | - | - | C-4 | 2.4 | - | - | F-1/F-9 | 90/10 |
| Re-69 | A-12 | 63.0 | (I)-1/(III)-1 | 20.0/5.0 | (II)-1 | 12.0 | - | - | - | - | F-1/F-7 | 80/20 |
| Re-70 | A-9 | 69.8 | (I)-11/(III)-3 | 11.0/19. 0 | - | - | - | - | E-1/E-2 | 0.1/0. 1 | F-1/F-8 | 85/15 |
| Re-71 | A-7 | 72.0 | (I)-2/(III)-3 | 10.0/10. 0 | (IV)-1 | 8.0 | - | - | - | - | F-4 | 100 |
| Re-72 | A-23 | 66.0 | (III)-4 | 30.0 | (II)-4/(IV)-2 | 2.0/ 2.0 | - | - | - | - | F-1/F-8 | 85/15 |
| Re-73 | A-7 | 60.0 | (I)-22/(III)-1 | 20.0/10. 0 | (II)-2/(IV)-1 | 5.0/ 5.0 | - | - | - | - | F-4 | 100 |
| Re-C1 | A-34 | 72.0 | (Z)-1 | 17.0 | (IV)-1 | 11.0 | - | - | - | - | F-1 | 100 |

(continued)

| Resist composition | Resin (A) | | Photoacid generator (B) | | Acid diffusion control agent | | Hydrophobic resin | | Surfactant | | Solvent | |
|---|---|---|---|---|---|---|---|---|---|---|---|---|
| | Type | mass % | Type | mass% | Type | mas s% | Type | mass% | Type | mass % | Type | Mixing ratio (mass ratio) |
| Re-C2 | A-34 | 65.0 | (Z)-2 | 24.0 | (IV)-1 | 11.0 | - | - | - | - | F-1 | 100 |

Examples 1-1 to 1-73 and Comparative Examples 1-1 to 1-2

EUV exposure, alkaline aqueous solution development

An underlayer film-forming composition AL412 (manufactured by Brewer Science, Inc.) was applied onto a silicon wafer having a diameter of 12 inches and baked at 205°C for 60 seconds to form an underlying film having a film thickness of 20 nm. A resist composition described in Tables 3 to 5 was applied onto the film and baked at 100°C for 60 seconds to form a resist film having a film thickness of 30 nm.

An EUV exposure apparatus (manufactured by Exitech Ltd, Micro Exposure Tool, NA: 0.3, Quadrupole, outer sigma: 0.68, inner sigma: 0.36) was used to subject the obtained silicon wafer having the resist film to pattern irradiation such that the resultant pattern would have an average line width of 20 nm. Note that, as the reticle, a mask having a line size = 20 nm and a line:space =1:1 was used.

The exposed resist film was baked at 90°C for 60 seconds, and subsequently the resist film was developed with an aqueous tetramethylammonium hydroxide solution (2.38 mass%) for 30 seconds, and subsequently rinsed with pure water for 30 seconds. Subsequently, this was spin-dried to obtain a positive pattern.

Evaluations

Pattern profile

The cross-sectional profile of the obtained pattern was observed with a critical dimension scanning electron microscope (SEM, manufactured by Hitachi, Ltd., S-9380II), and a pattern line width Lb at the bottom of the resist pattern and a pattern line width La at the top of the resist pattern were measured The value of Lb/La was used as an indicator and the cross-sectional squareness of the pattern profile was evaluated in accordance with the following grades. AA is the best, and E is the worst. Practically, the grades C or higher are desirable.

AA: $1.00 \leq (Lb/La) \leq 1.01$
A: $1.01 < (Lb/La) \leq 1.02$
B: $1.02 < (Lb/La) \leq 1.03$
C: $1.03 < (Lb/La) \leq 1.04$
D: $1.04 < (Lb/La) \leq 1.05$
E: $1.05 < (Lb/La)$

The results will be described in Table 6.

Table 6

| Table 4 | Resist composition | Pattern profile |
|---|---|---|
| Example 1-1 | Re-1 | AA |
| Example 1-2 | Re-2 | AA |
| Example 1-3 | Re-3 | AA |
| Example 1-4 | Re-4 | AA |
| Example 1-5 | Re-5 | AA |
| Example 1-6 | Re-6 | AA |
| Example 1-7 | Re-7 | AA |
| Example 1-8 | Re-8 | AA |
| Example 1-9 | Re-9 | AA |
| Example 1-10 | Re-10 | AA |
| Example 1-11 | Re-11 | AA |
| Example 1-12 | Re-12 | AA |
| Example 1-13 | Re-13 | AA |
| Example 1-14 | Re-14 | AA |

(continued)

| Table 4 | Resist composition | Pattern profile |
|---|---|---|
| Example 1-15 | Re-15 | AA |
| Example 1-16 | Re-16 | AA |
| Example 1-17 | Re-17 | AA |
| Example 1-18 | Re-18 | AA |
| Example 1-19 | Re-19 | AA |
| Example 1-20 | Re-20 | AA |
| Example 1-21 | Re-21 | AA |
| Example 1-22 | Re-22 | AA |
| Example 1-23 | Re-23 | AA |
| Example 1-24 | Re-24 | AA |
| Example 1-25 | Re-25 | AA |
| Example 1-26 | Re-26 | AA |
| Example 1-27 | Re-27 | AA |
| Example 1-28 | Re-28 | AA |
| Example 1-29 | Re-29 | AA |
| Example 1-30 | Re-30 | AA |
| Example 1-31 | Re-31 | AA |
| Example 1-32 | Re-32 | AA |
| Example 1-33 | Re-33 | AA |
| Example 1-34 | Re-34 | AA |
| Example 1-35 | Re-35 | AA |
| Example 1-36 | Re-36 | AA |
| Example 1-37 | Re-37 | AA |
| Example 1-38 | Re-38 | AA |
| Example 1-39 | Re-39 | AA |
| Example 1-40 | Re-40 | AA |
| Table 4 (continued) | Resist composition | Pattern profile |
| Example 1-41 | Re-41 | AA |
| Example 1-42 | Re-42 | AA |
| Example 1-43 | Re-43 | AA |
| Example 1-44 | Re-44 | AA |
| Example 1-45 | Re-45 | AA |
| Example 1-46 | Re-46 | AA |
| Example 1-47 | Re-47 | AA |
| Example 1-48 | Re-48 | AA |
| Example 1-49 | Re-49 | AA |
| Example 1-50 | Re-50 | AA |
| Example 1-51 | Re-51 | AA |

(continued)

| Table 4 (continued) | Resist composition | Pattern profile |
|---|---|---|
| Example 1-52 | Re-52 | AA |
| Example 1-53 | Re-53 | AA |
| Example 1-54 | Re-54 | AA |
| Example 1-55 | Re-55 | AA |
| Example 1-56 | Re-56 | AA |
| Example 1-57 | Re-57 | AA |
| Example 1-58 | Re-58 | AA |
| Example 1-59 | Re-59 | AA |
| Example 1-60 | Re-60 | AA |
| Example 1-61 | Re-61 | AA |
| Example 1-62 | Re-62 | AA |
| Example 1-63 | Re-63 | AA |
| Example 1-64 | Re-64 | AA |
| Example 1-65 | Re-65 | AA |
| Example 1-66 | Re-66 | AA |
| Example 1-67 | Re-67 | AA |
| Example 1-68 | Re-68 | AA |
| Example 1-69 | Re-69 | AA |
| Example 1-70 | Re-70 | AA |
| Example 1-71 | Re-71 | AA |
| Example 1-72 | Re-72 | AA |
| Example 1-73 | Re-73 | AA |
| Comparative Example 1-1 | Re-C1 | D |
| Comparative Example 1-2 | Re-C2 | E |
| - | - | - |
| - | - | - |
| - | - | - |
| - | - | - |
| - | - | - |

Table 6 above has demonstrated that resist compositions of the present invention provide ultrafine patterns formed by alkali development that have very good pattern profiles. On the other hand, the resist compositions of Comparative Examples were insufficient in terms of this performance.

Examples 2-1 to 2-73 and Comparative Examples 2-1 to 2-2

EUV exposure, organic solvent development

An underlayer film-forming composition AL412 (manufactured by Brewer Science, Inc.) was applied onto a silicon wafer having a diameter of 12 inches and baked at 205°C for 60 seconds to form an underlying film having a film thickness of 20 nm. A resist composition described in Tables 3 to 5 was applied onto the film and baked at 100°C for 60 seconds to form a resist film having a film thickness of 30 nm.

An EUV exposure apparatus (manufactured by Exitech Ltd, Micro Exposure Tool, NA: 0.3, Quadrupole, outer sigma: 0.68, inner sigma: 0.36) was used to subject the obtained silicon wafer having the resist film to pattern irradiation such that the resultant pattern would have an average line width of 20 nm. Note that, as the reticle, a mask having a line size = 20 nm and a line: space = 1:1 was used.

The exposed resist film was baked at 90°C for 60 seconds, and subsequently developed with n-butyl acetate for 30 seconds; and this was spin-dried to obtain a negative pattern.

Evaluations

Pattern profile

The cross-sectional profile of the obtained pattern was observed with a critical dimension scanning electron microscope (SEM, manufactured by Hitachi, Ltd., S-9380II), and a pattern line width Lb at the bottom of the resist pattern and a pattern line width La at the top of the resist pattern were measured The value of La/Lb was used as an indicator and the cross-sectional squareness of the pattern profile was evaluated in accordance with the following grades. AA is the best, and E is the worst. Practically, the grades C or higher are desirable.

AA:

$$1.00 \leq (La/Lb) \leq 1.01$$

A:

$$1.01 < (La/Lb) \leq 1.02$$

B:

$$1.02 < (La/Lb) \leq 1.03$$

C:

$$1.03 < (La/Lb) \leq 1.04$$

D:

$$1.04 < (La/Lb) \leq 1.05$$

E:

$$1.05 < (La/Lb)$$

The results will be described in Table 7.

Table 7

| Table 5 | Resist composition | Pattern profile |
|---|---|---|
| Example 2-1 | Re-1 | AA |
| Example 2-2 | Re-2 | AA |
| Example 2-3 | Re-3 | AA |
| Example 2-4 | Re-4 | AA |

(continued)

| Table 5 | Resist composition | Pattern profile |
|---|---|---|
| Example 2-5 | Re-5 | AA |
| Example 2-6 | Re-6 | AA |
| Example 2-7 | Re-7 | AA |
| Example 2-8 | Re-8 | AA |
| Example 2-9 | Re-9 | AA |
| Example 2-10 | Re-10 | AA |
| Example 2-11 | Re-11 | AA |
| Example 2-12 | Re-12 | AA |
| Example 2-13 | Re-13 | AA |
| Example 2-14 | Re-14 | AA |
| Example 2-15 | Re-15 | AA |
| Example 2-16 | Re-16 | AA |
| Example 2-17 | Re-17 | AA |
| Example 2-18 | Re-18 | AA |
| Example 2-19 | Re-19 | AA |
| Example 2-20 | Re-20 | AA |
| Example 2-21 | Re-21 | AA |
| Example 2-22 | Re-22 | AA |
| Example 2-23 | Re-23 | AA |
| Example 2-24 | Re-24 | AA |
| Example 2-25 | Re-25 | AA |
| Example 2-26 | Re-26 | AA |
| Example 2-27 | Re-27 | AA |
| Example 2-28 | Re-28 | AA |
| Example 2-29 | Re-29 | AA |
| Example 2-30 | Re-30 | AA |
| Example 2-31 | Re-31 | AA |
| Example 2-32 | Re-32 | AA |
| Example 2-33 | Re-33 | AA |
| Example 2-34 | Re-34 | AA |
| Example 2-35 | Re-35 | AA |
| Example 2-36 | Re-36 | AA |
| Example 2-37 | Re-37 | AA |
| Example 2-38 | Re-38 | AA |
| Example 2-39 | Re-39 | AA |
| Example 2-40 | Re-40 | AA |
| Table 5 (continued) | Resist composition | Pattern profile |
| Example 2-41 | Re-41 | AA |

(continued)

| Table 5 (continued) | Resist composition | Pattern profile |
|---|---|---|
| Example 2-42 | Re-42 | AA |
| Example 2-43 | Re-43 | AA |
| Example 2-44 | Re-44 | AA |
| Example 2-45 | Re-45 | AA |
| Example 2-46 | Re-46 | AA |
| Example 2-47 | Re-47 | AA |
| Example 2-48 | Re-48 | AA |
| Example 2-49 | Re-49 | AA |
| Example 2-50 | Re-50 | AA |
| Example 2-51 | Re-51 | AA |
| Example 2-52 | Re-52 | AA |
| Example 2-53 | Re-53 | AA |
| Example 2-54 | Re-54 | AA |
| Example 2-55 | Re-55 | AA |
| Example 2-56 | Re-56 | AA |
| Example 2-57 | Re-57 | AA |
| Example 2-58 | Re-58 | AA |
| Example 2-59 | Re-59 | AA |
| Example 2-60 | Re-60 | AA |
| Example 2-61 | Re-61 | AA |
| Example 2-62 | Re-62 | AA |
| Example 2-63 | Re-63 | AA |
| Example 2-64 | Re-64 | AA |
| Example 2-65 | Re-65 | AA |
| Example 2-66 | Re-66 | AA |
| Example 2-67 | Re-67 | AA |
| Example 2-68 | Re-68 | AA |
| Example 2-69 | Re-69 | AA |
| Example 2-70 | Re-70 | AA |
| Example 2-71 | Re-71 | AA |
| Example 2-72 | Re-72 | AA |
| Example 2-73 | Re-73 | AA |
| Comparative Example 2-1 | Re-C1 | D |
| Comparative Example 2-2 | Re-C2 | E |
| - | - | - |
| - | - | - |
| - | - | - |
| - | - | - |

(continued)

| Table 5 (continued) | Resist composition | Pattern profile |
|---|---|---|
| - | - | - |

Table 7 above has demonstrated that resist compositions of the present invention provide ultrafine patterns formed by organic solvent development that have very good pattern profiles. On the other hand, the resist compositions of Comparative Examples were insufficient in terms of this performance.

In addition, it has been demonstrated that, in the cases of exposure with KrF, ArF, or EB, similarly, very good pattern profiles are provided

**Claims**

**1.** An actinic ray-sensitive or radiation-sensitive resin composition comprising:

a resin; and
an ionic compound having, in at least one of a cationic moiety or an anionic moiety, a group represented by - $SF_4R$, wherein R represents a hydrogen atom, a halogen atom, or a monovalent substituent.

**2.** The actinic ray-sensitive or radiation-sensitive resin composition according to claim 1, wherein the ionic compound is an ionic compound having, in the cationic moiety, the group represented by $-SF_4R$.

**3.** The actinic ray-sensitive or radiation-sensitive resin composition according to claim 1 or 2, wherein the cationic moiety of the ionic compound is a sulfonium cation or an iodonium cation.

**4.** The actinic ray-sensitive or radiation-sensitive resin composition according to any one of claims 1 to 3, wherein the ionic compound is an ionic compound having, in the anionic moiety, the group represented by $-SF_4R$.

**5.** The actinic ray-sensitive or radiation-sensitive resin composition according to any one of claims 1 to 4, wherein the group represented by $-SF_4R$ is $-SF_5$.

**6.** The actinic ray-sensitive or radiation-sensitive resin composition according to any one of claims 1 to 5, wherein the resin has a repeating unit having a phenolic hydroxy group

**7.** The actinic ray-sensitive or radiation-sensitive resin composition according to any one of claims 1 to 6, wherein the resin has a repeating unit having at least one selected from the group consisting of a lactone group, a sultone group, and a carbonate group

**8.** An actinic ray-sensitive or radiation-sensitive film formed from the actinic ray-sensitive or radiation-sensitive resin composition according to any one of claims 1 to 7.

**9.** A pattern forming method comprising:

a step of using the actinic ray-sensitive or radiation-sensitive resin composition according to any one of claims 1 to 7 to form an actinic ray-sensitive or radiation-sensitive film on a substrate;
a step of exposing the actinic ray-sensitive or radiation-sensitive film; and
a step of using a developer to develop the exposed actinic ray-sensitive or radiation-sensitive film to form a pattern

**10.** A method for producing an electronic device, the method comprising the pattern forming method according to claim 9.

**11.** A sulfonium salt compound comprising, in at least one of a cationic moiety or an anionic moiety, a group represented by $-SF_4R$,
wherein R represents a hydrogen atom, a halogen atom, or a monovalent substituent.

**12.** The compound according to claim 11, wherein the sulfonium salt compound is a sulfonium salt compound having, in the cationic moiety, the group represented by $-SF_4R$.

**13.** The compound according to claim 11 or 12, wherein the sulfonium salt compound is a sulfonium salt compound having, in the anionic moiety, the group represented by $-SF_4R$.

**14.** An iodonium salt compound comprising, in at least one of a cationic moiety or an anionic moiety, a group represented by $-SF_4R$, wherein R represents a hydrogen atom, a halogen atom, or a monovalent substituent, and the anionic moiety is represented by any one of formulas (N1) to (N3) below:

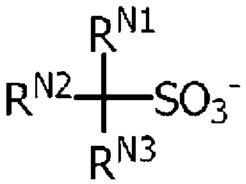
(N1)

$$R^{N4}-CO_2^-$$ (N2)

$$R^{N5}-L^{N1}-N^--L^{N2}-R^{N6}$$ (N3)

in the formula (N1),

$R^{N1}$ represents a hydrogen atom, a chlorine atom, a bromine atom, an iodine atom, a hydroxy group, a group represented by $-SF_4R$, or an organic group, wherein R represents a hydrogen atom, a halogen atom, or a monovalent substituent,
$R^{N2}$ to $R^{N3}$ each independently represent a hydrogen atom, a fluorine atom, a chlorine atom, a bromine atom, an iodine atom, a hydroxy group, a group represented by $-SF_4R$, or an organic group, wherein R represents a hydrogen atom, a halogen atom, or a monovalent substituent,
$R^{N1}$ to $R^{N3}$ may be bonded together to form a ring,
in the formula (N2),
$R^{N4}$ represents an organic group,
in the formula (N3),
$L^{N1}$ and $L^{N2}$ each independently represent $-SO_2-$, -CO-, or an alkylene group,
$R^{N5}$ and $R^{N6}$ each independently represent an organic group, and $R^{N5}$ and $R^{N6}$ may be bonded together to form a ring.

**15.** The compound according to claim 14, wherein the iodonium salt compound is an iodonium salt compound having, in the cationic moiety, the group represented by $-SF_4R$.

**16.** The compound according to claim 14 or 15, wherein the iodonium salt compound is an iodonium salt compound having, in the anionic moiety, the group represented by $-SF_4R$.

**17.** The compound according to any one of claims 11 to 16, wherein the group represented by $-SF_4R$ is $-SF_5$.

## INTERNATIONAL SEARCH REPORT

International application No.
**PCT/JP2022/045518**

**A. CLASSIFICATION OF SUBJECT MATTER**

***G03F 7/004***(2006.01)i; ***C07C 25/18***(2006.01)i; ***C07C 309/06***(2006.01)i; ***C07C 309/12***(2006.01)i; ***C07C 309/42***(2006.01)i; ***C07C 309/65***(2006.01)i; ***C07C 311/53***(2006.01)i; ***C07C 381/00***(2006.01)i; ***C07C 381/12***(2006.01)i; ***C07D 307/00***(2006.01)i; ***C07D 317/72***(2006.01)i; ***C07D 321/10***(2006.01)i; ***C07D 327/06***(2006.01)i; ***C07D 333/76***(2006.01)i; ***G03F 7/038***(2006.01)i; ***G03F 7/039***(2006.01)i; ***G03F 7/20***(2006.01)i

FI: G03F7/004 503A; G03F7/004 501; G03F7/039 601; G03F7/038 601; G03F7/20 501; G03F7/20 521; C07C309/12; C07C309/42; C07C311/53; C07C381/00; C07C309/06; C07C25/18; C07C309/65; C07D307/00; C07D321/10; C07D317/72; C07D333/76; C07D327/06; C07C381/12 CSP

According to International Patent Classification (IPC) or to both national classification and IPC

**B. FIELDS SEARCHED**

Minimum documentation searched (classification system followed by classification symbols)

G03F7/004; C07C25/18; C07C309/06; C07C309/12; C07C309/42; C07C309/65; C07C311/53; C07C381/00; C07C381/12; C07D307/00; C07D317/72; C07D321/10; C07D327/06; C07D333/76; G03F7/038; G03F7/039; G03F7/20

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | WO 2020/105346 A1 (SUMITOMO CHEMICAL CO) 28 May 2020 (2020-05-28) paragraphs [0059]-[0063], examples | 1, 4-5, 8-10 |
| A | | 2-3, 6-7, 11-17 |
| X | JP 2016-040240 A (UBE INDUSTRIES) 24 March 2016 (2016-03-24) claims, examples | 14-17 |
| A | | 1-13 |
| E, X | WO 2022/270134 A1 (JSR CORP) 29 December 2022 (2022-12-29) paragraphs [0061], [0074]-[0077], example 12 | 1, 3-11, 13, 17 |
| E, A | | 2, 12, 14-16 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **10 February 2023** | **28 February 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**
**Information on patent family members**

International application No.
**PCT/JP2022/045518**

| Patent document cited in search report | Publication date (day/month/year) | Patent family member(s) | Publication date (day/month/year) |
|---|---|---|---|
| WO 2020/105346 A1 | 28 May 2020 | CN 113056525 A<br>KR 10-2021-0093248 A<br>TW 202030264 A | |
| JP 2016-040240 A | 24 March 2016 | US 2016/0046566 A1<br>claims, examples | |
| WO 2022/270134 A1 | 29 December 2022 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2020180121 A
- US 20160070167 A
- JP 2014098921 A
- JP 2014041327 A
- WO 2018193954 A
- WO 2020004306 A
- WO 2020066824 A
- WO 2020158337 A
- US 20160070167 A1
- US 20150004544 A1
- US 20160237190 A1
- US 20160274458 A1
- JP 2014059543 A
- JP 2013061648 A